# EUROPÄISCHE PATENTSCHRIFT

(11) **EP 1 532 090 B1**
(45) Veröffentlichungstag und Bekanntmachung des Hinweises auf die Patenterteilung: **17.03.2010**
(21) Anmeldenummer: 03790821.7
(22) Anmeldetag: 28.07.2003
(51) Int. Cl.: C07C 25/22, C07C 22/08, C07C 25/00, C07C 25/24

(54) **CYCLOPENTA ¬B NAPHTHALINDERIVATE**
CYCLOPENTA ¬B NAPHTHALENE DERIVATIVES
DERIVATES DE CYCLOPENTA ¬B NAPHTALINE

(30) Priorität: 26.08.2002 DE 10238999; 02.06.2003 DE 10324843
(43) Veröffentlichungstag der Anmeldung: 25.05.2005
(73) Patentinhaber: Merck Patent GmbH, 64293 Darmstadt (DE)
(72) Erfinder: LIETZAU, Lars, 64295 Darmstadt (DE); BREMER, Matthias, 64295 Darmstadt (DE); KLASEN-MEMMER, Melanie, 67259 Heuchelheim (DE); HECKMEIER, Michael, 69502 Hemsbach (DE)
(86) Internationale Anmeldenummer: PCT/EP2003/008285
(87) Internationale Veröffentlichungsnummer: WO 2004/020375

(56) Entgegenhaltungen:
- EP-A- 1 223 209
- WO-A-02/46330
- WO-A-98/46547
- DE-A- 4 434 974
- CHEMICAL ABSTRACTS, vol. 122, no. 16, 17. April 1995 (1995-04-17) Columbus, Ohio, US; abstract no. 201408, YOKOKOJI, OSAMU ET AL: "Fluorine-containing indane derivatives and liquid crystal compositions containing same" XP002248134 & JP 06 263663 A (ASAHI GLASS CO LTD, JAPAN) 20. September 1994 (1994-09-20)

## Beschreibung

Die vorliegende Erfindung betrifft Cyclopenta[b]naphthalinderivate, deren Verwendung in flüssigkristallinen bzw. mesogenen Medien, flüssigkristalline bzw. mesogene Medien enthaltend diese Derivate, sowie elektrooptische Anzeigeelemente enthaltend diese flüssigkristallinen bzw. mesogenen Medien.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete sind insbesondere Anzeigedisplays für Uhren und Taschenrechner sowie große Anzeigetafeln, wie sie in Bahnhöfen, Flughäfen und Sportarenen verwendet werden. Weitere Anwendungsgebiete sind Displays von tragbaren Computern und Navigationssystemen sowie Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, dass viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante ε des flüssigkristallinen Mediums ist also für die beiden Orientierungen verschieden groß. Substanzen, deren Dielektrizitätskonstante bei senkrechter Orientierung der Molekül-Längsachsen zu den Kondensatorplatten größer ist als bei paralleler Anordnung, werden als dielektrisch positiv bezeichnet. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, dass die größere der dielektrischen Konstanten wirksam wird. Die Stärke der Wechselwirkung mit dem elektrischen Feld hängt dabei von der Differenz der beiden Konstanten ab. Bei kleinen Differenzen sind höhere Schaltspannungen erforderlich als bei großen. Durch den Einbau geeigneter polarer Gruppen, wie z.B. von Nitrilgruppen oder Fluor, in die Flüssigkristallmoleküle läßt sich ein weiter Bereich von Arbeitsspannungen realisieren.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment. Die Orientierung des größeren Dipolmoments entlang der Längsachse des Moleküls bestimmt auch die Orientierung des Moleküls in einer Flüssigkristallanzeige im feldfreien Zustand. Bei den am weitesten verbreiteten TN-Zellen (abgeleitet aus dem Englischen: "twisted nematic", verdrillt nematisch) ist eine nur etwa 5 bis 10 µm dicke flüssigkristalline Schicht zwischen zwei ebenen Glasplatten angeordnet, auf die jeweils eine elektrisch leitende, transparente Schicht aus Zinnoxid oder Indium-Zinnoxid als Elektrode aufgedampft ist. Zwischen diesen Filmen und der flüssigkristallinen Schicht befindet sich eine ebenfalls transparente Orientierungsschicht, die meist aus einem Kunststoff (z.B. Polyimiden) besteht. Sie dient dazu, durch Oberflächenkräfte die Längsachsen der benachbarten kristallinen Moleküle in eine Vorzugsrichtung zu bringen, so dass sie im spannungsfreien Zustand einheitlich mit der gleichen Orientierung flach oder mit demselben kleinen Anstellwinkel auf der Innenseite der Displayfläche aufliegen. Auf der Außenseite des Displays sind zwei Polarisationsfolien, die nur linear polarisiertes Licht ein- und austreten lassen, in einer bestimmten Anordnung aufgeklebt.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen zu erreichen. Schwierigkeiten bereitet jedoch noch die starke Blickwinkelabhängigkeit bei Flüssigkristallanzeigen, wie sie beispielsweise für Laptops verwendet werden. Die beste Abbildungsqualität läßt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Im feldfreien Zustand sind diese Moleküle senkrecht zur Glasfläche des Displays orientiert. Auf diese Weise konnte eine Verbesserung der Blickwinkelabhängigkeit erreicht werden. Derartige Displays werden als VA-TFT-Displays bezeichnet (abgeleitet aus dem Englischen: "vertically aligned").

In der DE 44 34 974 A1 werden tricyclische Verbindungen der allgemeinen Formel 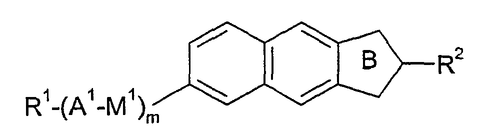 offenbart, in der die Symbole und Indizes folgende Bedeutungen haben:
R¹ ist -F, -CN, -Cl, -CF₃ oder hat, unabhängig von R², eine der bei R² aufgeführten Bedeutungen;
R² ist H oder ein geradkettiger oder verzweigter Alkylrest mit 1 bis 20 C-Atomen (mit oder ohne asymmetrisches C-Atom), wobei auch eine oder mehrere -CH₂-Gruppen (jedoch nicht die direkt an den Fünfring gebundene) durch -O-, -S-, -CH=CH-, -C≡C-, Cyclopropan-1,2-diyl, -Si(CH₃)₂-, 1,4-Phenylen, 1,4-Cyclohexylen, 1,3-Cyclopentylen, 1,3-Cyclobutylen, 1,3-Dioxan-2,5-diyl ersetzt sein können, mit der Maßgabe, dass Sauerstoffatome und Schwefelatome nicht unmittelbar verbunden sein dürfen, und wobei auch ein oder mehrere H-Atome des Alkylrestes durch F, Cl, Br oder OR³ substituiert sein können, oder eine optisch aktive oder racemische Gruppe;
Ring B ist 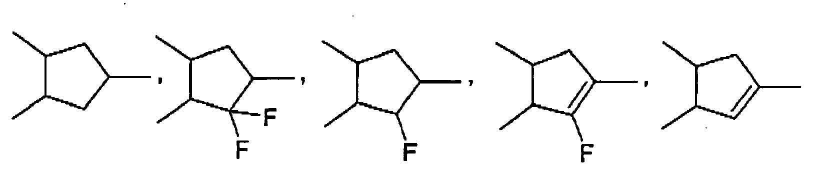
A¹ ist 1,4-Phenylen, 1,4-Cyclohexylen, Pyridin-2,5-diyl, Pyrimidin-2,5-diyl, (1,3)-Thiazol-2,5-diyl, (1,3)-Thiazol-2,4-diyl, wobei auch ein oder mehrere Wasserstoffe durch F substituiert sein können, (1,3,4)Thiadiazol-2,5-diyl;
M¹ ist eine Einfachbindung, -C≡C-, -CH₂CH₂-, -O-CO-, -CO-O-, -CO-, -OCH₂-, -CH₂O-, -O-CO-O-; und m ist Null oder Eins.

Das Δε der in diesem Dokument offenbarten Verbindungen ist jedoch nicht ausreichend, um beispielsweise in VA-TFT-Displays zufriedenstellende Eigenschaften zu gewährleisten.

Die Entwicklung auf dem Gebiet der flüssigkristallinen Materialien ist bei weitem noch nicht abgeschlossen. Zur Verbesserung der Eigenschaften flüssigkristalliner Anzeigenelemente ist man ständig bemüht, neue Verbindungen zu entwickeln, die eine Optimierung derartiger Displays ermöglichen.

WO 98746547 A1 offenbart Indene und ein Verfahren zu deren Herstellung.

WO 02/46330 und Chemnical Abstracts, Bd. 122, Nr. 16, Yokoji, Osamu et al. "Fluorine-containing indane derivatives and liquid crystal compositions containing same" offenbaren fluorierte Indane als Bestandteile flüssigkristalliner Mischungen. EP 1 223 209 A1 offfenbart fluorierte Fluorene als Bestandteile flüssigkristalliner Mischungen.

Eine Aufgabe der vorliegenden Erfindung war es, Verbindungen mit vorteilhaften Eigenschaften für den Einsatz in flüssigküstallinen Medien zur Verfügung zu stellen.

Diese Aufgabe wird erfindungsgemäß gelöst durch Cyclopenta[b]naphthalinderivate ausgewählt aus der Gruppe der allgemeinen Formeln (II) bis (XI) 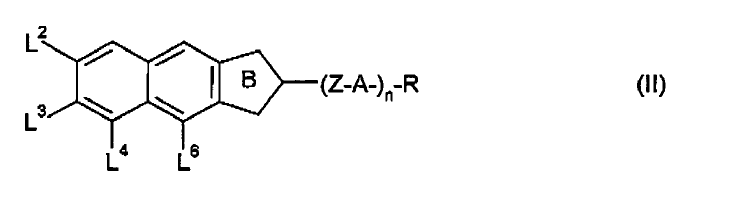 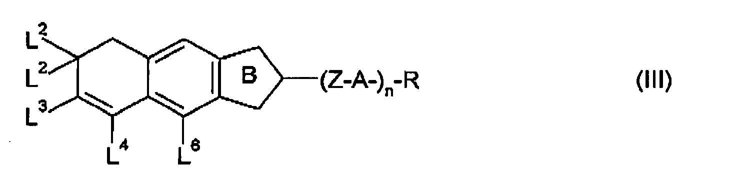 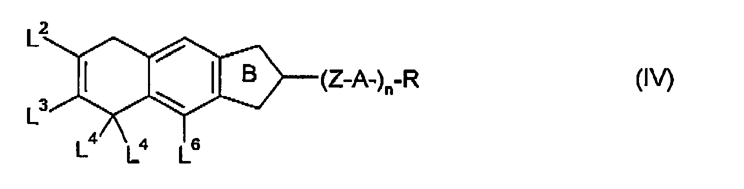 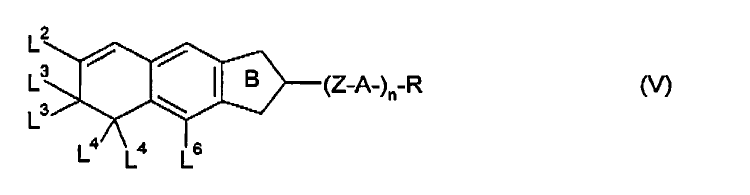 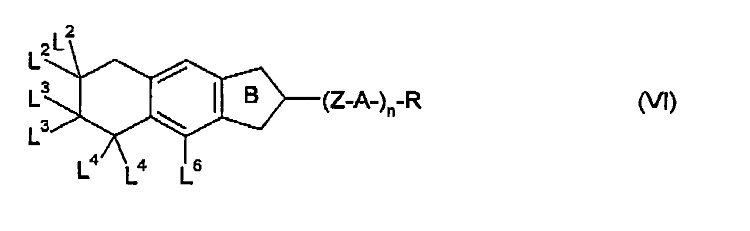 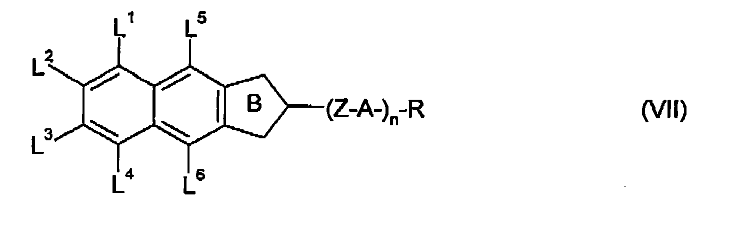 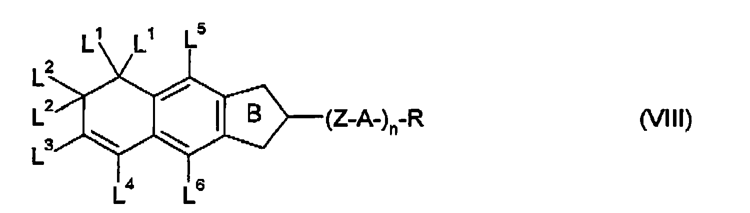 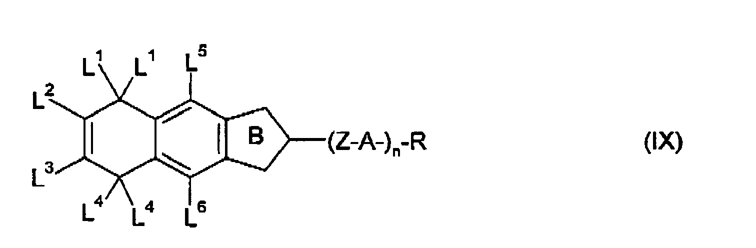 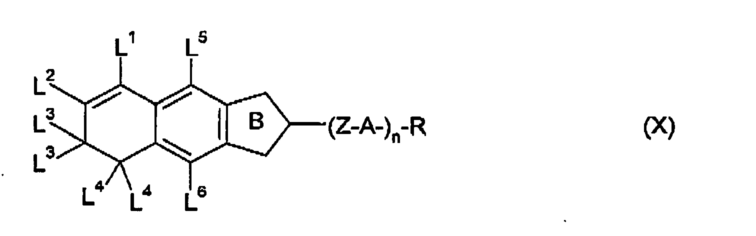 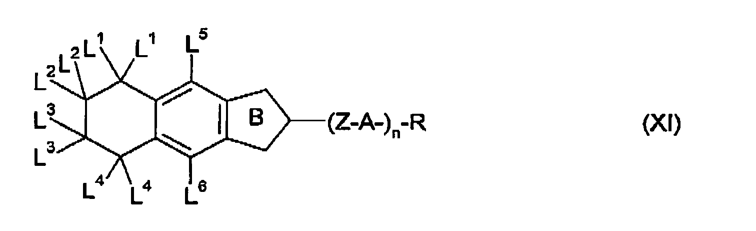 worin in den Formeln (II) bis (XI):
- Z: jeweils unabhängig voneinander eine Einfachbindung, eine Doppelbindung, -CF₂O-, -OCF₂-, -CH₂CH₂-, -CF₂CF₂-, -C(O)O-, -OC(O)-, -CH₂O-, -OCH₂-, -CF=CH-, -CH=CF-, -CF=CF-, -CH=CH- oder -C≡C-,
- A: jeweils unabhängig voneinander 1,4-phenylen, worin =CH- ein- oder zweimal durch =N- ersetzt sein kann, und das ein- bis viermal unabhängig voneinander mit Halogen (-F, -Cl, -Br, -I), -CN, -CH₃, -CH₂F, -CHF₂, -CF₃, -OCH₃, -OCH₂F, -OCHF₂ oder -OCF₃ substituiert sein kann, 1,4-Cyclohexylen, 1,4-Cyclohexenylen oder 1,4-Cyclohexadienylen, worin -CH₂- ein- oder zweimal unabhängig voneinander durch -O- oder -S- so ersetzt sein können, dass Heteroatome nicht direkt benachbart sind, und die ein- oder mehrfach durch Halogen substituiert sein können, 1,3-Cyclobutylen oder Bicyclo[2.2.2]octan,
- R: Wasserstoff, einen unsubstituierten, einen einfach durch -CF₃ oder mindestens einfach durch Halogen substituierten Alkyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN, -NCS, -SF₅, -CF₃, -OCF₃, -OCHF₂ oder -OCH₂F, und
- n: 1, 2 oder 3,
bedeuten,
worin in den Formeln (II) bis (VI) 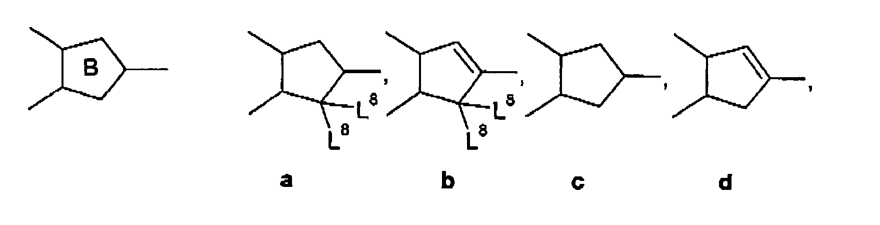 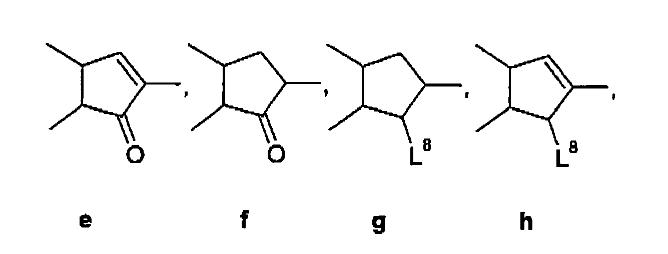 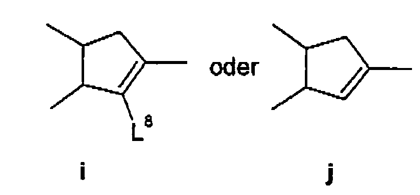
- L², L³, L⁸: jeweils unabhängig voneinander Wasserstoff, einen unsubstituierten oder einen mindestens einfach durch Halogen substituierten Alkyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -CO-, -COO-, -OCO- oder -OCF-O- so ersetzt sein können, dass Heteroatome nicht direkt benachbart sind, Halogen, -CN, -SCN, -NCS, -SF₅, -CF₃, -OCF₃, -OCHF₂, -OCH₂F oder -(Z-A-)ₙ-R, und
- L⁴, L⁶: jeweils unabhängig voneinander Wasserstoff, einen mindestens einfach durch Halogen substituierten Alkyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SF₅, -SCN, -NCS, -CF₃, -OCF₃, -OCHF₂ oder -OCH₂F, mit der Maßgabe, dass L⁴ und L⁶ nicht gleichzeitig Wasserstoff sein dürfen,
bedeuten, und
worin in den Formeln (VII) bis (XI) 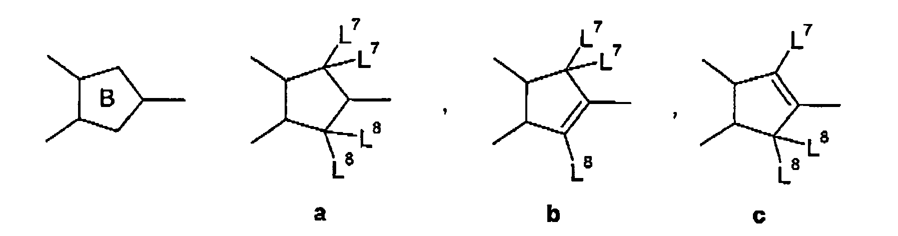 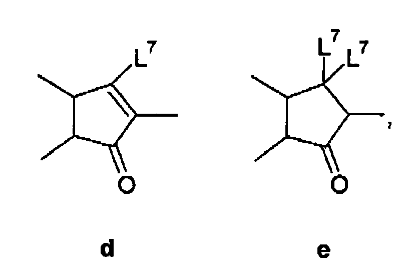 und
- L¹ - L⁸: jeweils unabhängig voneinander Wasserstoff, einen unsubstituierten oder einen mindestens einfach durch Halogen substituierten Alkyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN, -NCS, -SF₅, -CF₃, -OCF₃, -OCHF₂, -OCH₂F oder-(Z-A-)ₙ-R, mit der Maßgabe, dass L² und/oder L³ kein Wasserstoff ist,
bedeuten.

Eine weitere Aufgabe der vorliegenden Erfindung war es, flüssigkristalline Verbindungen insbesondere für die Verwendung in VA-TFT-Displays zur Verfügung zu stellen.

Diese Aufgabe wird erfindungsgemäß gelöst durch die Bereitstellung von Cyclopenta[b]naphthalinderivaten mit negativem Δε.

Gegenstand der vorliegenden Erfindung sind insbesondere Cyclopenta[b]naphthalinderivate der allgemeinen Formeln (II) bis (VI).

Bevorzugt sind Cyclopenta[b]naphthalinderivate der allgemeinen Formeln (II), (III), (V) und (VI) und besonders bevorzugt sind Cyclopenta[b]naphthalinderivate der allgemeinen Formeln (II) und (VI).

Die Verbindungen besitzen sämtlich ein negatives Δε und eignen sich daher insbesondere für eine Verwendung in VA-TFT-Displays. Vorzugsweise besitzen die erfindungsgemäßen Verbindungen ein Δε < -2 und besonders bevorzugt ein Δε < -5. Sie zeigen eine sehr gute Verträglichkeit mit den üblichen, in Flüssigkristallmischungen für Displays verwendeten Substanzen.

Durch die Substituenten, vorzugsweise Fluorsubstituenten, im Naphthalingerüst sowie die elektronegativen Atome im Ring B wird ein Dipolmoment senkrecht zur Moleküllängsachse erzeugt, das gegebenenfalls durch geeignete Substituenten in den Flügeleinheiten -(Z-A-)ₙ-R weiter verstärkt werden kann. Im feldfreien Zustand richten sich die Verbindungen der Formeln (II) bis (VI) mit ihrer Moleküllängsachse senkrecht zur behandelten oder beschichteten Glasfläche des Displays aus.

Bevorzugt als 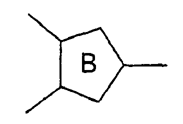 in den allgemeinen Formeln (II) bis (VI) sind 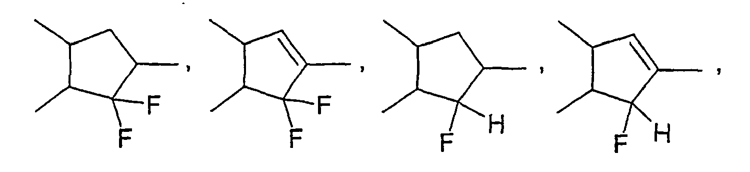 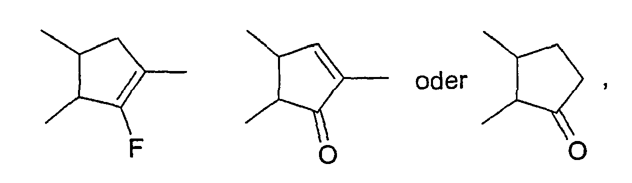 besonders bevorzugt sind 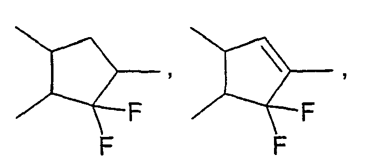 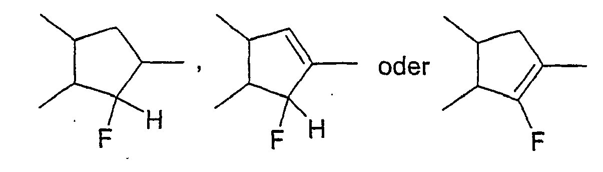 und insbesondere

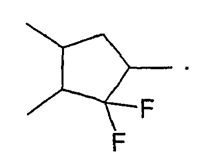

In den allgemeinen Formeln (II) bis (VI) sind A bevorzugt unabhängig voneinander gegebenenfalls substituiertes 1,4-Phenylen, gegebenenfalls substituiertes 1,4-Cyclohexylen, worin -CH₂- ein- oder zweimal durch -O-ersetzt sein kann, oder gegebenenfalls substituiertes 1,4-Cyclohexenylen.

Besonders bevorzugt sind A unabhängig voneinander

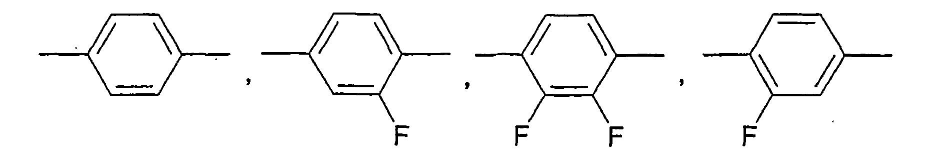

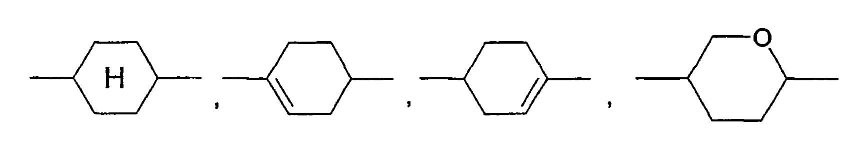

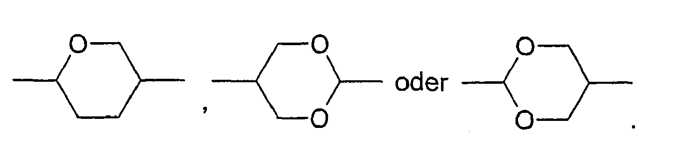

Bevorzugte Gruppen Z in den Verbindungen der allgemeinen Formeln (II) bis (VI) sind jeweils unabhängig voneinander eine Einfachbindung, -CF₂O-, -OCF₂-, -CF₂CF₂-, -CH=CH-, -CF=CH-, -CH=CF- oder -CF=CF-, besonders bevorzugt sind eine Einfachbindung, -CF₂O-, -OCF₂-, -CF₂CF₂-, -CF=CH-, -CH=CF- oder -CF=CF-.

R, L² und L³ in den allgemeinen Formeln (II) bis (VI) können jeweils unabhängig voneinander ein Alkylrest und/oder ein Alkoxyrest mit 1 bis 15 C-Atomen sein, der geradkettig oder verzweigt sein kann. Vorzugsweise ist er geradkettig, hat 1, 2, 3, 4, 5, 6 oder 7 C-Atome und ist demnach vorzugsweise Methyl, Ethyl, Propyl, Butyl, Pentyl, Hexyl, Heptyl, Methoxy, Ethoxy, Propoxy, Butoxy, Pentoxy, Hexoxy oder Heptoxy.

R, L² und L³ können jeweils unabhängig voneinander Oxaalkyl sein, vorzugsweise geradkettiges 2-Oxapropyl (= Methoxymethyl), 2- (= Ethoxymethyl) oder 3-Oxabutyl (= Methoxyethyl), 2-, 3- oder 4-Oxapentyl, 2-, 3-, 4- oder 5-Oxahexyl, 2-, 3-, 4-, 5- oder 6-Oxaheptyl.

R, L² und L³ können jeweils unabhängig voneinander ein Alkenylrest mit 2 bis 15 C-Atomen sein, der geradkettig oder verzweigt sein kann. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl.

R, L² und L³ können jeweils unabhängig voneinander ein Alkylrest mit 1 bis 15 C-Atomen sein, in dem eine CH₂-Gruppe durch -O- und eine durch -CO- ersetzt ist, wobei diese bevorzugt benachbart sind. Somit beinhaltet dieser eine Acyloxygruppe -CO-O- oder eine Oxycarbonylgruppe -O-CO-. Vorzugsweise ist dieser geradkettig und hat 2 bis 6 C-Atome.

R, L² und L³ können jeweils unabhängig voneinander ein Alkylrest mit 1 bis 15 C-Atomen sein, in dem eine CH₂-Gruppe durch unsubstituiertes oder substituiertes -CH=CH- und eine benachbarte CH₂-Gruppe durch CO oder CO-O oder O-CO ersetzt ist, wobei dieser geradkettig oder verzweigt sein kann. Vorzugsweise ist er geradkettig und hat 4 bis 13 C-Atome.

R, L² und L³ können jeweils unabhängig voneinander ein einfach durch-CN oder -CF₃ substituierter Alkylrest mit 1 bis 15 C-Atomen oder Alkenylrest mit 2 bis 15 C-Atomen sein, wobei diese vorzugsweise geradkettig sind. Die Substitution durch -CN oder -CF₃ ist in beliebiger Position möglich.

R, L² und L³ können jeweils unabhängig voneinander ein Alkylrest sein, in dem zwei oder mehr CH₂-Gruppen durch -O- und/oder -CO-O- ersetzt sind, wobei dieser geradkettig oder verzweigt sein kann. Vorzugsweise ist er verzweigt und hat 3 bis 12 C-Atome.

R, L², L³, L⁴ und L⁶ können jeweils unabhängig voneinander ein mindestens einfach durch Halogen substituierter Alkylrest mit 1 bis 15 C-Atomen oder Alkenylrest mit 2 bis 15 C-Atomen sein, wobei diese Reste vorzugsweise geradkettig sind und Halogen vorzugsweise -F oder -Cl ist. Bei Mehrfachsubstitution ist Halogen vorzugsweise -F. Die resultierenden Reste schließen auch perfluorierte Reste wie -CF₃ ein. Bei Einfachsubstitution kann der Fluor- oder Chlorsubstituent in beliebiger Position sein, vorzugsweise ist er in ω-Position.

Besonders bevorzugt ist R in den allgemeinen Formeln (II) bis (VI) ein Alkylrest, Alkoxyrest oder Alkenylrest mit 1 bis 7 bzw. 2 bis 7 C-Atomen.

Bevorzugt ist L² und L³ in den allgemeinen Formeln (II) bis (VI) Wasserstoff, ein Alkylrest, Alkoxyrest oder Alkenylrest mit 1 bis 7 bzw. 2 bis 7 C-Atomen oder ein Halogen, besonders bevorzugt Wasserstoff, ein Alkoxyrest mit 1 bis 7 C-Atomen, Fluor oder Chlor, und insbesondere Fluor.

Bevorzugt ist L⁴ und L⁶ in den allgemeinen Formeln (II) bis (VI) Wasserstoff, ein mindestens einfach durch Halogen substituierter Alkylrest, Alkoxyrest oder Alkenylrest mit 1 bis 7 bzw. 2 bis 7 C-Atomen oder ein Halogen, besonders bevorzugt -CF₃, Fluor oder Chlor, und insbesondere Fluor, mit der Maβgabe, dass L⁴ und L⁶ nicht gleichzeitig Wasserstoff sein dürfen.

Bevorzugt ist L⁸ in den allgemeinen Formeln (II) bis (VI) Fluor.

Bevorzugte Verbindungen der allgemeinen Formeln (II) bis (VI) weisen keine, eine oder zwei Flügeleinheiten ZA auf, das heißt n = 0, 1 oder 2, besonders bevorzugt ist n = 1.

Eine weitere Aufgabe der vorliegenden Erfindung war es, Verbindungen insbesondere für die Verwendung in mesogenen Steuermedien zur Verfügung zu stellen, wobei diese Steuermedien insbesondere in elektrooptischen Lichtsteuerelementen eingesetzt werden, die bei einer Temperatur betrieben werden, bei der das mesogene Steuermedium im nicht angesteuerten Zustand in der isotropen Phase vorliegt.

Diese Aufgabe wird erfindungsgemäß gelöst durch die Bereitstellung von Cyclopenta[b]naphthalinderivaten mit positivem Δε.

Gegenstand der vorliegenden Erfindung sind somit insbesondere Cyclopenta[b]naphthalinderivate der allgemeinen Formeln (VII) bis (XI) worin Z, A, R, n, L¹ bis L⁸ sowie 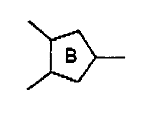 die in Bezug auf die Formeln (II) bis (XI) angegebenen Bedeutungen haben.

Bevorzugt sind Cyclopenta[b]naphthalinderivate der allgemeinen Formeln (VII) und (XI) und besonders bevorzugt sind Cyclopenta[b]naphthalinderivate der allgemeinen Formel (VII).

Die Verbindungen der Formeln (VII) bis (XI) besitzen sämtlich ein positives Δε. Vorzugsweise besitzen die erfindungsgemäßen Verbindungen der Formeln (VII) bis (XI) ein Δε > +10, besonders bevorzugt ein Δε > +15 und insbesondere ein Δε > +20. Sie zeigen eine sehr gute Verträglichkeit mit den üblichen, in mesogenen Steuermedien verwendeten Substanzen.

Die Cyclopenta[b]naphthalinderivate der allgemeinen Formel (VII) weisen dabei vorzugsweise die folgenden Strukturformeln auf: 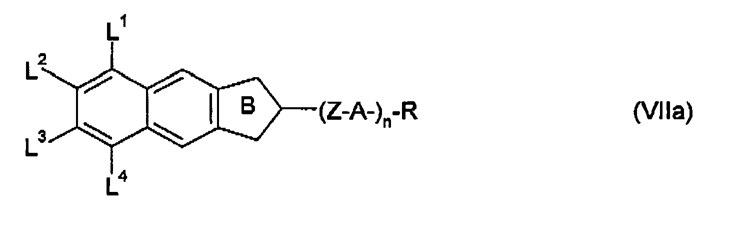 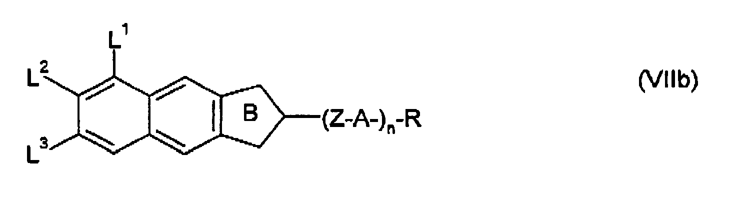 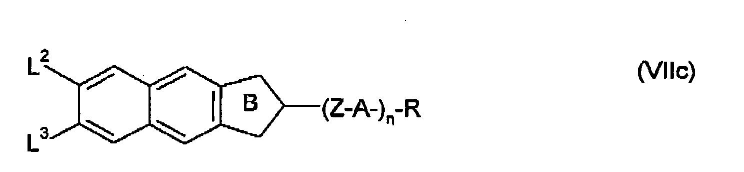 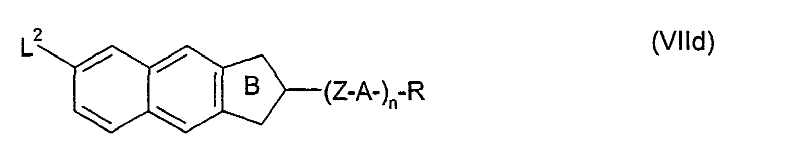 wobei die Strukturformeln (VIIa) und (VIIc) besonders bevorzugt sind.

Bevorzugt als 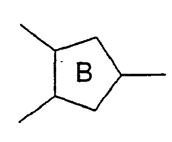 in den allgemeinen Formeln (VII) bis (XI) sind 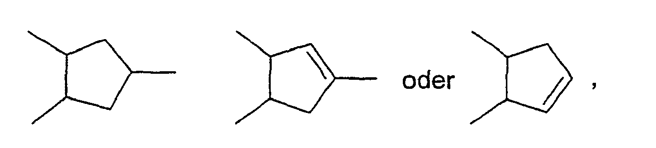 besonders bevorzugt ist

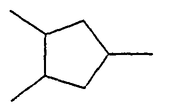

In den allgemeinen Formeln (VII) bis (XI) sind A bevorzugt unabhängig voneinander gegebenenfalls substituiertes 1,4-Phenylen, gegebenenfalls substituiertes 1,4-Cyclohexylen, worin -CH₂- ein- oder zweimal durch -O-ersetzt sein kann, oder gegebenenfalls substituiertes 1,4-Cyclohexenylen.

Besonders bevorzugt sind A unabhängig voneinander

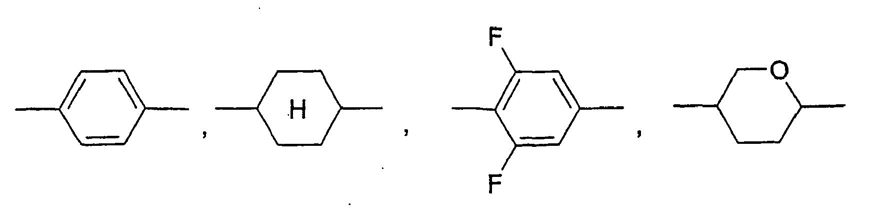

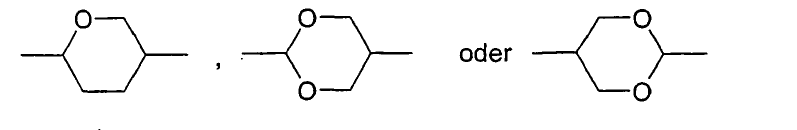

Bevorzugte Gruppen Z in den Verbindungen der allgemeinen Formeln (VII) bis (XI) sind jeweils unabhängig voneinander eine Einfachbindung, -CF₂O-, -OCF₂-, -CF₂CF₂-, -CH=CH-, -CF=CH-, -CH=CF- oder -CF=CF-, besonders bevorzugt sind eine Einfachbindung, -CF₂O-, -OCF₂-, -CF₂CF₂- oder -CF=CF-.

R und L¹ bis L⁸ in den allgemeinen Formeln (VII) bis (XI) können jeweils unabhängig voneinander ein Alkylrest und/oder ein Alkoxyrest mit 1 bis 15 C-Atomen sein, der geradkettig oder verzweigt sein kann. Vorzugsweise ist er geradkettig, hat 1, 2, 3, 4, 5, 6 oder 7 C-Atome und ist demnach vorzugsweise Methyl, Ethyl, Propyl, Butyl, Pentyl, Hexyl, Heptyl, Methoxy, Ethoxy, Propoxy, Butoxy, Pentoxy, Hexoxy oder Heptoxy.

R und L¹ bis L⁸ in den allgemeinen Formeln (VII) bis (XI) können jeweils unabhängig voneinander Oxaalkyl sein, vorzugsweise geradkettiges 2-Oxapropyl (= Methoxymethyl), 2- (= Ethoxymethyl) oder 3-Oxabutyl (= Methoxyethyl), 2-, 3- oder 4-Oxapentyl, 2-, 3-, 4- oder 5-Oxahexyl, 2-, 3-, 4-, 5- oder 6-Oxaheptyl.

R und L¹ bis L⁸ in den allgemeinen Formeln (VII) bis (XI) können jeweils unabhängig voneinander ein Alkenylrest mit 2 bis 15 C-Atomen sein, der geradkettig oder verzweigt sein kann. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl.

R und L¹ bis L⁸ in den allgemeinen Formeln (VII) bis (XI) können jeweils unabhängig voneinander ein Alkylrest mit 1 bis 15 C-Atomen sein, in dem eine CH₂-Gruppe durch -O- und eine durch -CO- ersetzt ist, wobei diese bevorzugt benachbart sind. Somit beinhaltet dieser eine Acyloxygruppe -CO-O- oder eine Oxycarbonylgruppe -O-CO-. Vorzugsweise ist dieser geradkettig und hat 2 bis 6 C-Atome.

R und L¹ bis L⁸ in den allgemeinen Formeln (VII) bis (XI) können jeweils unabhängig voneinander ein Alkylrest mit 1 bis 15 C-Atomen sein, in dem eine CH₂-Gruppe durch unsubstituiertes oder substituiertes -CH=CH- und eine benachbarte CH₂-Gruppe durch CO oder CO-O oder O-CO ersetzt ist, wobei dieser geradkettig oder verzweigt sein kann. Vorzugsweise ist er geradkettig und hat 4 bis 13 C-Atome.

R und L¹ bis L⁸ in den allgemeinen Formeln (VII) bis (XI) können jeweils unabhängig voneinander ein einfach durch -CN oder -CF₃ substituierter Alkylrest mit 1 bis 15 C-Atomen oder Alkenylrest mit 2 bis 15 C-Atomen sein, wobei diese vorzugsweise geradkettig sind. Die Substitution durch -CN oder -CF₃ ist in beliebiger Position möglich.

R und L¹ bis L⁸ in den allgemeinen Formeln (VII) bis (XI) können jeweils unabhängig voneinander ein Alkylrest sein, in dem zwei oder mehr CH₂-Gruppen durch -O- und/oder -CO-O- ersetzt sind, wobei dieser geradkettig oder verzweigt sein kann. Vorzugsweise ist er verzweigt und hat 3 bis 12 C-Atome.

R und L¹ bis L⁸ in den allgemeinen Formeln (VII) bis (XI) können jeweils unabhängig voneinander ein mindestens einfach durch Halogen substituierter Alkylrest mit 1 bis 15 C-Atomen oder Alkenylrest mit 2 bis 15 C-Atomen sein, wobei diese Reste vorzugsweise geradkettig sind und Halogen vorzugsweise -F oder -Cl ist. Bei Mehrfachsubstitution ist Halogen vorzugsweise -F. Die resultierenden Reste schließen auch perfluorierte Reste wie -CF₃ ein. Bei Einfachsubstitution kann der Fluor- oder Chlorsubstituent in beliebiger Position sein, vorzugsweise ist er in ω-Position.

Besonders bevorzugt ist R in den allgemeinen Formeln (VII) bis (XI) ein Alkylrest, Alkoxyrest oder Alkenylrest mit 1 bis 7 bzw. 2 bis 7 C-Atomen, insbesondere ein Alkylrest mit 1 bis 7 C-Atomen.

Bevorzugt ist L² und L³ in den allgemeinen Formeln (VII) bis (XI) unabhängig voneinander, gleich oder verschieden, Wasserstoff, Halogen, -CN, -SCN, -NCS, -SF₅, -CF₃, -CHF₂, -OCF₃ oder -OCHF₂, besonders bevorzugt Wasserstoff, Fluor, -CF₃ oder -OCF₃, mit der Maßgabe, dass L² und/oder L³ kein Wasserstoff.

Bevorzugt ist L¹ und L⁴ in den allgemeinen Formeln (VII) bis (XI) unabhängig voneinander, gleich oder verschieden, Wasserstoff oder Fluor. Besonders bevorzugt ist jedoch L¹ = L⁴ = H oder L¹ = L⁴ = F.

Bevorzugt ist L⁵ und L⁶ in den allgemeinen Formeln (VII) bis (XI) Wasserstoff.

Besonders bevorzugt sind Verbindungen der allgemeinen Formeln (VII) bis (XI) in denen L1 = L2 = L3 = L4 = F und L5 = L6 = H.

Bevorzugte Verbindungen der allgemeinen Formeln (VII) bis (XI) weisen keine, eine oder zwei Flügeleinheiten ZA auf, das heißt n = 0, 1 oder 2, besonders bevorzugt ist n = 1.

Die Verbindungen der allgemeinen Formeln (I), (II) bis (VI) sowie (VII) bis (XI) werden nach an sich bekannten Methoden dargestellt, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch in situ gebildet werden, derart, dass man sie aus dem Reaktionsgemisch nicht isoliert, sondern sofort weiter zu den Verbindungen der allgemeinen Formeln (I) bis (XI) umsetzt.

Die Synthesen verschiedener mehrfachsubstituierter Naphthalinderivate, die zum Aufbau des Fünfringes verwendet werden, werden in den Beispielen exemplarisch beschrieben. Die Ausgangssubstanzen sind nach allgemein zugänglichen Literaturvorschriften oder käuflich zu erhalten. Die beschriebenen Reaktionen sind ebenfalls als literaturbekannt anzusehen.

Eine beispielhafte Synthese zum Aufbau des Fünfrings ist im folgenden dargestellt. Die Synthese kann durch die Wahl geeigneter Ausgangsprodukte an die jeweils gewünschten Verbindungen der allgemeinen Formeln (I) bis (XI) angepasst werden.

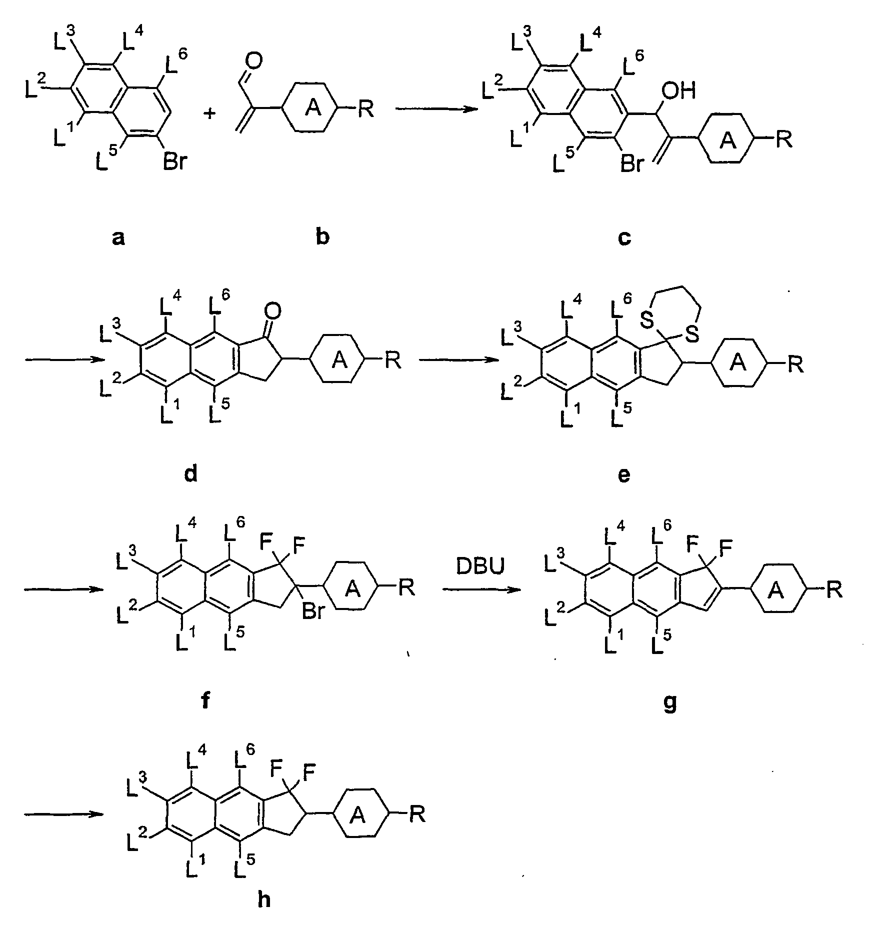

Ausgehend vom 3-Bromnaphthalin **a** wird durch Umsetzung mit dem α, βungesättigten Aldehyd **b** in Gegenwart von Lithiumdiisopropylamid (LDA) die Verbindung **c** erhalten. Diese reagiert unter Palladiumkatalyse in Gegenwart von Triethylamin unter Ringschluss zum Keton **d.** Aus dem Keton **d** und 1,3-Propandithiol wird in Gegenwart von BF₃-Diethylether das entsprechende Dithian **e** erhalten. Dieses wird mit 1,3-Dibrom-5,5-dimethylhydantoin (DBH) und HF in Pyridin zum Cyclopenta[b]naphthalinderivat **f** umgesetzt. Eliminierung von HBr in Gegenwart von Diazabicycloundecen (DBU) ergibt das Cyclopenta[b]naphthalinderivat **g.** Das Cyclopenta[b]naphthalinderivat **g** wird an Palladium/Kohle-Katalysator in Wasserstoffatmosphäre zum Cyctopenta[b]naphthalinderivat **h** hydriert.

Die dargestellten Reaktionen sind nur als beispielhaft aufzufassen. Der Fachmann kann entsprechende Variationen der vorgestellten Synthesen vornehmen sowie auch andere geeignete Synthesewege beschreiten, um Verbindungen der Formeln (I) bis (XI) zu erhalten.

Wie bereits erwähnt, können die Verbindungen der allgemeinen Formeln (I) bis (XI) in flüssigkristallinen Medien verwendet werden.

Gegenstand der vorliegenden Erfindung ist daher auch ein flüssigkristallines Medium mit mindestens zwei flüssigkristallinen Verbindungen, enthaltend mindestens eine Verbindung der allgemeinen Formeln (I) bis (XI).

Gegenstand der vorliegenden Erfindung sind auch flüssigkristalline Medien enthaltend neben einer oder mehreren erfindungsgemäßen Verbindungen der Formeln (I), (II), (III), (IV), (V), (VI), (VII), (VIII), (IX), (X) und/oder (XI) als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäurephenyl- oder -cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)-ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylen-Gruppen in diesen Verbindungen können auch fluoriert sein.

Die wichtigsten als weitere Bestandteile erfindungsgemäßer Medien in Frage kommenden Verbindungen lassen sich durch die Formeln (1), (2), (3), (4) und (5) charakterisieren:

R'-L-E-R" (1)

R'-L-COO-E-R" (2)

R'-L-OOC-E-R" (3)

R'-L-CH₂CH₂-E-R" (4)

R'-L-CF₂O-E-R" (5)

In den Formeln (1), (2), (3), (4) und (5) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2,5-diyl oder Pyridin-2,5-diyl, Dio 1,3-Dioxan-2,5-diyl und G 2-(trans-1,4-Cyclohexyl)-ethyl bedeuten.

Vorzugsweise ist einer der Reste L und E Cyc oder Phe. E ist vorzugsweise Cyc, Phe oder Phe-Cyc. Vorzugsweise enthalten die erfindungsgemäßen Medien eine oder mehrere Komponenten ausgewählt aus den Verbindungen der Formeln (1), (2), (3), (4) und (5), worin L und E ausgewählt sind aus der Gruppe Cyc und Phe und gleichzeitig eine oder mehrere Komponenten ausgewählt aus den Verbindungen der Formeln (1), (2), (3), (4) und (5), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (1), (2), (3), (4) und (5), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (1), (2), (3), (4) und (5) jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln (1 a), (2a), (3a), (4a) und (5a) bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln (1), (2), (3), (4) und (5) bedeutet E

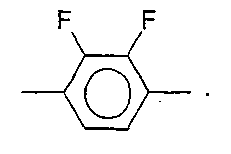

In den Verbindungen der Gruppe B, die mit den Teilformeln (1 b), (2b), (3b), (4b) und (5b) bezeichnet werden, haben R' und R" die bei den Verbindungen der Teilformeln (1a) bis (5a) angegebene Bedeutung und sind vorzugsweise Alkyl, Alkenyl, Alkoxy oder Alkoxyalkyl.

In einer weiteren kleineren Untergruppe der Verbindungen der Formeln (1), (2), (3), (4) und (5) bedeutet R" -CN; diese Untergruppe wird im folgenden als Gruppe C bezeichnet und die Verbindungen dieser Untergruppe werden entsprechend mit Teilformeln (1c), (2c), (3c), (4c) und (5c) beschrieben. In den Verbindungen der Teilformeln (1 c), (2c), (3c), (4c) und (5c) hat R' die bei den Verbindungen der Teilformeln (1a) bis (5a) angegebene Bedeutung und ist vorzugsweise Alkyl, Alkenyl, Alkoxy oder Alkoxyalkyl.

Neben den bevorzugten Verbindungen der Gruppen A, B und C sind auch andere Verbindungen der Formeln (1), (2), (3), (4) und (5) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben den erfindungsgemäßen Verbindungen der allgemeinen Formeln (I), (II), (III), (IV), (V), (VI), (VII), (VIII), (IX), (X) und/oder (XI) vorzugsweise eine oder mehrere Verbindungen aus den Gruppen A, B und/oder C. Die Massenanteile der Verbindungen aus diesen Gruppen an den erfindungsgemäßen Medien betragen:
Gruppe A: 0 bis 90%, vorzugsweise 20 bis 90%, insbesondere 30 bis 90%
Gruppe B: 0 bis 80%, vorzugsweise 10 bis 80%, insbesondere 10 bis 70%
Gruppe C: 0 bis 80%, vorzugsweise 5 bis 80%, insbesondere 5 bis 50%.

Die erfindungsgemäßen Medien enthalten vorzugsweise 1 bis 40%, besonders bevorzugt 5 bis 30% an den erfindungsgemäßen Verbindungen der Formeln (I), (II), (III), (IV), (V), (VI), (VII), (VIII), (IX), (X) und/oder (XI). Weiterhin bevorzugt sind Medien, enthaltend mehr als 40%, insbesondere 45 bis 90% an erfindungsgemäßen Verbindungen der Formeln (I), (II), (III), (IV), (V), (VI), (VII), (VIII), (IX), (X) und/oder (XI). Die Medien enthalten vorzugsweise drei, vier oder fünf erfindungsgemäße Verbindungen der Formeln (I), (II), (III), (IV), (V), (VI), (VII), (VIII), (IX), (X) und/oder (XI).

Beispiele für die Verbindungen der Formeln (1), (2), (3), (4) und (5) sind die nachstehend aufgeführten Verbindungen: 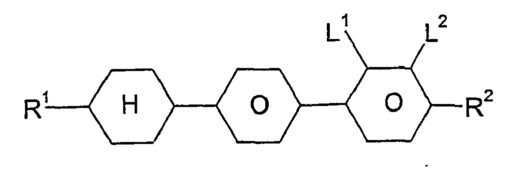 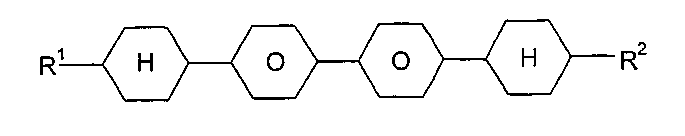 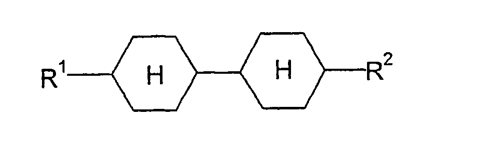 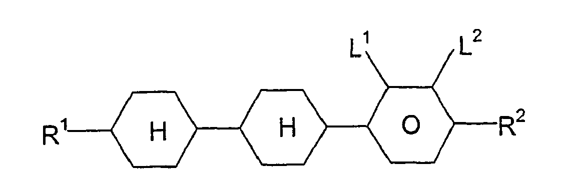 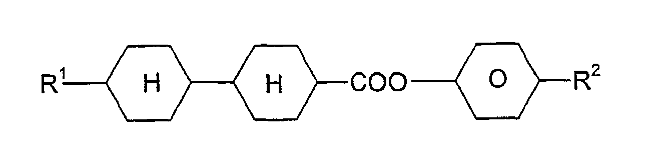 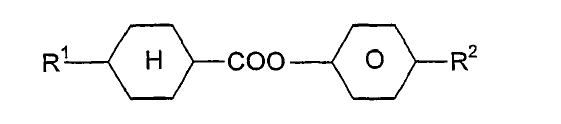 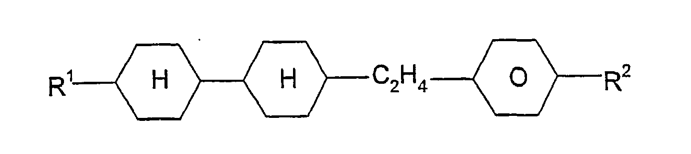 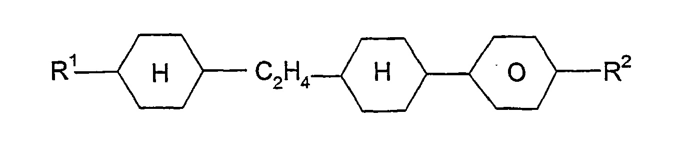 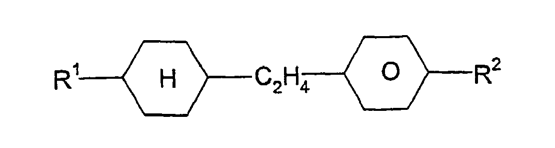 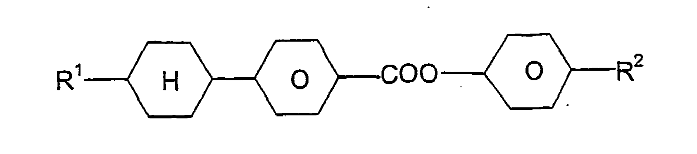 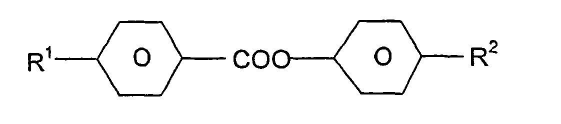 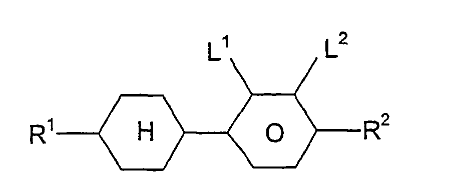 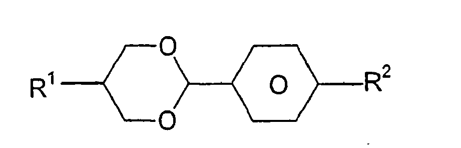 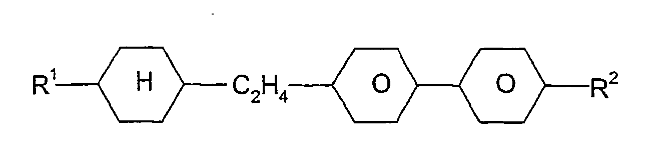 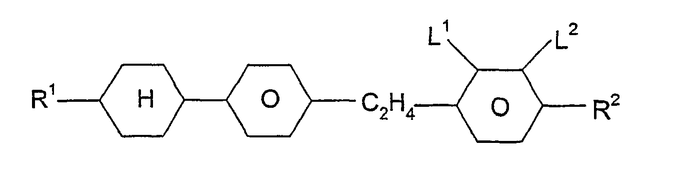 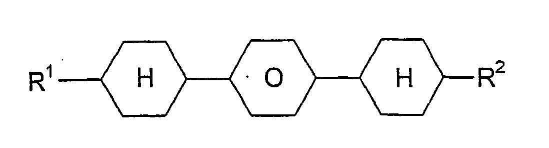 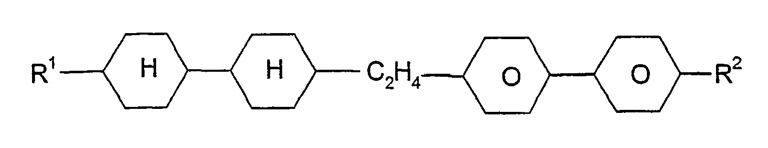 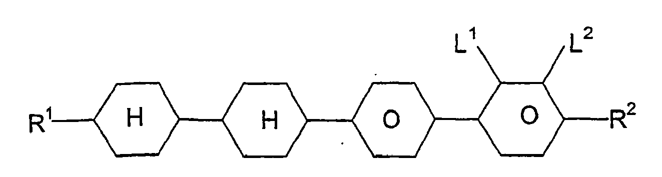 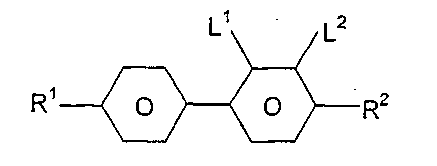 mit R¹, R² unabhängig voneinander -CₙH₂ₙ₊₁ oder -OCₙH₂ₙ₊₁ und n = 1 bis 8, sowie L¹, L² unabhängig voneinander -H oder -F, 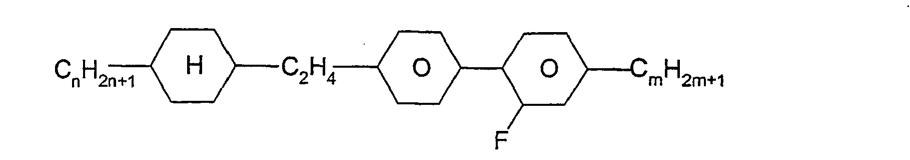 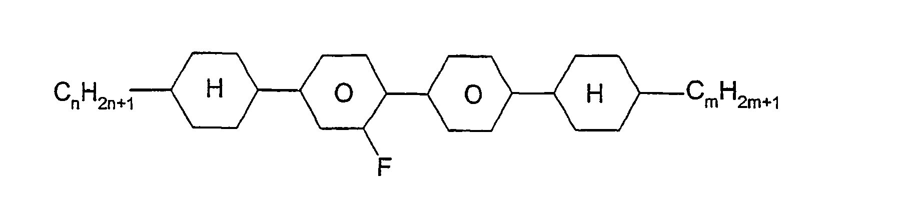 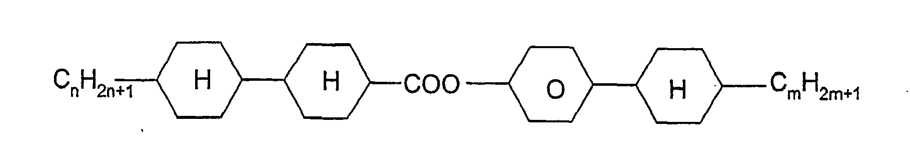 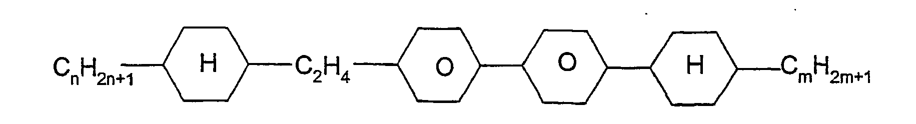 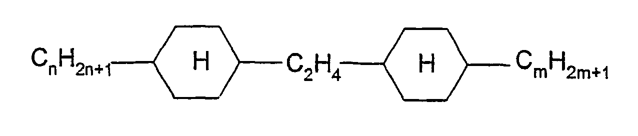 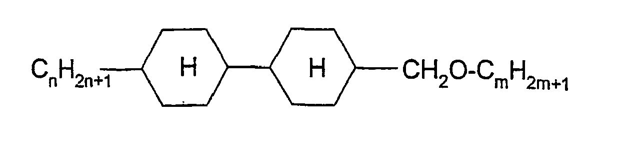 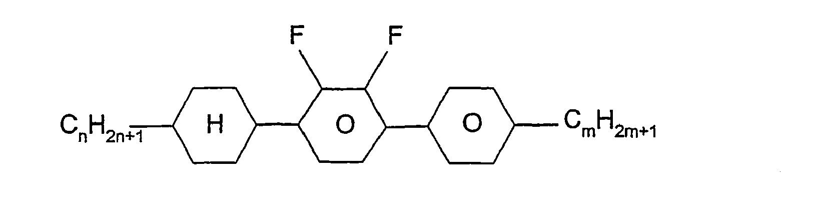 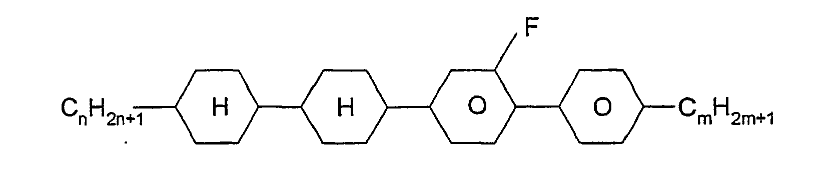 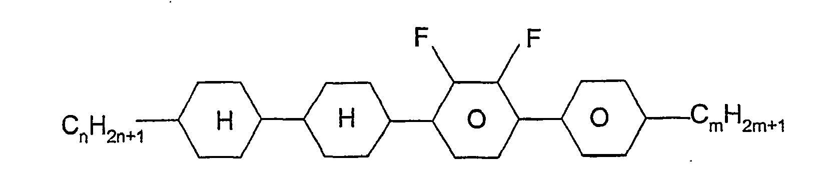 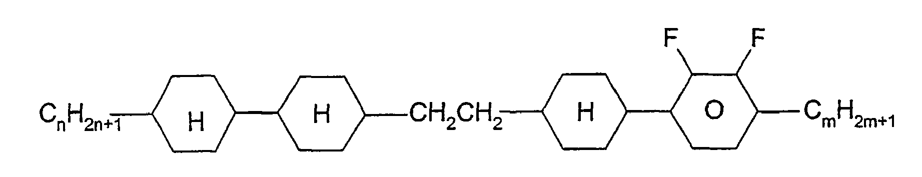 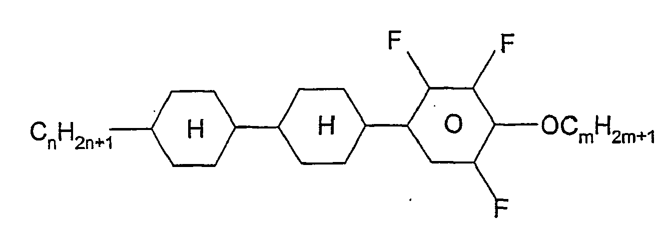 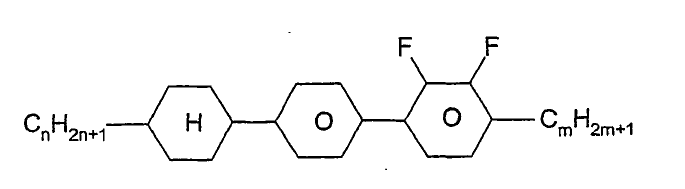 mit m, n unabhängig voneinander 1 bis 8.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen der vorliegenden Erfindung so modifiziert werden, dass sie in allen bisher bekannt gewordenen Arten von Flüssigkristallanzeigenelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formeln (II) bis (VI) eignen sich wegen ihres negativen Δε insbesondere für eine Verwendung in VA-TFT-Displays.

Gegenstand der vorliegenden Erfindung sind daher auch elektrooptische Flüssigkristallanzeigeelemente, enthaltend ein erfindungsgemäßes flüssigkristallines Medium.

Die Verbindungen der Formeln (VII) bis (XI) eignen sich wegen ihres hohen positiven Δε insbesondere für die Verwendung in mesogenen Steuermedien, wobei diese Steuermedien insbesondere in elektrooptischen Lichtsteuerelementen eingesetzt werden, die bei einer Temperatur betrieben werden, bei der das mesogene Steuermedium im nicht angesteuerten Zustand in der isotropen Phase vorliegt.

Gegenstand der vorliegenden Erfindung sind daher auch elektrooptische Lichtssteuerelemente, wie zum Beispiel in der DE 102 17 273 A1 offenbart, die eine Elektrodenanordnung, mindestens ein Element zur Polarisation des Lichts und ein mesogenes Steuermedium enthalten, wobei das Lichtsteuerelement bei einer Temperatur betrieben wird, bei der das mesogene Steuermedium im nicht angesteuerten Zustand in der isotropen Phase vorliegt, und die dadurch gekennzeichnet sind, dass das mesogene Steuermedium eine oder mehrere Verbindungen der Formeln (VII) bis (XI) enthält.

Die Erfindung wird im folgenden anhand von Ausführungsbeispielen näher erläutert, ohne dadurch jedoch eingeschränkt zu werden.

### Beispiele

Die Ausgangssubstanzen können nach allgemein zugänglichen Literaturvorschriften oder käuflich erhalten werden. Die beschriebenen Reaktionen sind literaturbekannt (Beispiele 1-10).

### A) Herstellung der Naphthalin-Derivate

### Beispiel 1

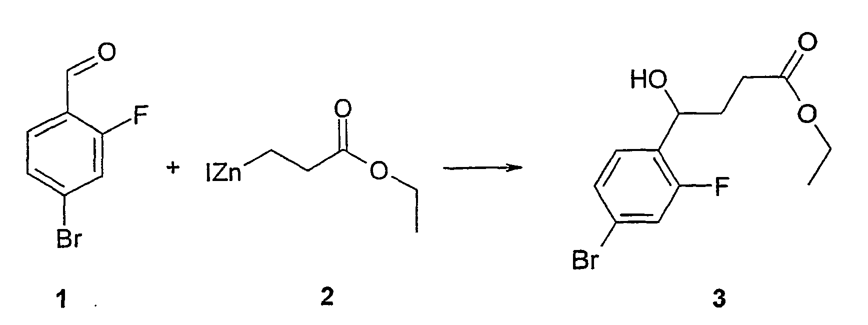

Bei -75°C wird eine Lösung von 20,0 g (98,5 mmol) des Aldehyds **1** in 100 ml THF mit 200 ml (100 mmol) einer 0,5 M Lösung der Zinkverbindung **2** in THF versetzt. Nach 30 Minuten wird die Kühlung entfernt. Der aufgetaute Ansatz wird mit Wasser versetzt, mit 1 N HCl-Lösung angesäuert und mit tert. Butylmethyl (MTB)-Ether extrahiert. Nach Trocknen, Einengen und Chromatographie an Kieselgel erhält man den Hydroxyester **3.**

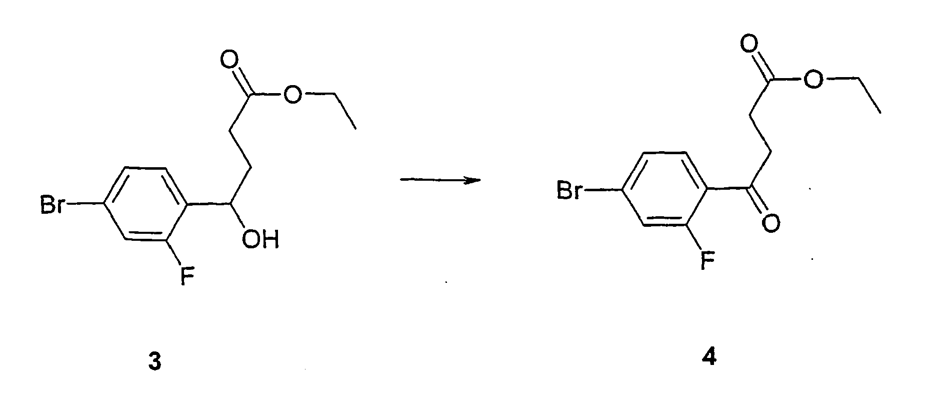

Eine Suspension von 40,0 mmol Pyridiniumchlorochromat (PCC) auf 50 g Celite^{®} in 150 ml Dichlormethan wird bei Raumtemperatur mit einer Lösung von 10,0 g (32,7 mmol) des Hydroxyesters **3** versetzt. Nach Beendigung der Reaktion (DC) wird der Ansatz filtriert und der Filterkuchen mit Methylenchlorid gewaschen. Nach Einengen und Chromatographie an Kieselgel erhält man den Ketoester **4**. 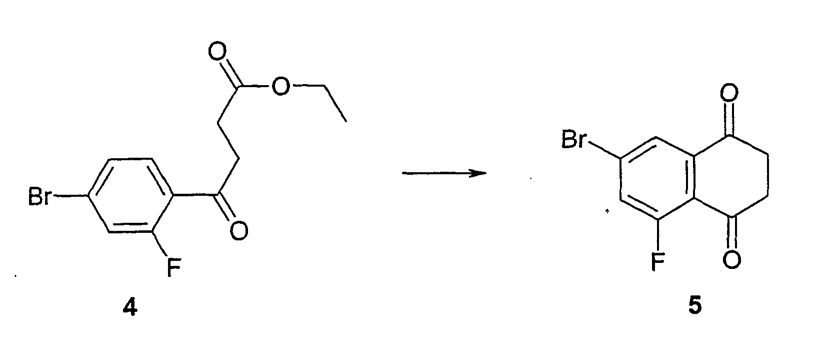 9,0 g (29,7 mmol) des Ketoesters **4** werden bei 60°C in 100 g Polyphosphorsäure gegeben. Anschließend wird die Temperatur für 4 Stunden auf 120°C erhöht. Nach dem Abkühlen wird der Ansatz auf Eis gegeben und mit tert. Butylmethyl (MTB)-Ether extrahiert. Nach Trocknen, Einengen und Kristallisation erhält man das Diketon **5**. 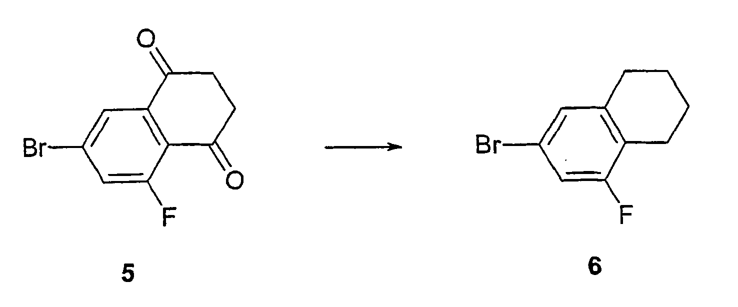 5,0 g (19,5 mmol) des Diketons **5** werden in 5 ml Ethanol gelöst, mit 3 ml 100 %igem Hydraziniumhydroxid und 0,5 ml Wasser versetzt und 30 Minuten unter Rückfluss erhitzt. Anschließend wird eine Lösung von 800 mg Natrium in 15 ml Ethanol in das Reaktionsgefäß gegeben. Der Ansatz wird bis zum Ende der Stickstoffentwicklung auf 140°C erhitzt. Anschließend wird 2/3 des Ethanols abdestilliert. Der Rückstand wird mit 50 ml Wasser verdünnt und mit Ether extrahiert. Der Extrakt wird mit 10 %iger KOH-, 5 %iger HCl- und 30 %iger Natriumhydrogensufit-Lösung gewaschen. Nach Trocknen, Einengen und Chromatographie an Kieselgel erhält man das Tetrahydronaphthalin **6**.

### Beispiel 2

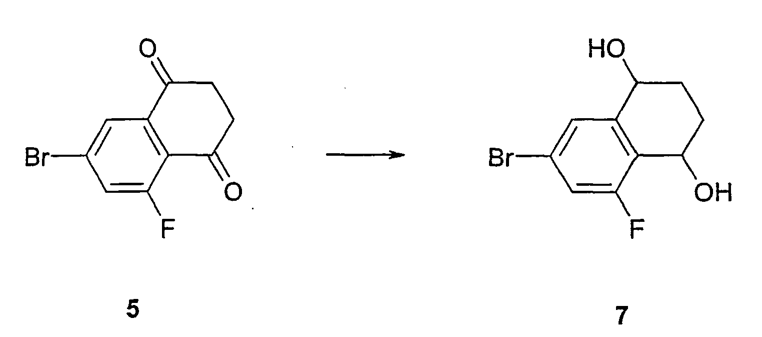

8,0 g (31,1 mmol) des Diketons **5** werden in 150 ml Ethanol gelöst und portionsweise mit 2,4 g (65,0 mmol) Natriumborhydrid versetzt. Nach Beendigung der Reaktion (DC) wird der Ansatz mit Wasser hydrolysiert, das Ethanol im Vakuum entfernt, der Rückstand in Wasser aufgenommen und mit Toluol extrahiert. Nach dem Einengen wird das Produkt ohne weitere Reinigung in der nächsten Stufe eingesetzt. 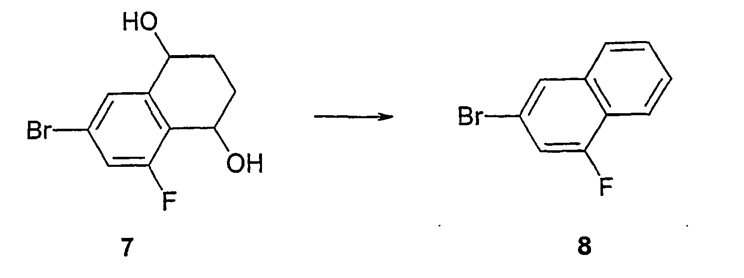 10,0 g (38,3 mmol) des Diols 7 werden in 200 ml Toluol gelöst, mit 1 g p-Toluolsulfonsäure versetzt und bis zur Beendigung der Wasserabscheidung unter Rückfluss erhitzt. Nach Einengen und Filtration über Kieselgel erhält man das Naphthalinderivat **8.**

### Beispiel 3

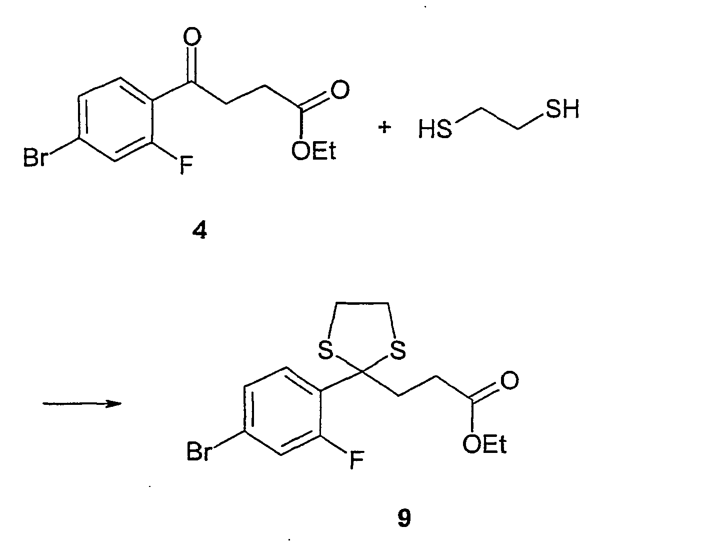

Eine Lösung von 15,0 g (49,5 mmol) des Ketoesters **4** und 8,4 ml (100 mmol) des Dithiols in 150 ml Dichlormethan wird unter Stickstoff mit 30 ml Bortrifluorid-Diethylether-Komplex versetzt und über Nacht gerührt. Der Ansatz wird langsam in gesättigte Natriumhydrogencarbonatlösung gegeben und entsäuert. Nach Trocknen, Einengen und Chromatographie an Kieselgel erhält man das geschützte Keton **9.**

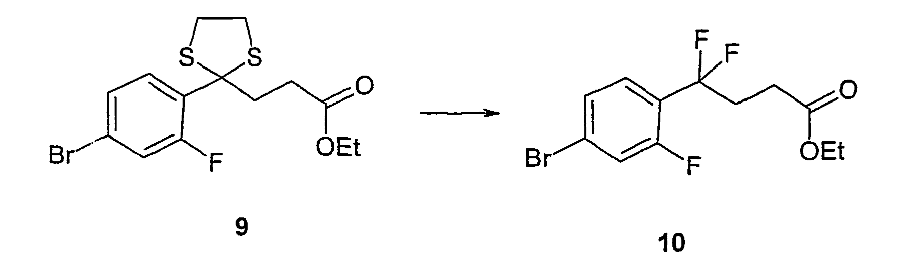

Eine Lösung von 10 g (26,4 mmol) des geschützten Ketons **9** in 60 ml Dichlormethan wird bei -75°C in eine Suspension von 30,2 g (105,2 mmol) 1,3-Dibrom-5,5-dimethylhydantoin in 60 ml Dichlormethan und 120 ml einer 65%igen Lösung von Fluorwasserstoff in Pyridin gegeben. Der Ansatz wird 3 Stunden langsam auf 0°C erwärmt und in 1500 ml einer eisgekühlten 2N Natronlauge gegeben, die mit 120 ml einer 39%igen Natriumhydrogensulfitlösung versetzt worden ist. Der pH wird auf 8 eingestellt und die wässrige Phase mit Methylenchlorid extrahiert. Nach Trocknen, Einengen und Chromatographie an Kieselgel erhält man den fluorierten Ester **10.**

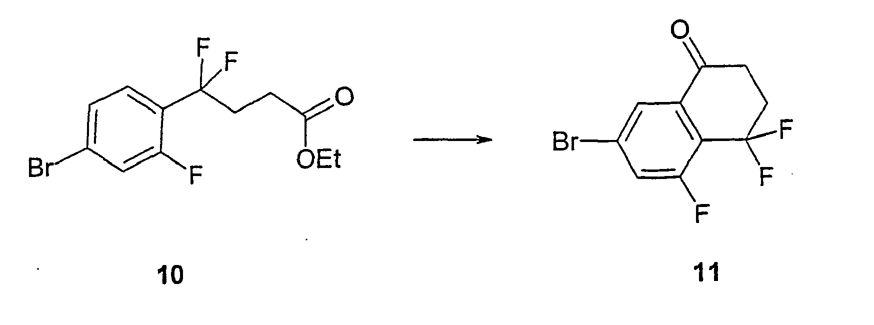

Der Ringschluss des fluorierten Esters **10** zur Verbindung **11** erfolgt, wie in Beispiel 1 beschrieben.

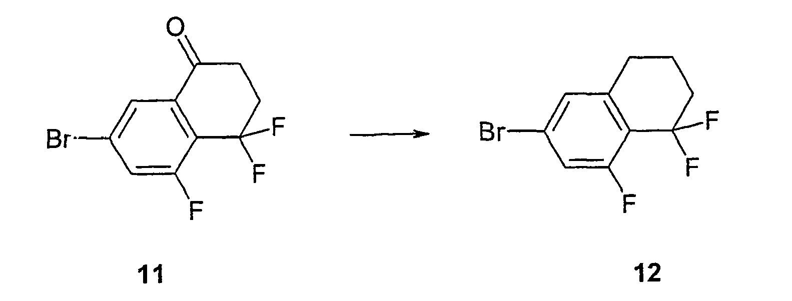

Die Reduktion zum Alkan **12** erfolgt, wie in Beispiel 1 beschrieben.

### Beispiel 4

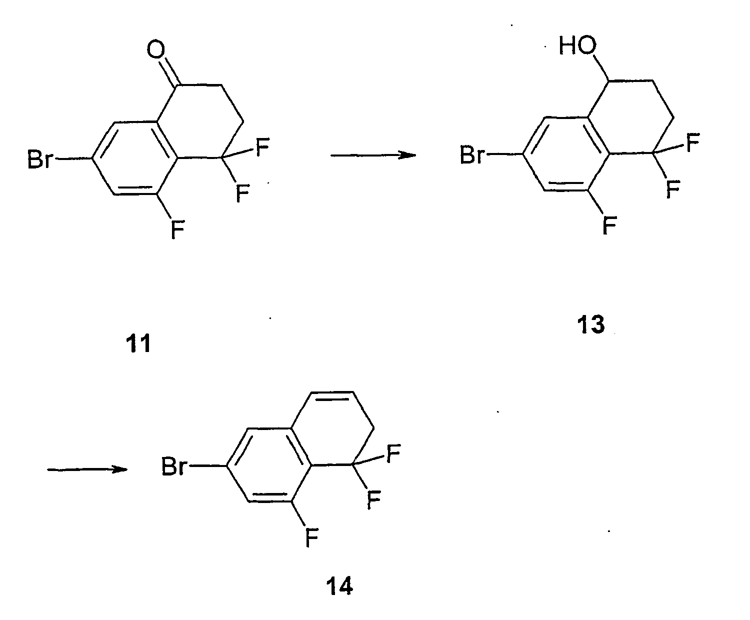

Die Reduktion der Verbindung **11** zum Alkohol **13** und die anschließende Wasserabspaltung zum Dihydronaphthalinderivat **14** erfolgen, wie in Beispiel 2 beschrieben.

### Beispiel 5

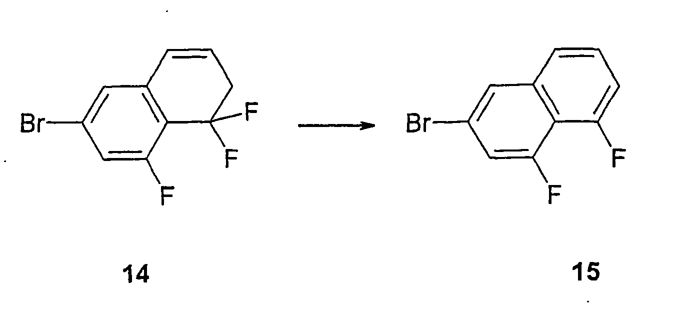

Eine Lösung von 9 g (34,2 mmol) des Dihydronaphthalinderivats **14** in 50 ml THF wird langsam in eine Suspension von 4,5 g (40,1 mmol) Kalium-tert.-butylat in 50 ml THF gegeben und anschließend über Nacht unter Rückfluss erhitzt. Der abgekühlte Ansatz wird mit Wasser verdünnt und mit Diethylether extrahiert. Nach Trocknen, Einengen und Chromatographie an Kieselgel erhält man das Naphthalin **15.**

### Beispiel 6

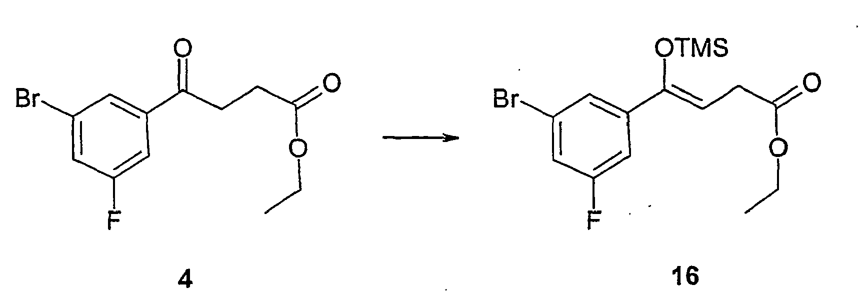

Bei -78°C wird eine Lösung von 6,8 g (21,4 mmol) des Ketoesters **4** in 80 ml THF mit 22 ml einer 2M Lithiumdiisopropylamid (LDA)-Lösung versetzt. Nach 1 Stunde werden 2,6 g (24,0 mmol) Chlortrimethylsilan hinzugefügt. Nach dem Auftauen werden die Lösungsmittel im Vakuum entfernt und der Rückstand ohne weitere Reinigung in der Folgestufe eingesetzt.

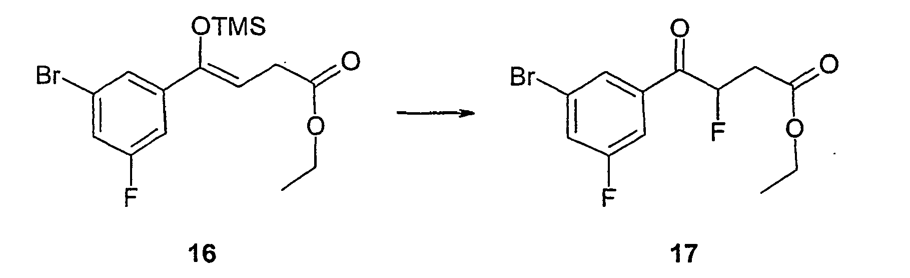

Eine Lösung von 5 g des rohen Enolethers **16** wird mit 4,9 g (19,8 mmol) N-Fluorpyridinium-triflat versetzt und über Nacht unter Rückfluss erhitzt. Das Lösungsmittel wird im Vakuum entfernt und der Rückstand mittels Chromatographie an Kieselgel gereinigt. Man erhält das fluorierte Produkt **17**.

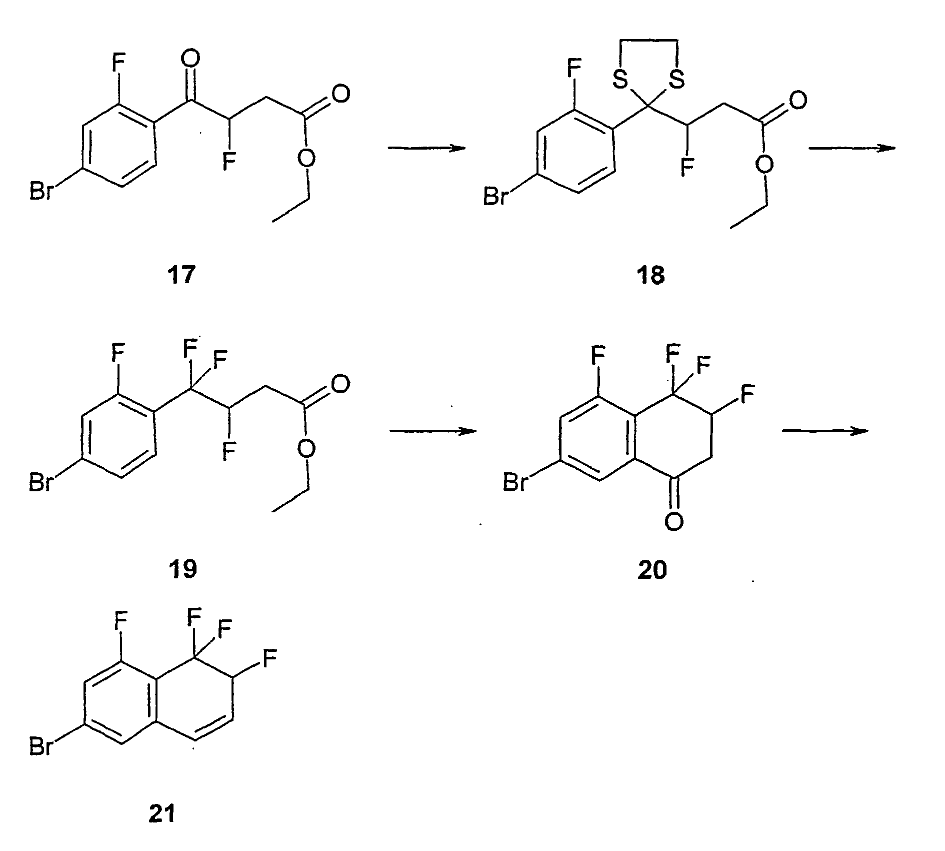

Die Umsetzungen vom fluorierten Produkt **17** bis hin zum Dihydronaphthalinderivat **21** werden wie in den Beispielen 3 und 4 bereits beschrieben durchgeführt.

### Beispiel 7

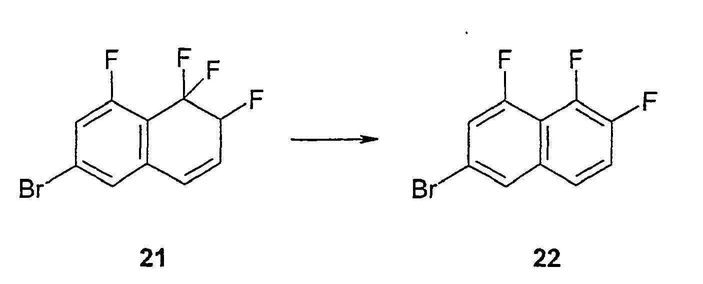

Die Umsetzung vom Dihydronaphthalinderivat **21** zum Naphthalinderivat **22** wird wie im Beispiel 5 bereits beschrieben durchgeführt.

### Beispiel 8

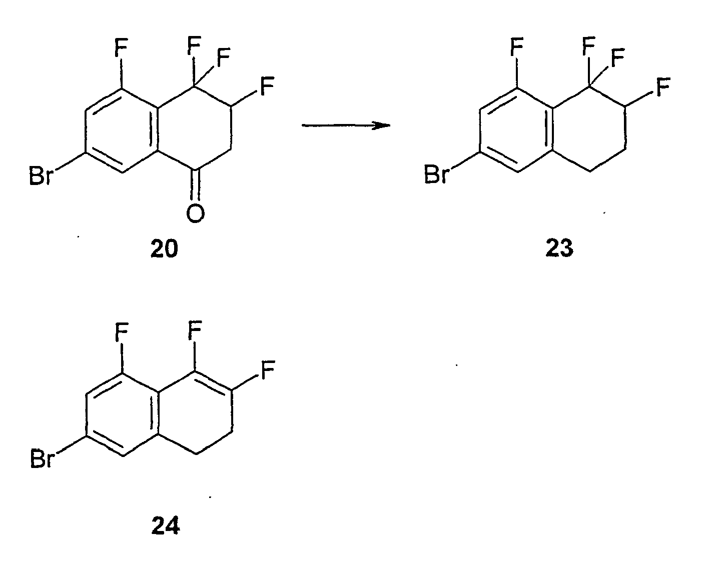

Die Umsetzung von der Verbindung **20** bis zum Dihydronaphthalinderivat **24** wird wie in den Beispielen 4 und 5 bereits beschrieben durchgeführt.

### Beispiel 9

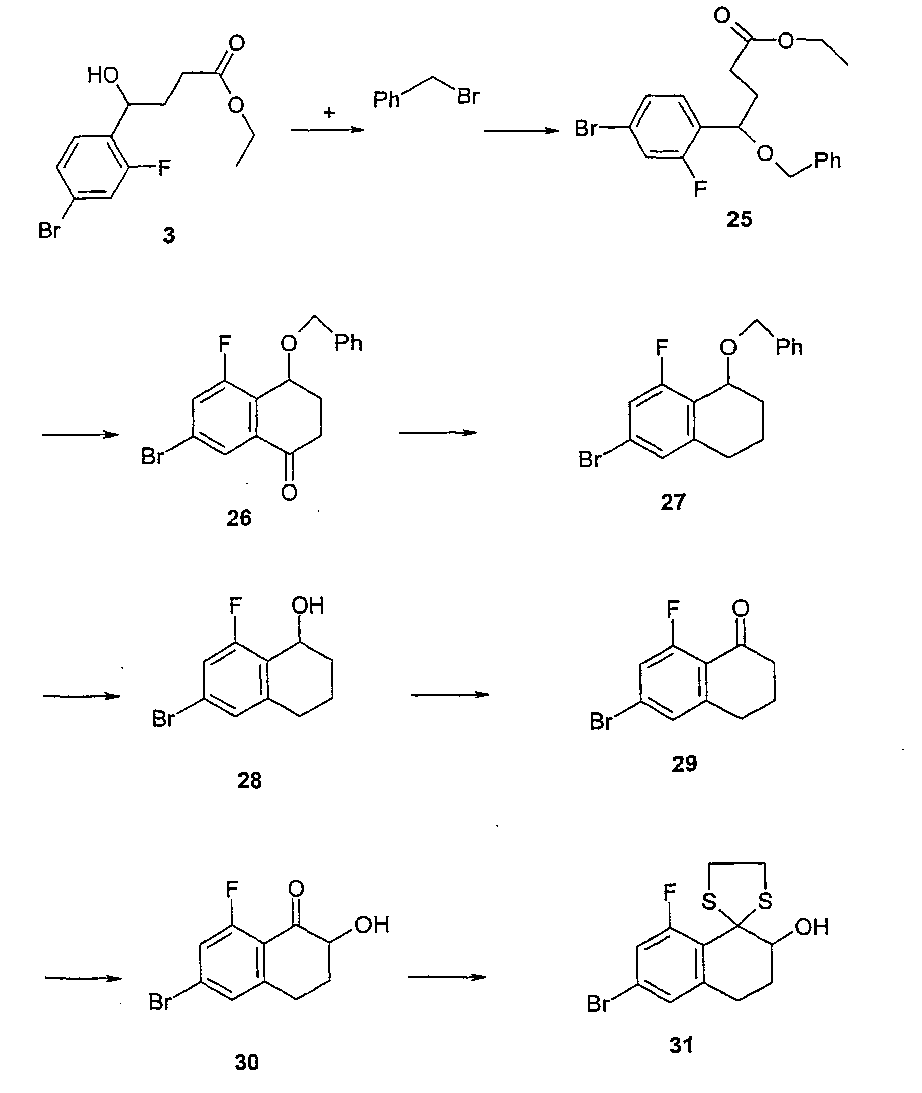

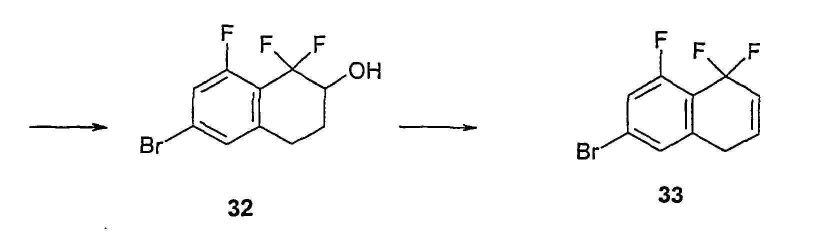

Der Hydroxyester **3** wird in Dimethylformamid (DMF) bei 120°C vier Stunden in Gegenwart von Kaliumcarbonat mit Benzylbromid umgesetzt. Nach dem Abkühlen wird der Ansatz auf Eiswasser gegeben und mit tert. Butylmethyl (MTB)-Ether extrahiert. Nach Trocknen, Einengen und Kristallisation erhält man den Ester **25.**

Die Umsetzung des Esters **25** zum Keton **26** sowie dessen Reduktion zum Ether **27** erfolgt, wie in Beispiel 1 beschrieben.

Der in THF gelöste Ether **27** wird an Palladium/Kohle-Katalysator in Wasserstoffatmosphäre umgesetzt. Nach Einengen und Chromatographie an Kieselgel erhält man die Hydroxyverbindung **28.**

Die Umsetzung der Hydroxyverbindung **28** zum Keton **29** erfolgt, wie in Beispiel 1 beschrieben.

Das Keton **29** wird in Methanol bei 0 bis 20°C vier Stunden mit Jodbenzoldiacetat und KOH umgesetzt. Man erhält das Hydroxyketon **30.**

Die Umsetzung des Hydroxyketons **30** zum Dithiolan **31** sowie dessen Umsetzung zur fluorierten Hydroxyverbindung **32** erfolgt, wie in Beispiel 3 beschrieben.

Die fluorierte Hydroxyverbindung **32** wird unter Eiskühlung mit Pyridin und POCl₃ gemischt. Anschließend wird Alkohol hinzugegeben. Die Umsetzung erfolgt vier Stunden bei 60°C. Nach dem Abkühlen wird der Ansatz auf Eiswasser gegeben und mit tert. Butylmethyl (MTB)-Ether extrahiert. Nach Trocknen, Einengen und Kristallisation erhält man die ungesättigte, fluorierte Verbindung **33.**

### Beispiel 10

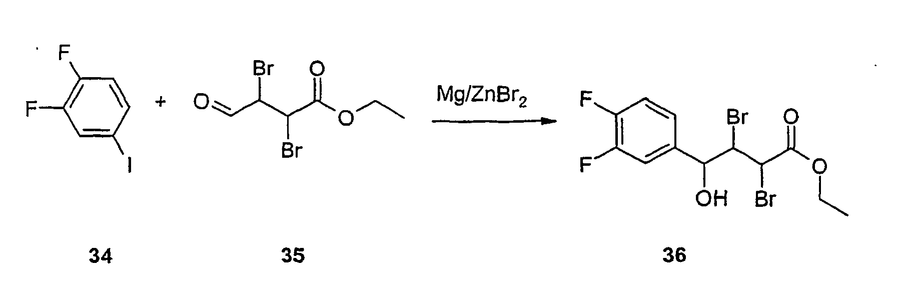

Aus 48,0 g (200 mmol) des Aromaten **34,** 4,8 g (200 mmol) Magnesium und 200 ml Toluol/THF (4:1) wird die entsprechende Grignard-Verbindung hergestellt. Anschliessend werden 22,5 g (100 mmol) Zinkbromid eingetragen. Nach 1 Stunde werden 57,6 g (200 mmol) des Aldehyds **35** in 50 ml Lösungsmittel zugegeben. Nach weiteren 2 Stunden wird der Ansatz mit Wasser versetzt und mit verd. HCl-Lösung angesäuert. Die wässrige Phase wird dreimal mit MTB-Ether extrahiert. Nach Trocknen, Einengen und Chromatographie erhält man 60,4 g des Esters **36.**

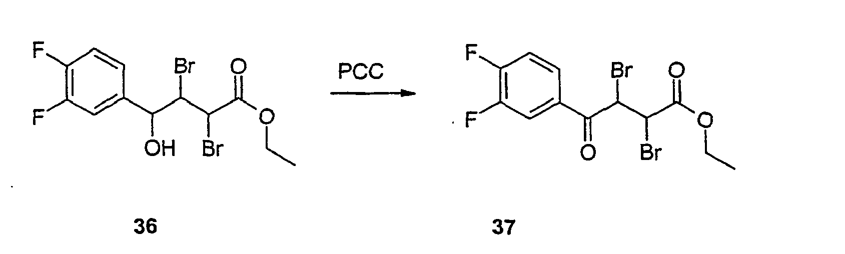

Eine Lösung von 50 g (124 mmol) des Esters **36** in 100 ml Dichlormethan wird bei Raumtemperatur zu einer Suspension von 40,0 g (186 mmol) Pyridiniumchlorochromat (PCC) und 80 g Celite in 300 ml Dichlormethan gegeben und bis zum vollständigen Umsatz (DC) gerührt. Nach Filtration, Einengen und Chromatographie erhält man 47,1 g des Oxoesters **37.** 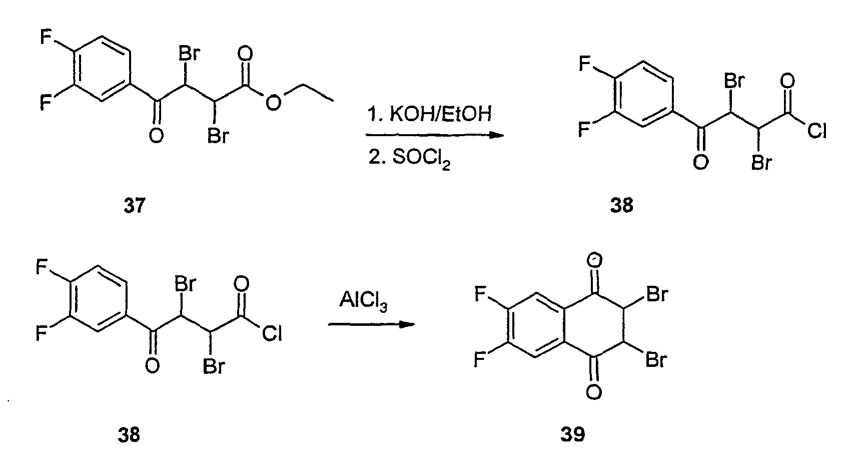 45 g (113 mol) des Esters **37** werden mit einer Lösung von 20 g Kaliumhydroxid in 50 ml Wasser und 150 ml Ethanol 20 Stunden unter Rückfluss erhitzt. Anschliessend wird der Alkohol entfernt, der Rückstand mit Wasser aufgenommen und mit HCl-Lösung angesäuert. Die wässrige Phase wird dreimal mit MTB-Ether extrahiert. Die organische Phase wird getrocknet und eingeengt. Der Rückstand wird mit 20 ml Thionylchlorid versetzt und bis zum Ende der Gasentwicklung unter Rückfluss erhitzt. Überschüssiges Thionylchlorid wird abdestilliert und der Rückstand ohne weitere Reinigung in der nächsten Stufe eingesetzt.

Eine Suspension von 18,0 g (136 mmol) Aluminiumchlorid in 50 ml Dichlormethan wird bei -25°C mit einer Lösung des Säurechlorids **38** in 50 ml Dichlormethan versetzt. Der Ansatz wird bis zum vollständigen Umsatz (DC) bei einer Temperatur unter -12°C gehalten. Anschliessend wird die Reaktion durch vorsichtige Wasserzugabe (50 ml) abgebrochen. Der angefallene Feststoff wird mittels HCl-Lösung in Lösung gebracht. Die wässrige Phase wird zweimal mit Dichlormethan extrahiert, die organische

Phase getrocknet und eingeengt. Nach Chromatographie erhält man 30,2 g des Diketons **39.** 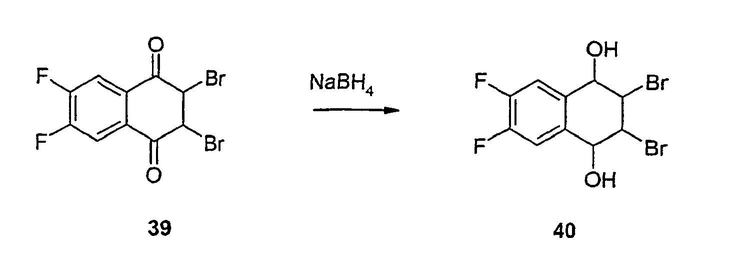 30,0 g (84,8 mmol) des Diketons **39** werden in 150 ml Ethanol gelöst und portionsweise mit 6,3 g (170 mmol) Natriumborhydrid versetzt. Nach Beendigung der Reaktion (DC) wird der Ansatz mit Wasser hydrolysiert, das Ethanol im Vakuum entfernt, der Rückstand in Wasser aufgenommen und mit Toluol extrahiert. Nach dem Einengen wird das Produkt ohne weitere Reinigung in der nächsten Stufe eingesetzt.

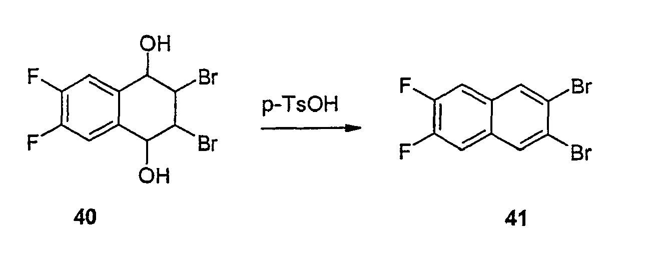

Das rohe Diol **40** wird in 200 ml Toluol gelöst, mit 2 g *p*-Toluolsulfonsäure versetzt und bis zur Beendigung der Wasserabscheidung unter Rückfluss erhitzt. Nach Einengen und Filtration über Kieselgel erhält man 24,7g des Naphthalins **41.**

### B) Herstellung der Cyclopenta[b]naphthalin-Derivate

### Beispiel 11

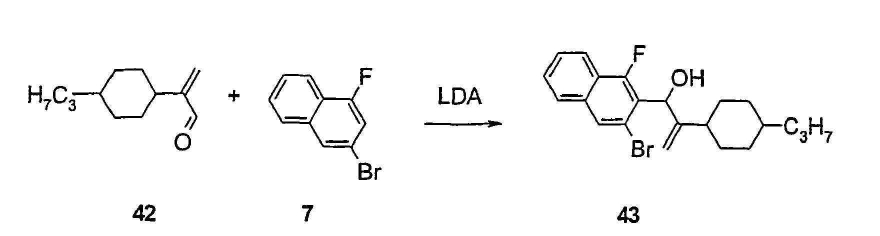

Bei -75°C werden 27,0 ml einer mit 100 ml THF verdünnten Lösung von 2 N Lithiumdiisopropylamid (LDA) in Cyclohexan/Ethylbenzol/THF (52,4 mmol) mit einer Lösung von 13,5 g (60,0 mmol) des Bromfluornaphthalins 7 in 10 ml THF versetzt. Nach 2 Stunden bei der tiefen Temperatur werden 8,5 g (47,3 mmol) des Aldehyds **42** in 10 ml THF hinzugefügt. Nach 30 Minuten wird die Kühlung entfernt, und der Ansatz bei 20°C mit 100 ml 1N HCl versetzt. Nach Extraktion der wässrigen Phase, Trocknen der organischen Phase, Einengen und Chromatographie erhält man den Allylalkohol **43.** 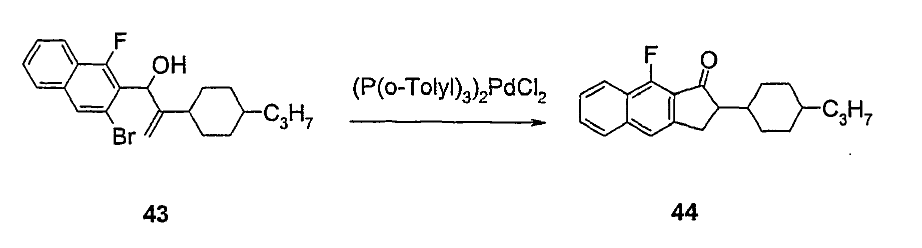 35,0 g (86,6 mmol) des Allylalkohols **43**, 5,5 g Bis(tri-*o*-tolylphosphin)palladiumdichlorid und 50 ml Triethylamin werden in 390 ml Acetonitril gelöst und bis zur vollständigen Umsetzung des Allylalkohols auf 90°C erwärmt. Der erkaltete Ansatz wird auf Wasser gegeben. Nach Extraktion, Trocknen, Einengen und Chromatographie erhält man das Keton **44.**

### Beispiel 12

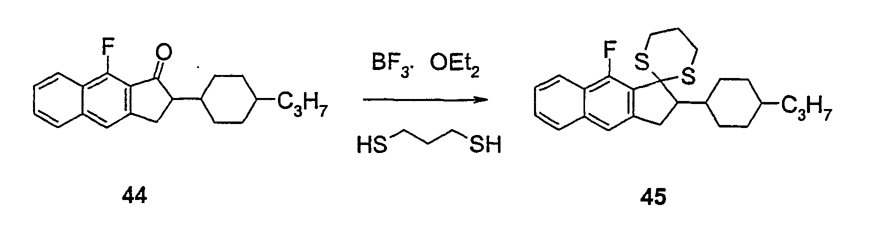

10,0 g (30,8 mmol) des Ketons **44** und 3,2 ml (31,0 mmol) Propandithiol werden in 50 ml Dichlormethan gelöst und bei 6 bis 7°C mit 7,0 ml Bortrifluorid-Diethylether-Komplex versetzt und anschließend über Nacht bei Raumtemperatur gerührt. Der Ansatz wird auf 10 ml gesättigte Natriumhydrogencarbonat-Lösung gegeben und bis zur Beendigung der Gasentwicklung gerührt. Nach Extraktion der wässrigen Phase, Trocknen der organischen Phase, Einengen und Filtration über Kieselgel wird der erhaltene Rückstand ohne weitere Reinigung in der nächsten Stufe eingesetzt. 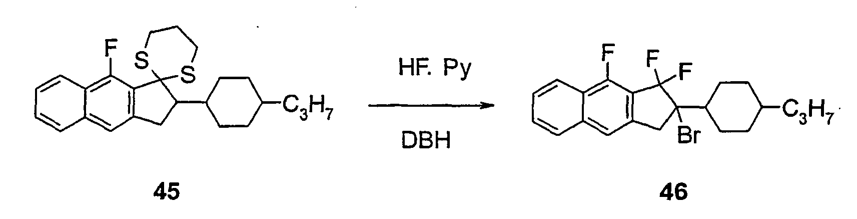 10,0 g des rohen Thioketals **45** gelöst in 30 ml Dichlormethan werden langsam bei -75°C in ein Gemisch aus 28,6 g (100 mmol) 1,3-Dibrom-5,5-dimethylhydantoin (DBH), 80 ml einer 65 %igen Lösung von Fluorwasserstoff in Pyridin und 50 ml Dichlormethan gegeben. Anschließend wird der Ansatz über Nacht bei Raumtemperatur gerührt. Das Reaktionsgemisch wird in eisgekühlte Hydrogensulfit-Lösung gegeben und mit gesättigter Natriumhydrogencarbonat-Lösung und Natronlauge entsäuert. Nach Extraktion, Trocknen, Einengen, erneutem Waschen mit Wasser, Chromatographie und Kristallisation aus Hexan erhält man das Cyclopenta[b]naphthalinderivat **46**. 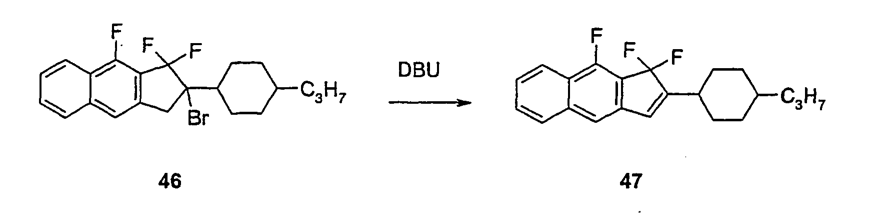 6,0 g (14,1 mmol) des Cyclopenta[b]naphthalinderivats **46** werden in 50 ml Dichlormethan gelöst, mit 2,4 ml (16,0 mmol) 1,8-Diazabicyclo[5.4.0]-undec-7-en (DBU) versetzt und bei Raumtemperatur gerührt, bis das Edukt vollständig umgesetzt ist. Der Ansatz wird mit Wasser und gesättigter Natriumchlorid-Lösung gewaschen, eingeengt und chromatographiert. Es wird das Cyclopenta[b]naphthalinderivat **47** isoliert.

### Beispiel 13

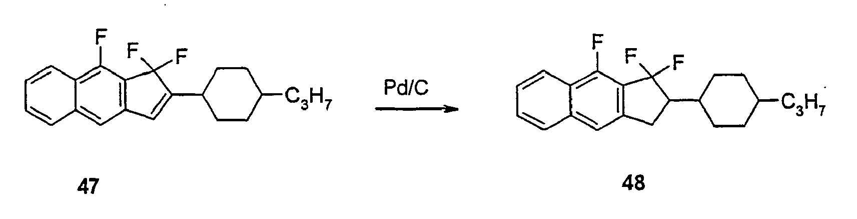

4,0 g (11,6 mmol) des Cyclopenta[b]naphthalinderivats **47** werden in 50 ml THF gelöst und bei Raumtemperatur und Normaldruck am Palladiumkatalysator hydriert. Nach Einengen, Chromatographie an Kieselgel und Kristallisation erhält man das Cyclopenta[b]naphthalinderivat **48.**

Die Δn- und Δε-Werte der erfindungsgemäßen Verbindung wurden durch Extrapolation aus flüssigkristallinen Mischungen erhalten, die zu 5 % aus der erfindungsgemäßen Verbindung und zu 95 % aus einer der beiden kommerziell erhältlichen Flüssigkristallmischungen ZLI 4792 bzw. ZLI 2857 (Fa. Merck, Darmstadt) bestanden.
- Δn:: 0,1418 (ZLI 4792, 589 nm, 20°C)
- Δε:: -4,9 (ZLI 2857, 1 kHz, 20°C)
- Klärpunkt:: 158,6°C (ZLI 4792)

### Beispiel 14

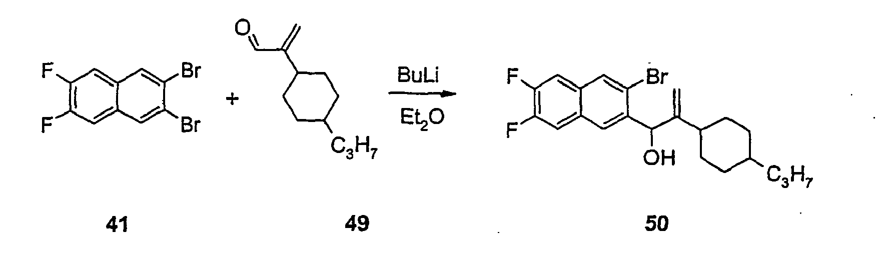

Eine Lösung von 20,0 g (62,1 mmol) des Naphthalins **41** in 100 ml Diethylether wird bei -75°C mit 38,0 ml einer n-Butyllithium-Lösung in n-Hexan versetzt und 1 Stunde gerührt. Anschliessend werden 11,2 g ( 62,1 mmol) des Aldehyds **49** in 50 ml Diethylether hinzugegeben und über Nacht gerührt. Der Ansatz wird mit Wasser versetzt. Die wässrige Phase wird mit Diethylether extrahiert, die organische Phase getrocknet und eingeengt. Nach Chromatographie erhält man 21,2 g des Allylalkohols 50. 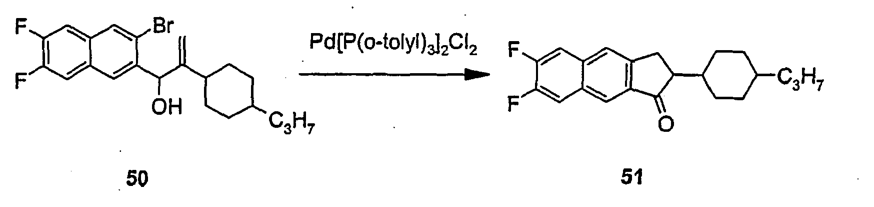 20,0 g (47,2 mmol) des Allylalkohols **50** werden in 175 ml Acetonitril und 25 ml Triethylamin gelöst, mit 2,5 g Bis-tri-o-tolylphosphinpalladium(II)-chlorid versetzt und bis zum Verschwinden des Ausgangsmaterials (HPLC) auf 90°C erwärmt. Anschliessend wird der Ansatz auf gesättigte Natriumchloridlösung gegeben. Nach Extraktion mit MTB-Ether, Trocknen, Einengen und Chromatographie an Kieselgel erhält man 10,5 g des Ketons **51**. 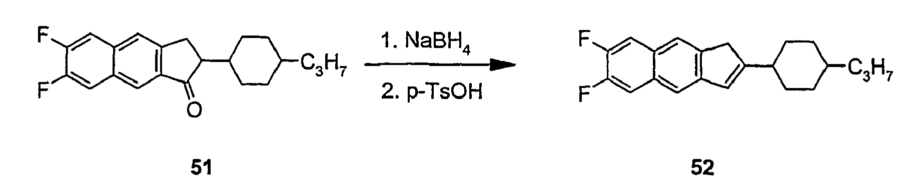 10,0 g (29,2 mmol) des Ketons **51** werden in 75 ml Ethanol gelöst und portionsweise mit 3,2 g (86 mmol) Natriumborhydrid versetzt. Nach Beendigung der Reaktion (DC) wird der Ansatz mit Wasser hydrolysiert, das Ethanol im Vakuum entfernt, der Rückstand in Wasser aufgenommen und mit Toluol extrahiert. Nach dem Einengen wird das Produkt ohne weitere Reinigung in der nächsten Stufe eingesetzt. Der rohe Alkohol wird in 100 ml Toluol gelöst, mit 1 g p-Toluolsulfonsäure versetzt und bis zur Beendigung der Wasserabscheidung unter Rückfluss erhitzt. Nach Einengen und Filtration über Kieselgel erhält man 8,5 des Naphthalins **52.** 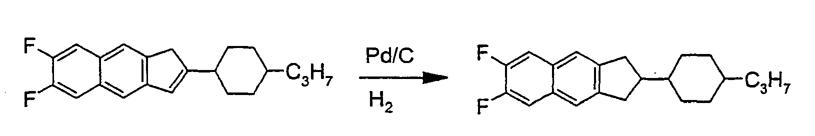 8,0 g (25,5 mmol) des Naphthalins **52** werden in 50 ml THF gelöst und am Palladiumkatalysator hydriert. Nach Einengen und Chromatographie an Kieselgel erhält man 7,9 g der hydrierten Substanz **53.**

In Analogie zu den Beispielen 1 bis 14 oder in Analogie zu bekannten Syntheseschritten werden die folgenden Verbindungen hergestellt:

### Beispiele 15 bis 29

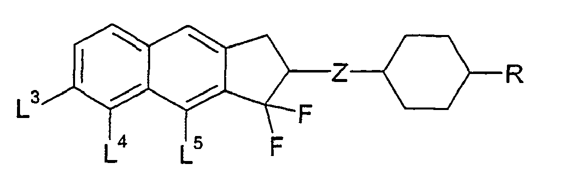

| **Beispiel** | **L³** | **L⁴** | **L⁵** | **Z** | **R** |
|---|---|---|---|---|---|
| 15 | H | H | F | Bd. | CH₃ |
| 16 | H | F | F | Bd. | C₂H₅ |
| 17 | H | F | F | Bd. | C₃H₇ |
| 18 | F | F | F | Bd. | C₃H₇ |
| 19 | F | F | F | Bd. | C₄H₉ |
| 20 | OC₂H₅ | F | F | Bd. | C₃H₇ |
| 21 | OC₂H₅ | F | F | Bd. | C₅H₁₁ |
| 22 | H | H | F | -CF₂CF₂- | CH₃ |
| 23 | H | H | F | -CF₂CF₂- | C₃H₇ |
| 24 | H | F | F | -CF₂CF₂- | C₂H₅ |
| 25 | H | F | F | -CF₂CF₂- | C₃H₇ |
| 26 | F | F | F | -CF₂CF₂- | C₃H₇ |
| 27 | F | F | F | -CF₂CF₂- | C₄H₉ |
| 28 | OC₂H₅ | F | F | -CF₂CF₂- | C₃H₇ |
| 29 | OC₂H₅ | F | F | -CF₂CF₂- | C₅H₁₁ |

| | | | | | |
|---|---|---|---|---|---|
| Bd. = Einfachbindung | | | | | |

### Beispiele 30 bis 53

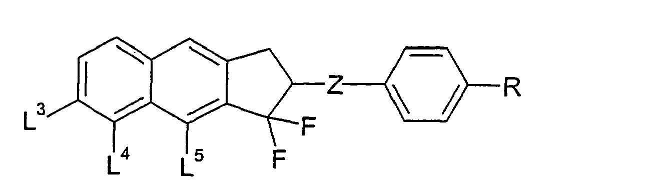

| **Beispiel** | **L³** | **L⁴** | **L⁵** | **Z** | **R** |
|---|---|---|---|---|---|
| 30 | H | H | F | Bd. | CH₃ |
| 31 | H | H | F | Bd. | C₃H₇ |
| 32 | H | F | F | Bd. | C₂H₅ |
| 33 | H | F | F | Bd. | C₃H₇ |
| 34 | F | F | F | Bd. | C₃H₇ |
| 35 | F | F | F | Bd. | C₄H₉ |
| 36 | OC₂H₅ | F | F | Bd. | C₃H₇ |
| 37 | OC₂H₅ | F | F | Bd. | C₅H₁₁ |
| 38 | H | H | F | -CF₂O- | CH₃ |
| 39 | H | H | F | -CF₂O- | C₃H₇ |
| 40 | H | F | F | -CF₂O- | C₂H₅ |
| 41 | H | F | F | -CF₂O- | C₃H₇ |
| 42 | F | F | F | -CF₂O- | C₃H₇ |
| 43 | F | F | F | -CF₂O- | C₄H₉ |
| 44 | OC₂H₅ | F | F | -CF₂O- | C₃H₇ |
| 45 | OC₂H₅ | F | F | -CF₂O- | C₅H₁₁ |
| 46 | H | H | F | -CF₂CF₂- | CH₃ |
| 47 | H | H | F | -CF₂CF₂- | C₃H₇ |
| 48 | H | F | F | -CF₂CF₂- | C₂H₅ |
| 49 | H | F | F | -CF₂CF₂- | C₃H₇ |
| 50 | F | F | F | -CF₂CF₂- | C₃H₇ |
| 51 | F | F | F | -CF₂CF₂- | C₄H₉ |
| 52 | OC₂H₅ | F | F | -CF₂CF₂- | C₃H₇ |
| 53 | OC₂H₅ | F | F | -CF₂CF₂- | C₅H₁₁ |

| | | | | | |
|---|---|---|---|---|---|
| Bd. = Einfachbindung | | | | | |

### Beispiele 54 bis 77

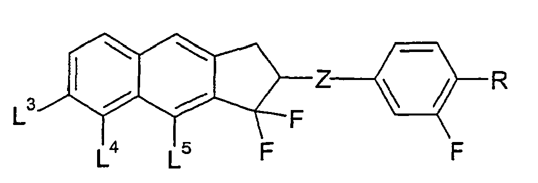

| **Beispiel** | **L³** | **L⁴** | **L⁵** | **Z** | **R** |
|---|---|---|---|---|---|
| 54 | H | H | F | Bd. | CH₃ |
| 55 | H | H | F | Bd. | C₃H₇ |
| 56 | H | F | F | Bd. | C₂H₅ |
| 57 | H | F | F | Bd. | C₃H₇ |
| 58 | F | F | F | Bd. | C₃H₇ |
| 59 | F | F | F | Bd. | C₄H₉ |
| 60 | OC₂H₅ | F | F | Bd. | C₃H₇ |
| 61 | OC₂H₅ | F | F | Bd. | C₅H₁₁ |
| 62 | H | H | F | -CF₂O- | CH₃ |
| 63 | H | H | F | -CF₂O- | C₃H₇ |
| 64 | H | F | F | -CF₂O- | C₂H₅ |
| 65 | H | F | F | -CF₂O- | C₃H₇ |
| 66 | F | F | F | -CF₂O- | C₃H₇ |
| 67 | F | F | F | -CF₂O- | C₄H₉ |
| 68 | OC₂H₅ | F | F | -CF₂O- | C₃H₇ |
| 69 | OC₂H₅ | F | F | -CF₂O- | C₅H₁₁ |
| 70 | H | H | F | -CF₂CF₂- | CH₃ |
| 71 | H | H | F | -CF₂CF₂- | C₃H₇ |
| 72 | H | F | F | -CF₂CF₂- | C₂H₅ |
| 73 | H | F | F | -CF₂CF₂- | C₃H₇ |
| 74 | F | F | F | -CF₂CF₂- | C₃H₇ |
| 75 | F | F | F | -CF₂CF₂- | C₄H₉ |
| 76 | OC₂H₅ | F | F | -CF₂CF₂- | C₃H₇ |
| 77 | OC₂H₅ | F | F | -CF₂CF₂- | C₅H₁₁ |

| | | | | | |
|---|---|---|---|---|---|
| Bd. = Einfachbindung | | | | | |

### Beispiele 78 bis 93

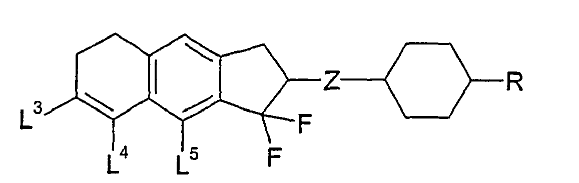

| **Beispiel** | **L³** | **L⁴** | **L⁵** | **Z** | **R** |
|---|---|---|---|---|---|
| 78 | H | H | F | Bd. | CH₃ |
| 79 | H | H | F | Bd. | C₃H₇ |
| 80 | H | F | F | Bd. | C₂H₅ |
| 81 | H | F | F | Bd. | C₃H₇ |
| 82 | F | F | F | Bd. | C₃H₇ |
| 83 | F | F | F | Bd. | C₄H₉ |
| 84 | OC₂H₅ | F | F | Bd. | C₃H₇ |
| 85 | OC₂H₅ | F | F | Bd. | C₅H₁₁ |
| 86 | H | H | F | -CF₂CF₂- | CH₃ |
| 87 | H | H | F | -CF₂CF₂- | C₃H₇ |
| 88 | H | F | F | -CF₂CF₂- | C₂H₅ |
| 89 | H | F | F | -CF₂CF₂- | C₃H₇ |
| 90 | F | F | F | -CF₂CF₂- | C₃H₇ |
| 91 | F | F | F | -CF₂CF₂- | C₄H₉ |
| 92 | OC₂H₅ | F | F | -CF₂CF₂- | C₃H₇ |
| 93 | OC₂H₅ | F | F | -CF₂CF₂- | C₅H₁₁ |

| | | | | | |
|---|---|---|---|---|---|
| Bd. = Einfachbindung | | | | | |

### Beispiele 94 bis 117

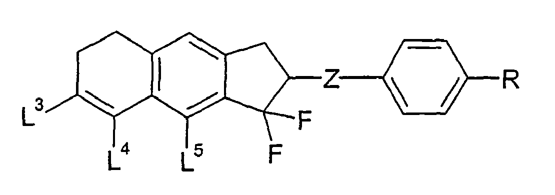

| **Beispiel** | **L³** | **L⁴** | **L⁵** | **Z** | **R** |
|---|---|---|---|---|---|
| 94 | H | H | F | Bd. | CH₃ |
| 95 | H | H | F | Bd. | C₃H₇ |
| 96 | H | F | F | Bd. | C₂H₅ |
| 97 | H | F | F | Bd. | C₃H₇ |
| 98 | F | F | F | Bd. | C₃H₇ |
| 99 | F | F | F | Bd. | C₄H₉ |
| 100 | OC₂H₅ | F | F | Bd. | C₃H₇ |
| 101 | OC₂H₅ | F | F | Bd. | C₅H₁₁ |
| 102 | H | H | F | -CF₂O- | CH₃ |
| 103 | H | H | F | -CF₂O- | C₃H₇ |
| 104 | H | F | F | -CF₂O- | C₂H₅ |
| 105 | H | F | F | -CF₂O- | C₃H₇ |
| 106 | F | F | F | -CF₂O- | C₃H₇ |
| 107 | F | F | F | -CF₂O- | C₄H₉ |
| 108 | OC₂H₅ | F | F | -CF₂O- | C₃H₇ |
| 109 | OC₂H₅ | F | F | -CF₂O- | C₅H₁₁ |
| 110 | H | H | F | -CF₂CF₂- | CH₃ |
| 111 | H | H | F | -CF₂CF₂- | C₃H₇ |
| 112 | H | F | F | -CF₂CF₂- | C₂H₅ |
| 113 | H | F | F | -CF₂CF₂- | C₃H₇ |
| 114 | F | F | F | -CF₂CF₂- | C₃H₇ |
| 115 | F | F | F | -CF₂CF₂- | C₄H₉ |
| 116 | OC₂H₅ | F | F | -CF₂CF₂- | C₃H₇ |
| 117 | OC₂H₅ | F | F | -CF₂CF₂- | C₅H₁₁ |

| | | | | | |
|---|---|---|---|---|---|
| Bd. = Einfachbindung | | | | | |

### Beispiele 118 bis 141

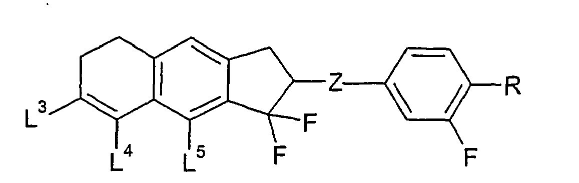

| **Beispiel** | **L³** | **L⁴** | **L⁵** | **Z** | **R** |
|---|---|---|---|---|---|
| 118 | H | H | F | Bd. | CH₃ |
| 119 | H | H | F | Bd. | C₃H₇ |
| 120 | H | F | F | Bd. | C₂H₅ |
| 121 | H | F | F | Bd. | C₃H₇ |
| 122 | F | F | F | Bd. | C₃H₇ |
| 123 | F | F | F | Bd. | C₄H₉ |
| 124 | OC₂H₅ | F | F | Bd. | C₃H₇ |
| 125 | OC₂H₅ | F | F | Bd. | C₅H₁₁ |
| 126 | H | H | F | -CF₂O- | CH₃ |
| 127 | H | H | F | -CF₂O- | C₃H₇ |
| 128 | H | F | F | -CF₂O- | C₂H₅ |
| 129 | H | F | F | -CF₂O- | C₃H₇ |
| 130 | F | F | F | -CF₂O- | C₃H₇ |
| 131 | F | F | F | -CF₂O- | C₄H₉ |
| 132 | OC₂H₅ | F | F | -CF₂O- | C₃H₇ |
| 133 | OC₂H₅ | F | F | -CF₂O- | C₅H₁₁ |
| 134 | H | H | F | -CF₂CF₂- | CH₃ |
| 135 | H | H | F | -CF₂CF₂- | C₃H₇ |
| 136 | H | F | F | -CF₂CF₂- | C₂H₅ |
| 137 | H | F | F | -CF₂CF₂- | C₃H₇ |
| 138 | F | F | F | -CF₂CF₂- | C₃H₇ |
| 139 | F | F | F | -CF₂CF₂- | C₄H₉ |
| 140 | OC₂H₅ | F | F | -CF₂CF₂- | C₃H₇ |
| 141 | OC₂H₅ | F | F | -CF₂CF₂- | C₅H₁₁ |

| | | | | | |
|---|---|---|---|---|---|
| Bd. = Einfachbindung | | | | | |

### Beispiele 142 bis 157

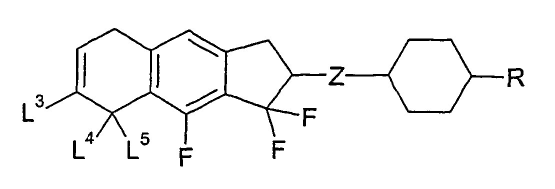

| **Beispiel** | **L³** | **L⁴** | **L⁵** | **Z** | **R** |
|---|---|---|---|---|---|
| 142 | H | H | H | Bd. | CH₃ |
| 143 | H | H | H | Bd. | C₃H₇ |
| 144 | H | F | F | Bd. | C₂H₅ |
| 145 | H | F | F | Bd. | C₃H₇ |
| 146 | F | F | F | Bd. | C₃H₇ |
| 147 | F | F | F | Bd. | C₄H₉ |
| 148 | OC₂H₅ | F | F | Bd. | C₃H₇ |
| 149 | OC₂H₅ | F | F | Bd. | C₅H₁₁ |
| 150 | H | H | H | -CF₂CF₂- | CH₃ |
| 151 | H | H | H | -CF₂CF₂- | C₃H₇ |
| 152 | H | F | F | -CF₂CF₂- | C₂H₅ |
| 153 | H | F | F | -CF₂CF₂- | C₃H₇ |
| 154 | F | F | F | -CF₂CF₂- | C₃H₇ |
| 155 | F | F | F | -CF₂CF₂- | C₄H₉ |
| 156 | OC₂H₅ | F | F | -CF₂CF₂- | C₃H₇ |
| 157 | OC₂H₅ | F | F | -CF₂CF₂- | C₅H₁₁ |

| | | | | | |
|---|---|---|---|---|---|
| Bd. = Einfachbindung | | | | | |

### Beispiele 158 bis 181

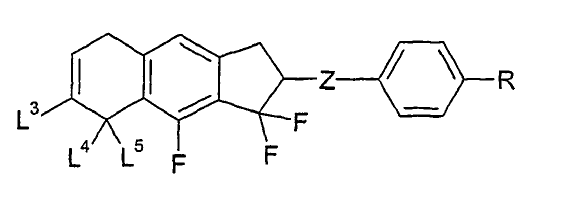

| **Beispiel** | **L³** | **L⁴** | **L⁵** | **Z** | **R** |
|---|---|---|---|---|---|
| 158 | H | H | H | Bd. | CH₃ |
| 159 | H | H | H | Bd. | C₃H₇ |
| 160 | H | F | F | Bd. | C₂H₅ |
| 161 | H | F | F | Bd. | C₃H₇ |
| 162 | F | F | F | Bd. | C₃H₇ |
| 163 | F | F | F | Bd. | C₄H₉ |
| 164 | OC₂H₅ | F | F | Bd. | C₃H₇ |
| 165 | OC₂H₅ | F | F | Bd. | C₅H₁₁ |
| 166 | H | H | H | -CF₂O- | CH₃ |
| 167 | H | H | H | -CF₂O- | C₃H₇ |
| 168 | H | F | F | -CF₂O- | C₂H₅ |
| 169 | H | F | F | -CF₂O- | C₃H₇ |
| 170 | F | F | F | -CF₂O- | C₃H₇ |
| 171 | F | F | F | -CF₂O- | C₄H₉ |
| 172 | OC₂H₅ | F | F | -CF₂O- | C₃H₇ |
| 173 | OC₂H₅ | F | F | -CF₂O- | C₅H₁₁ |
| 174 | H | H | H | -CF₂CF₂- | CH₃ |
| 175 | H | H | H | -CF₂CF₂- | C₃H₇ |
| 176 | H | F | F | -CF₂CF₂- | C₂H₅ |
| 177 | H | F | F | -CF₂CF₂- | C₃H₇ |
| 178 | F | F | F | -CF₂CF₂- | C₃H₇ |
| 179 | F | F | F | -CF₂CF₂- | C₄H₉ |
| 180 | OC₂H₅ | F | F | -CF₂CF₂- | C₃H₇ |
| 181 | OC₂H₅ | F | F | -CF₂CF₂- | C₅H₁₁ |

| | | | | | |
|---|---|---|---|---|---|
| Bd. = Einfachbindung | | | | | |

### Beispiele 182 bis 205

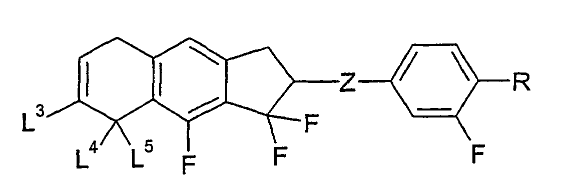

| **Beispiel** | **L³** | **L⁴** | **L⁵** | **Z** | **R** |
|---|---|---|---|---|---|
| 182 | H | H | H | Bd. | CH₃ |
| 183 | H | H | H | Bd. | C₃H₇ |
| 184 | H | F | F | Bd. | C₂H₅ |
| 185 | H | F | F | Bd. | C₃H₇ |
| 186 | F | F | F | Bd. | C₃H₇ |
| 187 | F | F | F | Bd. | C₄H₉ |
| 188 | OC₂H₅ | F | F | Bd. | C₃H₇ |
| 189 | OC₂H₅ | F | F | Bd. | C₅H₁₁ |
| 190 | H | H | H | -CF₂O- | CH₃ |
| 191 | H | H | H | -CF₂O- | C₃H₇ |
| 192 | H | F | F | -CF₂O- | C₂H₅ |
| 193 | H | F | F | -CF₂O- | C₃H₇ |
| 194 | F | F | F | -CF₂O- | C₃H₇ |
| 195 | F | F | F | -CF₂O- | C₄H₉ |
| 196 | OC₂H₅ | F | F | -CF₂O- | C₃H₇ |
| 197 | OC₂H₅ | F | F | -CF₂O- | C₅H₁₁ |
| 198 | H | H | H | -CF₂CF₂- | CH₃ |
| 199 | H | H | H | -CF₂CF₂- | C₃H₇ |
| 200 | H | F | F | -CF₂CF₂- | C₂H₅ |
| 201 | H | F | F | -CF₂CF₂- | C₃H₇ |
| 202 | F | F | F | -CF₂CF₂- | C₃H₇ |
| 203 | F | F | F | -CF₂CF₂- | C₄H₉ |
| 204 | OC₂H₅ | F | F | -CF₂CF₂- | C₃H₇ |
| 205 | OC₂H₅ | F | F | -CF₂CF₂- | C₅H₁₁ |

| | | | | | |
|---|---|---|---|---|---|
| Bd. = Einfachbindung | | | | | |

### Beispiele 206 bis 217

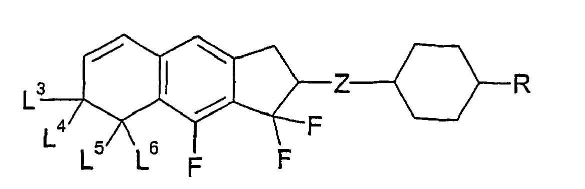

| **Beispiel** | **L³** | **L⁴** | **L⁵** | **L⁶** | **Z** | **R** |
|---|---|---|---|---|---|---|
| 206 | H | H | H | H | Bd. | CH₃ |
| 207 | H | H | H | H | Bd. | C₃H₇ |
| 208 | H | H | F | F | Bd. | C₂H₅ |
| 209 | H | H | F | F | Bd. | C₃H₇ |
| 210 | F | F | F | F | Bd. | C₃H₇ |
| 211 | F | F | F | F | Bd. | C₄H₉ |
| 212 | H | H | H | H | -CF₂CF₂- | CH₃ |
| 213 | H | H | H | H | -CF₂CF₂- | C₃H₇ |
| 214 | H | H | F | F | -CF₂CF₂- | C₂H₅ |
| 215 | H | H | F | F | -CF₂CF₂- | C₃H₇ |
| 216 | F | F | F | F | -CF₂CF₂- | C₃H₇ |
| 217 | F | F | F | F | -CF₂CF₂- | C₄H₉ |

| | | | | | | |
|---|---|---|---|---|---|---|
| Bd. = Einfachbindung | | | | | | |

### Beispiele 218 bis 235

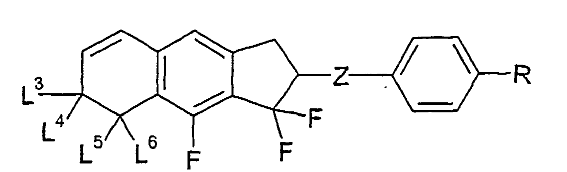

| **Beispiel** | **L³** | **L⁴** | **L⁵** | **L⁶** | **Z** | **R** |
|---|---|---|---|---|---|---|
| 218 | H | H | H | H | Bd. | CH₃ |
| 219 | H | H | H | H | Bd. | C₃H₇ |
| 220 | H | H | F | F | Bd. | C₂H₅ |
| 221 | H | H | F | F | Bd. | C₃H₇ |
| 222 | F | F | F | F | Bd. | C₃H₇ |
| 223 | F | F | F | F | Bd. | C₄H₉ |
| 224 | H | H | H | H | -CF₂O- | CH₃ |
| 225 | H | H | H | H | -CF₂O- | C₃H₇ |
| 226 | H | H | F | F | -CF₂O- | C₂H₅ |
| 227 | H | H | F | F | -CF₂O- | C₃H₇ |
| 228 | F | F | F | F | -CF₂O- | C₃H₇ |
| 229 | F | F | F | F | -CF₂O- | C₄H₉ |
| 230 | H | H | H | H | -CF₂CF₂- | CH₃ |
| 231 | H | H | H | H | -CF₂CF₂- | C₃H₇ |
| 232 | H | H | F | F | -CF₂CF₂- | C₂H₅ |
| 233 | H | H | F | F | -CF₂CF₂- | C₃H₇ |
| 234 | F | F | F | F | -CF₂CF₂- | C₃H₇ |
| 235 | F | F | F | F | -CF₂CF₂- | C₄H₉ |

| | | | | | | |
|---|---|---|---|---|---|---|
| Bd. = Einfachbindung | | | | | | |

### Beispiele 236 bis 253

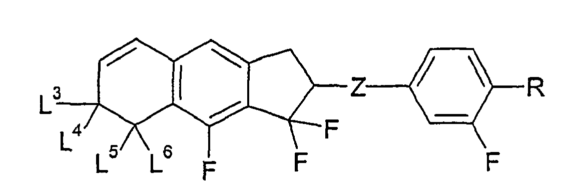

| **Beispiel** | **L³** | **L⁴** | **L⁵** | **L⁶** | **Z** | **R** |
|---|---|---|---|---|---|---|
| 236 | H | H | H | H | Bd. | CH₃ |
| 237 | H | H | H | H | Bd. | C₃H₇ |
| 238 | H | H | F | F | Bd. | C₂H₅ |
| 239 | H | H | F | F | Bd. | C₃H₇ |
| 240 | F | F | F | F | Bd. | C₃H₇ |
| 241 | F | F | F | F | Bd. | C₄H₉ |
| 242 | H | H | H | H | -CF₂O- | CH₃ |
| 243 | H | H | H | H | -CF₂O- | C₃H₇ |
| 244 | H | H | F | F | -CF₂O- | C₂H₅ |
| 245 | H | H | F | F | -CF₂O- | C₃H₇ |
| 246 | F | F | F | F | -CF₂O- | C₃H₇ |
| 247 | F | F | F | F | -CF₂O- | C₄H₉ |
| 248 | H | H | H | H | -CF₂CF₂- | CH₃ |
| 249 | H | H | H | H | -CF₂CF₂- | C₃H₇ |
| 250 | H | H | F | F | -CF₂CF₂- | C₂H₅ |
| 251 | H | H | F | F | -CF₂CF₂- | C₃H₇ |
| 252 | F | F | F | F | -CF₂CF₂- | C₃H₇ |
| 253 | F | F | F | F | -CF₂CF₂- | C₄H₉ |

| | | | | | | |
|---|---|---|---|---|---|---|
| Bd. = Einfachbindung | | | | | | |

### Beispiele 254 bis 265

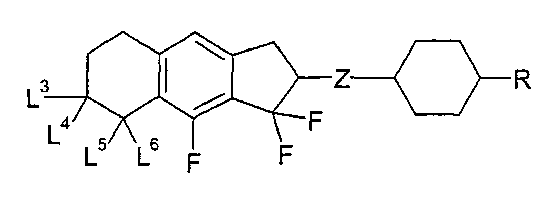

| **Beispiel** | **L³** | **L⁴** | **L⁵** | **L⁶** | **Z** | **R** |
|---|---|---|---|---|---|---|
| 254 | H | H | H | H | Bd. | CH₃ |
| 255 | H | H | H | H | Bd. | C₃H₇ |
| 256 | H | H | F | F | Bd. | C₂H₅ |
| 257 | H | H | F | F | Bd. | C₃H₇ |
| 258 | F | F | F | F | Bd. | C₃H₇ |
| 259 | F | F | F | F | Bd. | C₄H₉ |
| 260 | H | H | H | H | -CF₂CF₂- | CH₃ |
| 261 | H | H | H | H | -CF₂CF₂- | C₃H₇ |
| 262 | H | H | F | F | -CF₂CF₂- | C₂H₅ |
| 263 | H | H | F | F | -CF₂CF₂- | C₃H₇ |
| 264 | F | F | F | F | -CF₂CF₂- | C₃H₇ |
| 265 | F | F | F | F | -CF₂CF₂- | C₄H₉ |

| | | | | | | |
|---|---|---|---|---|---|---|
| Bd. = Einfachbindung | | | | | | |

### Beispiele 266 bis 283

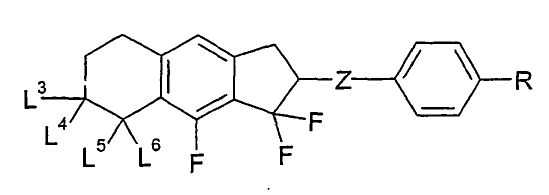

| **Beispiel** | **L³** | **L⁴** | **L⁵** | **L⁶** | **Z** | **R** |
|---|---|---|---|---|---|---|
| 266 | H | H | H | H | Bd. | CH₃ |
| 267 | H | H | H | H | Bd. | C₃H₇ |
| 268 | H | H | F | F | Bd. | C₂H₅ |
| 269 | H | H | F | F | Bd. | C₃H₇ |
| 270 | F | F | F | F | Bd. | C₃H₇ |
| 271 | F | F | F | F | Bd. | C₄H₉ |
| 272 | H | H | H | H | -CF₂O- | CH₃ |
| 273 | H | H | H | H | -CF₂O- | C₃H₇ |
| 274 | H | H | F | F | -CF₂O- | C₂H₅ |
| 275 | H | H | F | F | -CF₂O- | C₃H₇ |
| 276 | F | F | F | F | -CF₂O- | C₃H₇ |
| 277 | F | F | F | F | -CF₂O- | C₄H₉ |
| 278 | H | H | H | H | -CF₂CF₂- | CH₃ |
| 279 | H | H | H | H | -CF₂CF₂- | C₃H₇ |
| 280 | H | H | F | F | -CF₂CF₂- | C₂H₅ |
| 281 | H | H | F | F | -CF₂CF₂- | C₃H₇ |
| 282 | F | F | F | F | -CF₂CF₂- | C₃H₇ |
| 283 | F | F | F | F | -CF₂CF₂- | C₄H₉ |

| | | | | | | |
|---|---|---|---|---|---|---|
| Bd. = Einfachbindung | | | | | | |

### Beispiele 284 bis 301

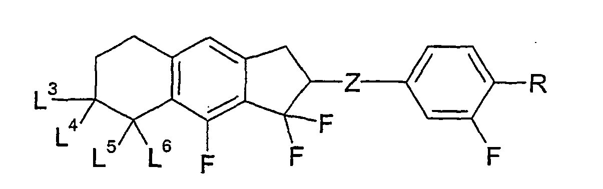

| **Beispiel** | **L³** | **L⁴** | **L⁵** | **L⁶** | **Z** | **R** |
|---|---|---|---|---|---|---|
| 284 | H | H | H | H | Bd. | CH₃ |
| 285 | H | H | H | H | Bd. | C₃H₇ |
| 286 | H | H | F | F | Bd. | C₂H₅ |
| 287 | H | H | F | F | Bd. | C₃H₇ |
| 288 | F | F | F | F | Bd. | C₃H₇ |
| 289 | F | F | F | F | Bd. | C₄H₉ |
| 290 | H | H | H | H | -CF₂O- | CH₃ |
| 291 | H | H | H | H | -CF₂O- | C₃H₇ |
| 292 | H | H | F | F | -CF₂O- | C₂H₅ |
| 293 | H | H | F | F | -CF₂O- | C₃H₇ |
| 294 | F | F | F | F | -CF₂O- | C₃H₇ |
| 295 | F | F | F | F | -CF₂O- | C₄H₉ |
| 296 | H | H | H | H | -CF₂CF₂- | CH₃ |
| 297 | H | H | H | H | -CF₂CF₂- | C₃H₇ |
| 298 | H | H | F | F | -CF₂CF₂- | C₂H₅ |
| 299 | H | H | F | F | -CF₂CF₂- | C₃H₇ |
| 300 | F | F | F | F | -CF₂CF₂- | C₃H₇ |
| 301 | F | F | F | F | -CF₂CF₂- | C₄H₉ |

| | | | | | | |
|---|---|---|---|---|---|---|
| Bd. = Einfachbindung | | | | | | |

**Tabelle 1**

| Δε- und Δn-Werte für Substanzen einzelner Beispiele | | |
|---|---|---|
| Beispiel Nr. | Δε | Δn |
| 17 | -6,3 | 0,143 |
| 18 | -8,0 | 0,143 |
| 20 | -7,3 | 0,166 |
| 79 | -3,9 | 0,127 |
| 81 | -9,1 | 0,117 |
| 82 | -10,2 | 0,121 |
| 143 | -3,3 | 0,091 |
| 145 | -11,8 | 0,081 |
| 146 | -9,2 | 0,081 |
| 207 | -2,2 | 0,128 |
| 209 | -9,6 | 0,115 |
| 210 | -6,9 | 0,106 |
| 255 | -3,0 | 0,095 |
| 257 | -10,3 | 0,079 |
| 258 | -7,9 | 0,079 |

### Beispiele 302 bis 337

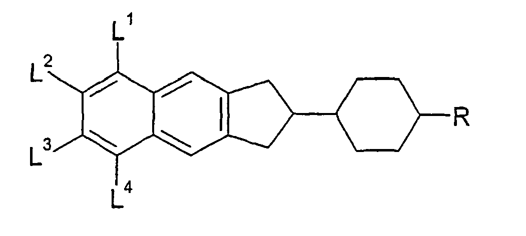

| **Beispiel** | **L¹** | **L²** | **L³** | **L⁴** | **R** |
|---|---|---|---|---|---|
| 302 | H | F | H | H | CH₃ |
| 303 | H | F | H | H | C₃H₇ |
| 304 | H | F | H | H | C₅H₁₁ |
| 305 | H | F | F | H | C₂H₅ |
| 306 | H | F | F | H | C₃H₇ |
| 307 | H | F | F | H | C₆H₁₃ |
| 308 | F | F | F | F | CH₃ |
| 309 | F | F | F | F | C₃H₇ |
| 310 | F | F | F | F | C₅H₁₁ |
| 311 | H | CF₃ | H | H | C₂H₅ |
| 312 | H | CF₃ | H | H | C₃H₇ |
| 313 | H | CF₃ | H | H | C₆H₁₃ |
| 314 | H | OCF₃ | H | H | CH₃ |
| 315 | H | OCF₃ | H | H | C₃H₇ |
| 316 | H | OCF₃ | H | H | C₅H₁₁ |
| 317 | H | CN | H | H | C₂H₅ |
| 318 | H | CN | H | H | C₃H₇ |
| 319 | H | CN | H | H | C₆H₁₃ |
| 320 | H | CF₃ | F | H | C₂H₅ |
| 321 | H | CF₃ | F | H | C₃H₇ |
| 322 | H | CF₃ | F | H | C₆H₁₃ |
| 323 | H | OCF₃ | F | H | CH₃ |
| 324 | H | OCF₃ | F | H | C₃H₇ |
| 325 | H | OCF₃ | F | H | C₅H₁₁ |
| 326 | H | CF₃ | CF₃ | H | C₂H₅ |
| 327 | H | CF₃ | CF₃ | H | C₃H₇ |
| 328 | H | CF₃ | CF₃ | H | C₆H₁₃ |
| 329 | H | CF₃ | OCF₃ | H | CH₃ |
| 330 | H | CF₃ | OCF₃ | H | C₃H₇ |
| 331 | H | CF₃ | OCF₃ | H | C₅H₁₁ |
| 332 | H | OCF₃ | OCF₃ | H | C₂H₅ |
| 333 | H | OCF₃ | OCF₃ | H | C₃H₇ |
| 334 | H | OCF₃ | OCF₃ | H | C₆H₁₃ |
| 335 | H | CN | CN | H | C₂H₅ |
| 336 | H | CN | CN | H | C₃H₇ |
| 337 | H | CN | CN | H | C₆H₁₃ |

**Tabelle 2**

| Δε- und Δn-Werte für Substanzen einzelner Beispiele | | |
|---|---|---|
| Beispiel Nr. | Δε | Δn |
| 306 | 12,9 | 0,179 |
| 309 | 17,0 | 0,158 |
| 312 | 12,7 | 0,160 |
| 321 | 23,2 | 0,159 |
| 324 | 12,4 | 0,172 |
| 327 | 31,4 | 0,159 |
| 330 | 19,7 | 0,143 |
| 333 | 17,4 | 0,158 |

### Beispiele 338 bis 367

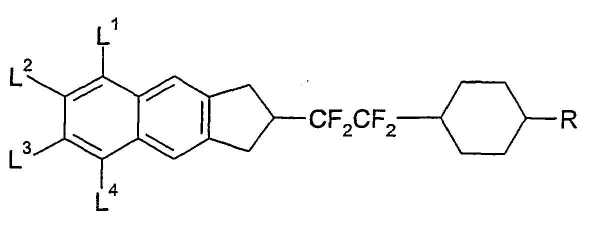

| **Beispiel** | **L¹** | **L²** | **L³** | **L⁴** | **R** |
|---|---|---|---|---|---|
| 338 | H | F | H | H | CH₃ |
| 339 | H | F | H | H | C₃H₇ |
| 340 | H | F | H | H | C₅H₁₁ |
| 341 | H | F | F | H | C₂H₅ |
| 342 | H | F | F | H | C₃H₇ |
| 343 | H | F | F | H | C₆H₁₃ |
| 344 | F | F | F | F | CH₃ |
| 345 | F | F | F | F | C₃H₇ |
| 346 | F | F | F | F | C₅H₁₁ |
| 347 | H | CF₃ | H | H | C₂H₅ |
| 348 | H | CF₃ | H | H | C₃H₇ |
| 349 | H | CF₃ | H | H | C₆H₁₃ |
| 350 | H | OCF₃ | H | H | CH₃ |
| 351 | H | OCF₃ | H | H | C₃H₇ |
| 352 | H | OCF₃ | H | H | C₅H₁₁ |
| 353 | H | CF₃ | F | H | C₂H₅ |
| 354 | H | CF₃ | F | H | C₃H₇ |
| 355 | H | CF₃ | F | H | C₆H₁₃ |
| 356 | H | OCF₃ | F | H | CH₃ |
| 357 | H | OCF₃ | F | H | C₃H₇ |
| 358 | H | OCF₃ | F | H | C₅H₁₁ |
| 359 | H | CF₃ | CF₃ | H | C₂H₅ |
| 360 | H | CF₃ | CF₃ | H | C₃H₇ |
| 361 | H | CF₃ | CF₃ | H | C₆H₁₃ |
| 362 | H | CF₃ | OCF₃ | H | CH₃ |
| 363 | H | CF₃ | OCF₃ | H | C₃H₇ |
| 364 | H | CF₃ | OCF₃ | H | C₅H₁₁ |
| 365 | H | OCF₃ | OCF₃ | H | C₂H₅ |
| 366 | H | OCF₃ | OCF₃ | H | C₃H₇ |
| 367 | H | OCF₃ | OCF₃ | H | C₆H₁₃ |

### Beispiele 368 bis 397

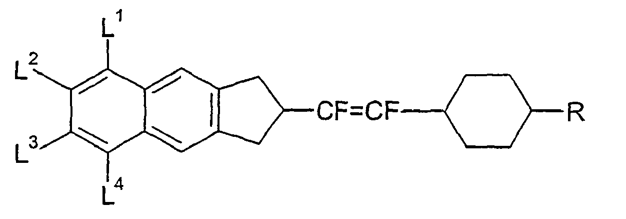

| **Beispiel** | **L¹** | **L²** | **L³** | **L⁴** | **R** |
|---|---|---|---|---|---|
| 368 | H | F | H | H | CH₃ |
| 369 | H | F | H | H | C₃H₇ |
| 370 | H | F | H | H | C₅H₁₁ |
| 371 | H | F | F | H | C₂H₅ |
| 372 | H | F | F | H | C₃H₇ |
| 373 | H | F | F | H | C₆H₁₃ |
| 374 | F | F | F | F | CH₃ |
| 375 | F | F | F | F | C₃H₇ |
| 376 | F | F | F | F | C₅H₁₁ |
| 377 | H | CF₃ | H | H | C₂H₅ |
| 378 | H | CF₃ | H | H | C₃H₇ |
| 379 | H | CF₃ | H | H | C₆H₁₃ |
| 380 | H | OCF₃ | H | H | CH₃ |
| 381 | H | OCF₃ | H | H | C₃H₇ |
| 382 | H | OCF₃ | H | H | C₅H₁₁ |
| 383 | H | CF₃ | F | H | C₂H₅ |
| 384 | H | CF₃ | F | H | C₃H₇ |
| 385 | H | CF₃ | F | H | C₆H₁₃ |
| 386 | H | OCF₃ | F | H | CH₃ |
| 387 | H | OCF₃ | F | H | C₃H₇ |
| 388 | H | OCF₃ | F | H | C₅H₁₁ |
| 389 | H | CF₃ | CF₃ | H | C₂H₅ |
| 390 | H | CF₃ | CF₃ | H | C₃H₇ |
| 391 | H | CF₃ | CF₃ | H | C₆H₁₃ |
| 392 | H | CF₃ | OCF₃ | H | CH₃ |
| 393 | H | CF₃ | OCF₃ | H | C₃H₇ |
| 394 | H | CF₃ | OCF₃ | H | C₅H₁₁ |
| 395 | H | OCF₃ | OCF₃ | H | C₂H₅ |
| 396 | H | OCF₃ | OCF₃ | H | C₃H₇ |
| 397 | H | OCF₃ | OCF₃ | H | C₆H₁₃ |

### Beispiele 398 bis 427

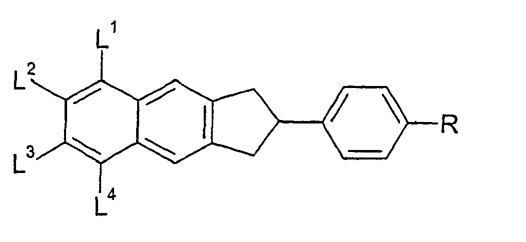

| **Beispiel** | **L¹** | **L²** | **L³** | **L⁴** | **R** |
|---|---|---|---|---|---|
| 398 | H | F | H | H | CH₃ |
| 399 | H | F | H | H | C₃H₇ |
| 400 | H | F | H | H | C₅H₁₁ |
| 401 | H | F | F | H | C₂H₅ |
| 402 | H | F | F | H | C₃H₇ |
| 403 | H | F | F | H | C₆H₁₃ |
| 404 | F | F | F | F | CH₃ |
| 405 | F | F | F | F | C₃H₇ |
| 406 | F | F | F | F | C₅H₁₁ |
| 407 | H | CF₃ | H | H | C₂H₅ |
| 408 | H | CF₃ | H | H | C₃H₇ |
| 409 | H | CF₃ | H | H | C₆H₁₃ |
| 410 | H | OCF₃ | H | H | CH₃ |
| 411 | H | OCF₃ | H | H | C₃H₇ |
| 412 | H | OCF₃ | H | H | C₂H₁₁ |
| 413 | H | CF₃ | F | H | C₂H₅ |
| 414 | H | CF₃ | F | H | C₃H₇ |
| 415 | H | CF₃ | F | H | C₆H₁₃ |
| 416 | H | OCF₃ | F | H | CH₃ |
| 417 | H | OCF₃ | F | H | C₃H₇ |
| 418 | H | OCF₃ | F | H | C₅H₁₁ |
| 419 | H | CF₃ | CF₃ | H | C₂H₅ |
| 420 | H | CF₃ | CF₃ | H | C₃H₇ |
| 421 | H | CF₃ | CF₃ | H | C₆H₁₃ |
| 422 | H | CF₃ | OCF₃ | H | CH₃ |
| 423 | H | CF₃ | OCF₃ | H | C₃H₇ |
| 424 | H | CF₃ | OCF₃ | H | C₅H₁₁ |
| 425 | H | OCF₃ | OCF₃ | H | C₂H₅ |
| 426 | H | OCF₃ | OCF₃ | H | C₃H₇ |
| 427 | H | OCF₃ | OCF₃ | H | C₆H₁₃ |

### Beispiele 428 bis 457

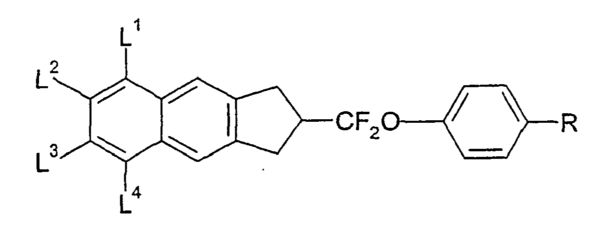

| **Beispiel** | **L¹** | **L²** | **L³** | **L⁴** | **R** |
|---|---|---|---|---|---|
| 428 | H | F | H | H | CH₃ |
| 429 | H | F | H | H | C₃H₇ |
| 430 | H | F | H | H | C₅H₁₁ |
| 431 | H | F | F | H | C₂H₅ |
| 432 | H | F | F | H | C₃H₇ |
| 433 | H | F | F | H | C₆H₁₃ |
| 434 | F | F | F | F | CH₃ |
| 435 | F | F | F | F | C₃H₇ |
| 436 | F | F | F | F | C₅H₁₁ |
| 437 | H | CF₃ | H | H | C₂H₅ |
| 438 | H | CF₃ | H | H | C₃H₇ |
| 439 | H | CF₃ | H | H | C₆H₁₃ |
| 440 | H | OCF₃ | H | H | CH₃ |
| 441 | H | OCF₃ | H | H | C₃H₇ |
| 442 | H | OCF₃ | H | H | C₅H₁₁ |
| 443 | H | CF₃ | F | H | C₂H₅ |
| 444 | H | CF₃ | F | H | C₃H₇ |
| 445 | H | CF₃ | F | H | C₆H₁₃ |
| 446 | H | OCF₃ | F | H | CH₃ |
| 447 | H | OCF₃ | F | H | C₃H₇ |
| 448 | H | OCF₃ | F | H | C₅H₁₁ |
| 449 | H | CF₃ | CF₃ | H | C₂H₅ |
| 450 | H | CF₃ | CF₃ | H | C₃H₇ |
| 451 | H | CF₃ | CF₃ | H | C₆H₁₃ |
| 452 | H | CF₃ | OCF₃ | H | CH₃ |
| 453 | H | CF₃ | OCF₃ | H | C₃H₇ |
| 454 | H | CF₃ | OCF₃ | H | C₅H₁₁ |
| 455 | H | OCF₃ | OCF₃ | H | C₂H₅ |
| 456 | H | OCF₃ | OCF₃ | H | C₃H₇ |
| 457 | H | OCF₃ | OCF₃ | H | C₆H₁₃ |

### Beispiele 458 bis 487

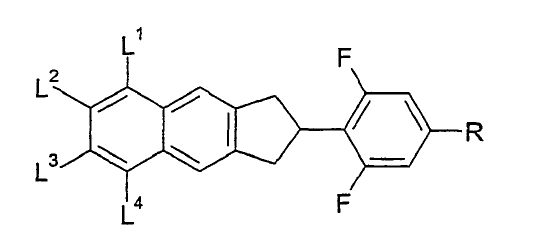

| **Beispiel** | **L¹** | **L²** | **L³** | **L⁴** | **R** |
|---|---|---|---|---|---|
| 458 | H | F | H | H | CH₃ |
| 459 | H | F | H | H | C₃H₇ |
| 460 | H | F | H | H | C₅H₁₁ |
| 461 | H | F | F | H | C₂H₅ |
| 462 | H | F | F | H | C₃H₇ |
| 463 | H | F | F | H | C₆H₁₃ |
| 464 | F | F | F | F | CH₃ |
| 465 | F | F | F | F | C₃H₇ |
| 466 | F | F | F | F | C₅H₁₁ |
| 467 | H | CF₃ | H | H | C₂H₅ |
| 468 | H | CF₃ | H | H | C₃H₇ |
| 469 | H | CF₃ | H | H | C₆H₁₃ |
| 470 | H | OCF₃ | H | H | CH₃ |
| 471 | H | OCF₃ | H | H | C₃H₇ |
| 472 | H | OCF₃ | H | H | C₅H₁₁ |
| 473 | H | CF₃ | F | H | C₂H₅ |
| 474 | H | CF₃ | F | H | C₃H₇ |
| 475 | H | CF₃ | F | H | C₆H₁₃ |
| 476 | H | OCF₃ | F | H | CH₃ |
| 477 | H | OCF₃ | F | H | C₃H₇ |
| 478 | H | OCF₃ | F | H | C₅H₁₁ |
| 479 | H | CF₃ | CF₃ | H | C₂H₅ |
| 480 | H | CF₃ | CF₃ | H | C₃H₇ |
| 481 | H | CF₃ | CF₃ | H | C₆H₁₃ |
| 482 | H | CF₃ | OCF₃ | H | CH₃ |
| 483 | H | CF₃ | OCF₃ | H | C₃H₇ |
| 484 | H | CF₃ | OCF₃ | H | C₅H₁₁ |
| 485 | H | OCF₃ | OCF₃ | H | C₂H₅ |
| 486 | H | OCF₃ | OCF₃ | H | C₃H₇ |
| 487 | H | OCF₃ | OCF₃ | H | C₆H₁₃ |

### Beispiele 488 bis 517

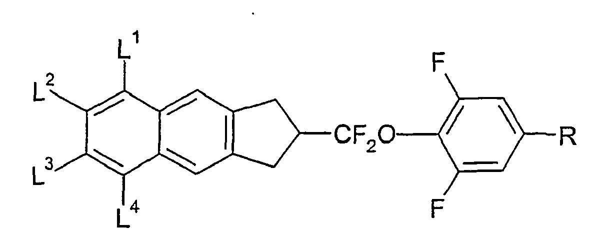

| **Beispiel** | **L¹** | **L²** | **L³** | **L⁴** | **R** |
|---|---|---|---|---|---|
| 488 | H | F | H | H | CH₃ |
| 489 | H | F | H | H | C₃H₇ |
| 490 | H | F | H | H | C₅H₁₁ |
| 491 | H | F | F | H | C₂H₅ |
| 492 | H | F | F | H | C₃H₇ |
| 493 | H | F | F | H | C₆H₁₃ |
| 494 | F | F | F | F | CH₃ |
| 495 | F | F | F | F | C₃H₇ |
| 496 | F | F | F | F | C₅H₁₁ |
| 497 | H | CF₃ | H | H | C₂H₅ |
| 498 | H | CF₃ | H | H | C₃H₇ |
| 499 | H | CF₃ | H | H | C₆H₁₃ |
| 500 | H | OCF₃ | H | H | CH₃ |
| 501 | H | OCF₃ | H | H | C₃H₇ |
| 502 | H | OCF₃ | H | H | C₅H₁₁ |
| 503 | H | CF₃ | F | H | C₂H₅ |
| 504 | H | CF₃ | F | H | C₃H₇ |
| 505 | H | CF₃ | F | H | C₆H₁₃ |
| 506 | H | OCF₃ | F | H | CH₃ |
| 507 | H | OCF₃ | F | H | C₃H₇ |
| 508 | H | OCF₃ | F | H | C₅H₁₁ |
| 509 | H | CF₃ | CF₃ | H | C₂H₅ |
| 510 | H | CF₃ | CF₃ | H | C₃H₇ |
| 511 | H | CF₃ | CF₃ | H | C₆H₁₃ |
| 512 | H | CF₃ | OCF₃ | H | CH₃ |
| 513 | H | CF₃ | OCF₃ | H | C₃H₇ |
| 514 | H | CF₃ | OCF₃ | H | C₅H₁₁ |
| 515 | H | OCF₃ | OCF₃ | H | C₂H₅ |
| 516 | H | OCF₃ | OCF₃ | H | C₃H₇ |
| 517 | H | OCF₃ | OCF₃ | H | C₆H₁₃ |

### Beispiele 518 bis 547

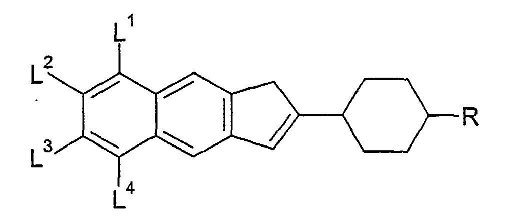

| **Beispiel** | **L¹** | **L²** | **L³** | **L⁴** | **R** |
|---|---|---|---|---|---|
| 518 | H | F | H | H | CH₃ |
| 519 | H | F | H | H | C₃H₇ |
| 520 | H | F | H | H | C₅H₁₁ |
| 521 | H | F | F | H | C₂H₅ |
| 522 | H | F | F | H | C₃H₇ |
| 523 | H | F | F | H | C₆H₁₃ |
| 524 | F | F | F | F | CH₃ |
| 525 | F | F | F | F | C₃H₇ |
| 526 | F | F | F | F | C₅H₁₁ |
| 527 | H | CF₃ | H | H | C₂H₅ |
| 528 | H | CF₃ | H | H | C₃H₇ |
| 529 | H | CF₃ | H | H | C₆H₁₃ |
| 530 | H | OCF₃ | H | H | CH₃ |
| 531 | H | OCF₃ | H | H | C₃H₇ |
| 532 | H | OCF₃ | H | H | C₅H₁₁ |
| 533 | H | CF₃ | F | H | C₂H₅ |
| 534 | H | CF₃ | F | H | C₃H₇ |
| 535 | H | CF₃ | F | H | C₆H₁₃ |
| 536 | H | OCF₃ | F | H | CH₃ |
| 537 | H | OCF₃ | F | H | C₃H₇ |
| 538 | H | OCF₃ | F | H | C₅H₁₁ |
| 539 | H | CF₃ | CF₃ | H | C₂H₅ |
| 540 | H | CF₃ | CF₃ | H | C₃H₇ |
| 541 | H | CF₃ | CF₃ | H | C₆H₁₃ |
| 542 | H | CF₃ | OCF₃ | H | CH₃ |
| 543 | H | CF₃ | OCF₃ | H | C₃H₇ |
| 544 | H | CF₃ | OCF₃ | H | C₅H₁₁ |
| 545 | H | OCF₃ | OCF₃ | H | C₂H₅ |
| 546 | H | OCF₃ | OCF₃ | H | C₃H₇ |
| 547 | H | OCF₃ | OCF₃ | H | C₆H₁₃ |

### Beispiele 548 bis 577

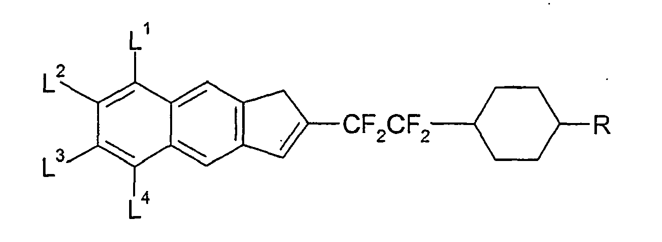

| **Beispiel** | **L¹** | **L²** | **L³** | **L⁴** | **R** |
|---|---|---|---|---|---|
| 548 | H | F | H | H | CH₃ |
| 549 | H | F | H | H | C₃H₇ |
| 550 | H | F | H | H | C₅H₁₁ |
| 551 | H | F | F | H | C₂H₅ |
| 552 | H | F | F | H | C₃H₇ |
| 553 | H | F | F | H | C₆H₁₃ |
| 554 | F | F | F | F | CH₃ |
| 555 | F | F | F | F | C₃H₇ |
| 556 | F | F | F | F | C₅H₁₁ |
| 557 | H | CF₃ | H | H | C₂H₅ |
| 558 | H | CF₃ | H | H | C₃H₇ |
| 559 | H | CF₃ | H | H | C₆H₁₃ |
| 560 | H | OCF₃ | H | H | CH₃ |
| 561 | H | OCF₃ | H | H | C₃H₇ |
| 562 | H | OCF₃ | H | H | C₅H₁₁ |
| 563 | H | CF₃ | F | H | C₂H₅ |
| 564 | H | CF₃ | F | H | C₃H₇ |
| 565 | H | CF₃ | F | H | C₆H₁₃ |
| 566 | H | OCF₃ | F | H | CH₃ |
| 567 | H | OCF₃ | F | H | C₃H₇ |
| 568 | H | OCF₃ | F | H | C₅H₁₁ |
| 569 | H | CF₃ | CF₃ | H | C₂H₅ |
| 570 | H | CF₃ | CF₃ | H | C₃H₇ |
| 571 | H | CF₃ | CF₃ | H | C₆H₁₃ |
| 572 | H | CF₃ | OCF₃ | H | CH₃ |
| 573 | H | CF₃ | OCF₃ | H | C₃H₇ |
| 574 | H | CF₃ | OCF₃ | H | C₅H₁₁ |
| 575 | H | OCF₃ | OCF₃ | H | C₂H₅ |
| 576 | H | OCF₃ | OCF₃ | H | C₃H₇ |
| 577 | H | OCF₃ | OCF₃ | H | C₆H₁₃ |

### Beispiele 578 bis 607

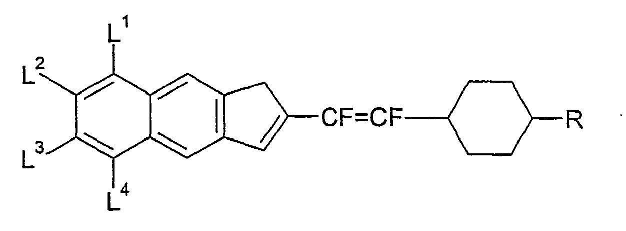

| **Beispiel** | **L¹** | **L²** | **L³** | **L⁴** | **R** |
|---|---|---|---|---|---|
| 578 | H | F | H | H | CH₃ |
| 579 | H | F | H | H | C₃H₇ |
| 580 | H | F | H | H | C₅H₁₁ |
| 581 | H | F | F | H | C₂H₅ |
| 582 | H | F | F | H | C₃H₇ |
| 583 | H | F | F | H | C₆H₁₃ |
| 584 | F | F | F | F | CH₃ |
| 585 | F | F | F | F | C₃H₇ |
| 586 | F | F | F | F | C₅H₁₁ |
| 587 | H | CF₃ | H | H | C₂H₅ |
| 588 | H | CF₃ | H | H | C₃H₇ |
| 589 | H | CF₃ | H | H | C₆H₁₃ |
| 590 | H | OCF₃ | H | H | CH₃ |
| 591 | H | OCF₃ | H | H | C₃H₇ |
| 592 | H | OCF₃ | H | H | C₅H₁₁ |
| 593 | H | CF₃ | F | H | C₂H₅ |
| 594 | H | CF₃ | F | H | C₃H₇ |
| 595 | H | CF₃ | F | H | C₆H₁₃ |
| 596 | H | OCF₃ | F | H | CH₃ |
| 597 | H | OCF₃ | F | H | C₃H₇ |
| 598 | H | OCF₃ | F | H | C₅H₁₁ |
| 599 | H | CF₃ | CF₃ | H | C₂H₅ |
| 600 | H | CF₃ | CF₃ | H | C₃H₇ |
| 601 | H | CF₃ | CF₃ | H | C₆H₁₃ |
| 602 | H | CF₃ | OCF₃ | H | CH₃ |
| 603 | H | CF₃ | OCF₃ | H | C₃H₇ |
| 604 | H | CF₃ | OCF₃ | H | C₅H₁₁ |
| 605 | H | OCF₃ | OCF₃ | H | C₂H₅ |
| 606 | H | OCF₃ | OCF₃ | H | C₃H₇ |
| 607 | H | OCF₃ | OCF₃ | H | C₆H₁₃ |

### Beispiele 608 bis 637

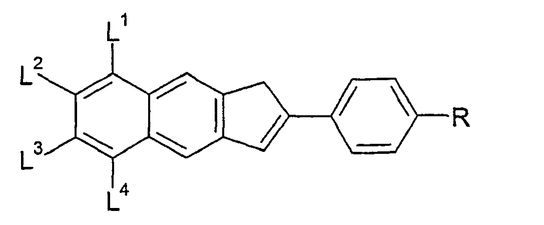

| **Beispiel** | **L¹** | **L²** | **L³** | **L⁴** | **R** |
|---|---|---|---|---|---|
| 608 | H | F | H | H | CH₃ |
| 609 | H | F | H | H | C₃H₇ |
| 610 | H | F | H | H | C₅H₁₁ |
| 611 | H | F | F | H | C₂H₅ |
| 612 | H | F | F | H | C₃H₇ |
| 613 | H | F | F | H | C₆H₁₃ |
| 614 | F | F | F | F | CH₃ |
| 615 | F | F | F | F | C₃H₇ |
| 616 | F | F | F | F | C₅H₁₁ |
| 617 | H | CF₃ | H | H | C₂H₅ |
| 618 | H | CF₃ | H | H | C₃H₇ |
| 619 | H | CF₃ | H | H | C₆H₁₃ |
| 620 | H | OCF₃ | H | H | CH₃ |
| 621 | H | OCF₃ | H | H | C₃H₇ |
| 622 | H | OCF₃ | H | H | C₅H₁₁ |
| 623 | H | CF₃ | F | H | C₂H₅ |
| 624 | H | CF₃ | F | H | C₃H₇ |
| 625 | H | CF₃ | F | H | C₆H₁₃ |
| 626 | H | OCF₃ | F | H | CH₃ |
| 627 | H | OCF₃ | F | H | C₃H₇ |
| 628 | H | OCF₃ | F | H | C₅H₁₁ |
| 629 | H | CF₃ | CF₃ | H | C₂H₅ |
| 630 | H | CF₃ | CF₃ | H | C₃H₇ |
| 631 | H | CF₃ | CF₃ | H | C₆H₁₃ |
| 632 | H | CF₃ | OCF₃ | H | CH₃ |
| 633 | H | CF₃ | OCF₃ | H | C₃H₇ |
| 634 | H | CF₃ | OCF₃ | H | C₅H₁₁ |
| 635 | H | OCF₃ | OCF₃ | H | C₂H₅ |
| 636 | H | OCF₃ | OCF₃ | H | C₃H₇ |
| 637 | H | OCF₃ | OCF₃ | H | C₆H₁₃ |

### Beispiele 638 bis 667

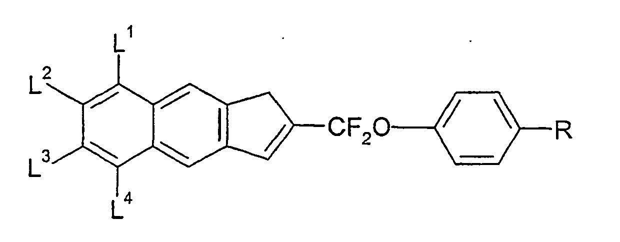

| **Beispiel** | **L¹** | **L²** | **L³** | **L⁴** | **R** |
|---|---|---|---|---|---|
| 638 | H | F | H | H | CH₃ |
| 639 | H | F | H | H | C₃H₇ |
| 640 | H | F | H | H | C₅H₁₁ |
| 641 | H | F | F | H | C₂H₅ |
| 642 | H | F | F | H | C₃H₇ |
| 643 | H | F | F | H | C₆H₁₃ |
| 644 | F | F | F | F | CH₃ |
| 645 | F | F | F | F | C₃H₇ |
| 646 | F | F | F | F | C₅H₁₁ |
| 647 | H | CF₃ | H | H | C₂H₅ |
| 648 | H | CF₃ | H | H | C₃H₇ |
| 649 | H | CF₃ | H | H | C₆H₁₃ |
| 650 | H | OCF₃ | H | H | CH₃ |
| 651 | H | OCF₃ | H | H | C₃H₇ |
| 652 | H | OCF₃ | H | H | C₅H₁₁ |
| 653 | H | CF₃ | F | H | C₂H₅ |
| 654 | H | CF₃ | F | H | C₃H₇ |
| 655 | H | CF₃ | F | H | C₆H₁₃ |
| 656 | H | OCF₃ | F | H | CH₃ |
| 657 | H | OCF₃ | F | H | C₃H₇ |
| 658 | H | OCF₃ | F | H | C₅H₁₁^{,} |
| 659 | H | CF₃ | CF₃ | H | C₂H₅ |
| 660 | H | CF₃ | CF₃ | H | C₃H₇ |
| 661 | H | CF₃ | CF₃ | H | C₆H₁₃ |
| 662 | H | CF₃ | OCF₃ | H | CH₃ |
| 663 | H | CF₃ | OCF₃ | H | C₃H₇ |
| 664 | H | CF₃ | OCF₃ | H | C₅H₁₁ |
| 665 | H | OCF₃ | OCF₃ | H | C₂H₅ |
| 666 | H | OCF₃ | OCF₃ | H | C₃H₇ |
| 667 | H | OCF₃ | OCF₃ | H | C₆H₁₃ |

### Beispiele 668 bis 697

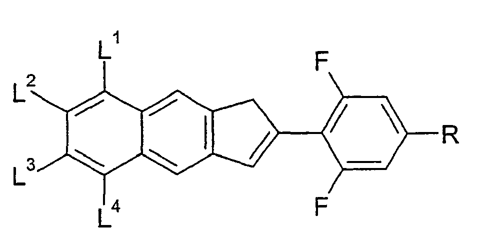

| **Beispiel** | **L¹** | **L²** | **L³** | **L⁴** | **R** |
|---|---|---|---|---|---|
| 668 | H | F | H | H | CH₃ |
| 669 | H | F | H | H | C₃H₇ |
| 670 | H | F | H | H | C₅H₁₁ |
| 671 | H | F | F | H | C₂H₅ |
| 672 | H | F | F | H | C₃H₇ |
| 673 | H | F | F | H | C₆H₁₃ |
| 674 | F | F | F | F | CH₃ |
| 675 | F | F | F | F | C₃H₇ |
| 676 | F | F | F | F | C₅H₁₁ |
| 677 | H | CF₃ | H | H | C₂H₅ |
| 678 | H | CF₃ | H | H | C₃H₇ |
| 679 | H | CF₃ | H | H | C₆H₁₃ |
| 680 | H | OCF₃ | H | H | CH₃ |
| 681 | H | OCF₃ | H | H | C₃H₇ |
| 682 | H | OCF₃ | H | H | C₅H₁₁ |
| 683 | H | CF₃ | F | H | C₂H₅ |
| 684 | H | CF₃ | F | H | C₃H₇ |
| 685 | H | CF₃ | F | H | C₆H₁₃ |
| 686 | H | OCF₃ | F | H | CH₃ |
| 687 | H | OCF₃ | F | H | C₃H₇ |
| 688 | H | OCF₃ | F | H | C₅H₁₁ |
| 689 | H | CF₃ | CF₃ | H | C₂H₅ |
| 690 | H | CF₃ | CF₃ | H | C₃H₇ |
| 691 | H | CF₃ | CF₃ | H | C₆H₁₃ |
| 692 | H | CF₃ | OCF₃ | H | CH₃ |
| 693 | H | CF₃ | OCF₃ | H | C₃H₇ |
| 694 | H | CF₃ | OCF₃ | H | C_{S}H₁₁ |
| 695 | H | OCF₃ | OCF₃ | H | C₂H₅ |
| 696 | H | OCF₃ | OCF₃ | H | C₃H₇ |
| 697 | H | OCF₃ | OCF₃ | H | C₆H₁₃ |

### Beispiele 698 bis 727

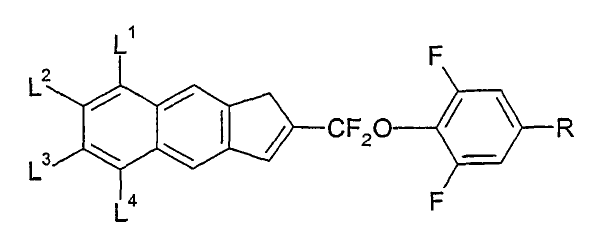

| **Beispiel** | **L¹** | **L²** | **L³** | **L⁴** | **R** |
|---|---|---|---|---|---|
| 698 | H | F | H | H | CH₃ |
| 699 | H | F | H | H | C₃H₇ |
| 700 | H | F | H | H | C₅H₁₁ |
| 701 | H | F | F | H | C₂H₅ |
| 702 | H | F | F | H | C₃H₇ |
| 703 | H | F | F | H | C₆H₁₃ |
| 704 | F | F | F | F | CH₃ |
| 705 | F | F | F | F | C₃H₇ |
| 706 | F | F | F | F | C₅H₁₁ |
| 707 | H | CF₃ | H | H | C₂H₅ |
| 708 | H | CF₃ | H | H | C₃H₇ |
| 709 | H | CF₃ | H | H | C₆H₁₃ |
| 710 | H | OCF₃ | H | H | CH₃ |
| 711 | H | OCF₃ | H | H | C₃H₇ |
| 712 | H | OCF₃ | H | H | C₅H₁₁ |
| 713 | H | CF₃ | F | H | C₂H₅ |
| 714 | H | CF₃ | F | H | C₃H₇ |
| 715 | H | CF₃ | F | H | C₆H₁₃ |
| 716 | H | OCF₃ | F | H | CH₃ |
| 717 | H | OCF₃ | F | H | C₃H₇ |
| 718 | H | OCF₃ | F | H | C₅H₁₁ |
| 719 | H | CF₃ | CF₃ | H | C₂H₅ |
| 720 | H | CF₃ | CF₃ | H | C₃H₇ |
| 721 | H | CF₃ | CF₃ | H | C₆H₁₃ |
| 722 | H | CF₃ | OCF₃ | H | CH₃ |
| 723 | H | CF₃ | OCF₃ | H | C₃H₇ |
| 724 | H | CF₃ | OCF₃ | H | C₅H₁₁ |
| 725 | H | OCF₃ | OCF₃ | H | C₂H₅ |
| 726 | H | OCF₃ | OCF₃ | H | C₃H₇ |
| 727 | H | OCF₃ | OCF₃ | H | C₆H₁₃ |

## Patentansprüche

1. Cyclopenta[b]naphthalinderivate der allgemeinen Formeln (II) bis (XI) 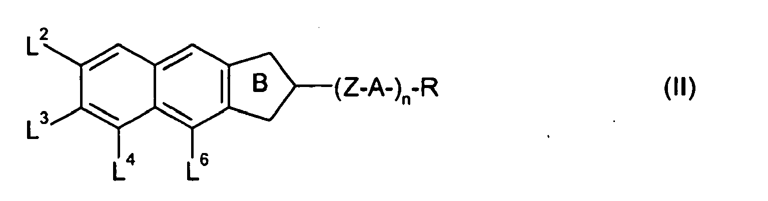 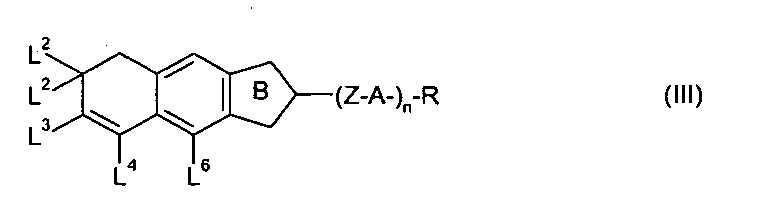 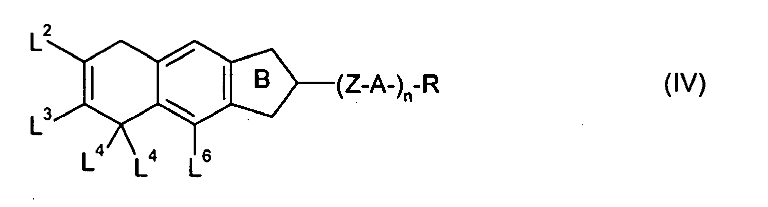 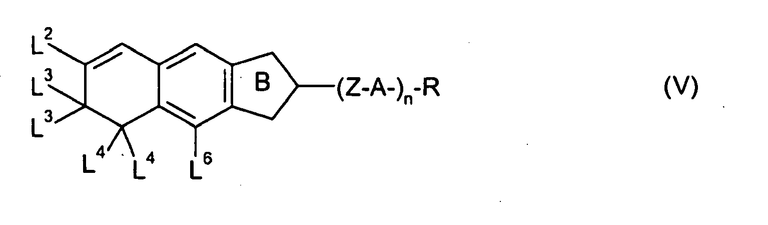 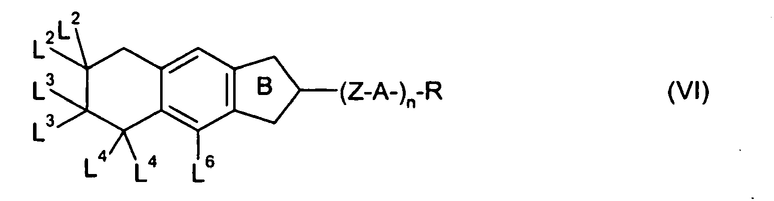 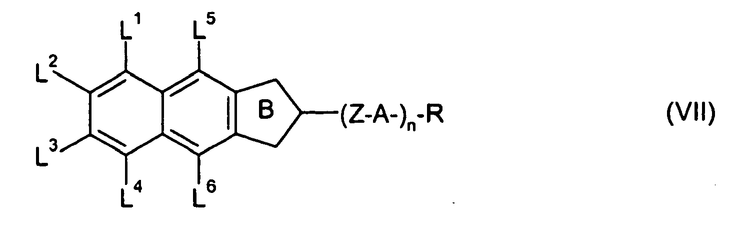 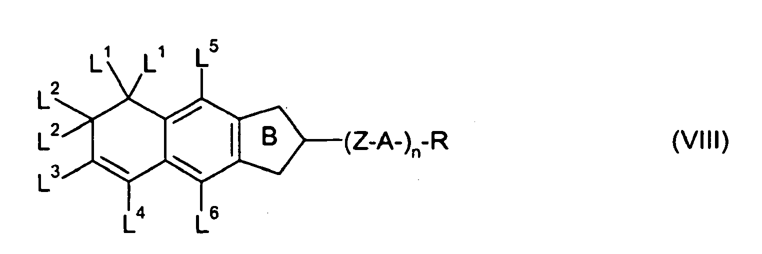 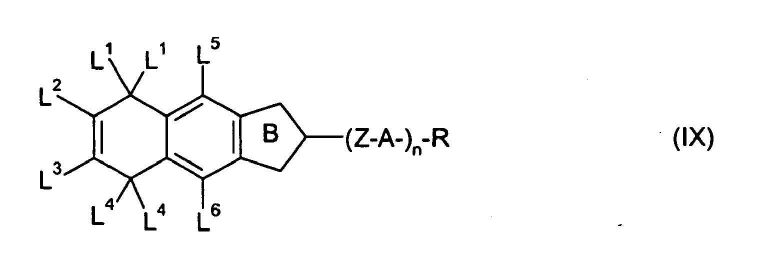 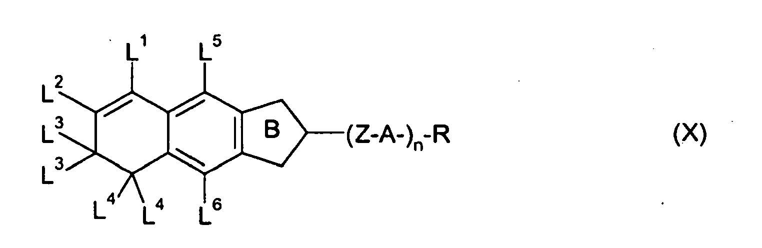 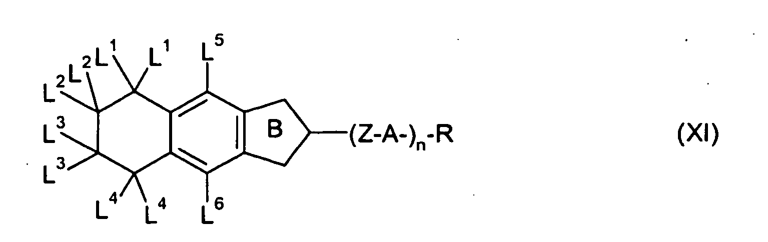 worin in den Formeln (II) bis (XI):
Z jeweils unabhängig voneinander eine Einfachbindung, eine Doppelbindung, -CF₂O-, -OCF₂-, -CH₂CH₂-, -CF₂CF₂-, -C(O)O-, -OC(O)-, -CH₂O-, -OCH₂-, -CF=CH-, -CH=CF-, -CF=CF-, -CH=CH- oder -C≡C-,
A jeweils unabhängig voneinander 1,4-Phenylen, worin =CH- ein- oder zweimal durch =N- ersetzt sein kann, und das ein- bis viermal unabhängig voneinander mit Halogen (-F, -Cl, -Br, -I), -CN, -CH₃, -CH₂F, -CHF₂, -CF₃, -OCH₃, -OCH₂F, -OCHF₂ oder -OCF₃ substituiert sein kann, 1,4-Cyclohexylen, 1,4-Cyclohexenylen oder 1,4-Cyclohexadienylen, worin -CH₂- ein- oder zweimal unabhängig voneinander durch -O- oder -S- so ersetzt sein können, dass Heteroatome nicht direkt benachbart sind, und die ein- oder mehrfach durch Halogen substituiert sein können, 1,3-Cyclobutylen oder Bicyclo[2.2.2]octan,
R Wasserstoff, einen unsubstituierten, einen einfach durch -CF₃ oder mindestens einfach durch Halogen substituierten Alkyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN, -NCS, -SF₅, -CF₃, -OCF₃, -OCHF₂ oder -OCH₂F, und
n 1, 2 oder 3,
bedeuten,
worin in den Formeln (II) bis (VI) 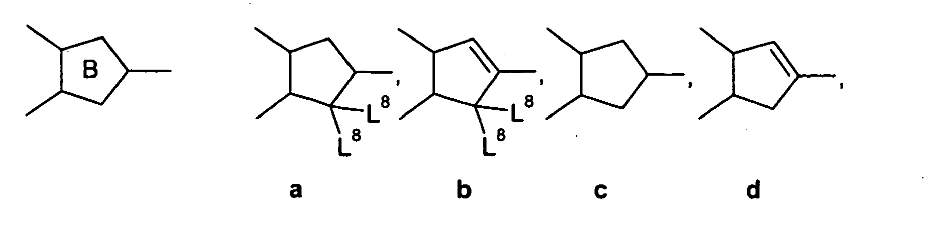 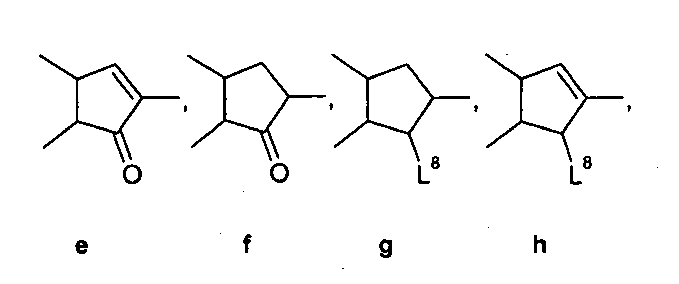 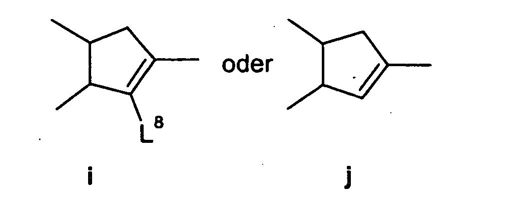
L², L³,L⁸ jeweils unabhängig voneinander Wasserstoff, einen unsubstituierten oder einen mindestens einfach durch Halogen substituierten Alkyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN, -NCS, -SF₅, -CF₃, -OCF₃, -OCHF₂, -OCH₂F oder -(Z-A-)ₙ-R, und
L⁴, L⁶ jeweils unabhängig voneinander Wasserstoff, einen mindestens einfach durch Halogen substituierten Alkyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SF₅, -SCN, -NCS, -CF₃, -OCF₃, -OCHF₂ oder -OCH₂F, mit der Maßgabe, dass L⁴ und L⁶ nicht gleichzeitig Wasserstoff sein dürfen,
bedeuten, und
worin in den Formeln (VII) bis (XI) 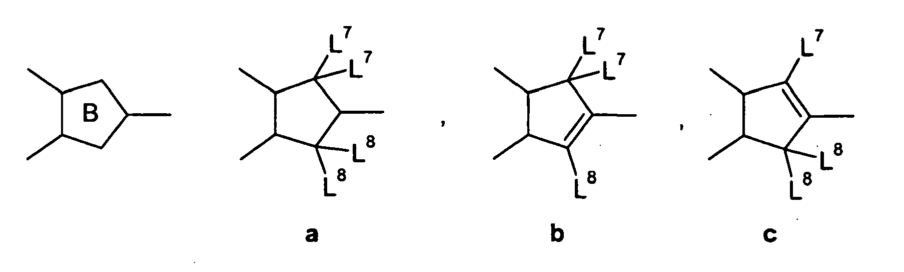 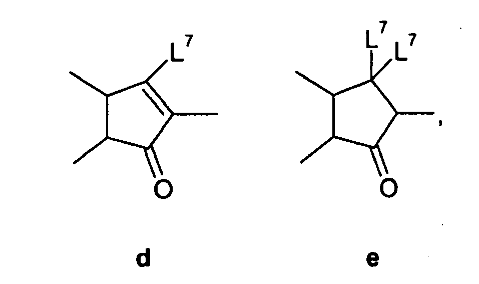 und
L¹ - L⁸ jeweils unabhängig voneinander Wasserstoff, einen unsubstituierten oder einen mindestens einfach durch Halogen substituierten Alkyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -CO-, -COO-, -OCO- oder -OCO-O- so ersetzt sein können, dass Heteroatome nicht direkt benachbart sind, Halogen, -CN, -SCN, -NCS, -SF₅, -CF₃, -OCF₃, -OCHF₂, -OCH₂F oder -(Z-A-)ₙ-R, mit der Maßgabe, dass L² und/oder L³ kein Wasserstoff ist,
bedeuten.

2. Cyclopenta[b]naphthalinderivate gemäß Anspruch 1, **dadurch gekennzeichnet, dass** in den Formeln (II) bis (VI) 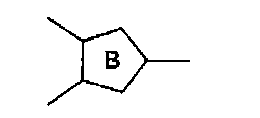 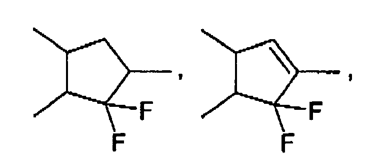 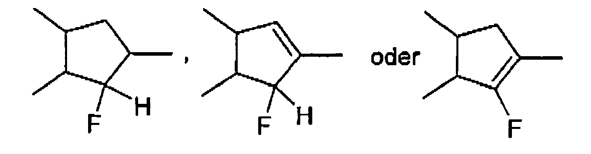 ist.

3. Cyclopenta[b]naphthalinderivate gemäß Anspruch 1 oder 2,
**dadurch gekennzeichnet, dass** in den Formeln (II) bis (VI) A 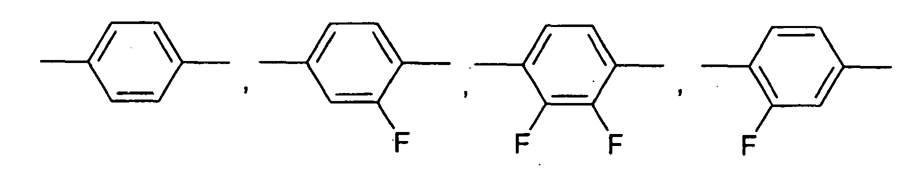 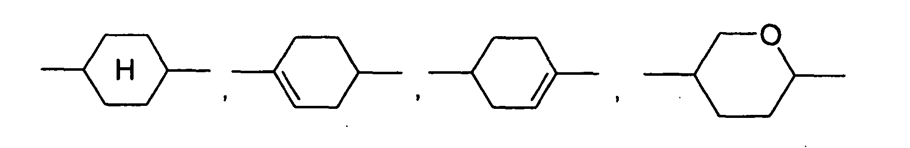 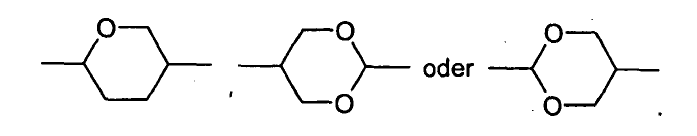 ist.

4. Cyclopenta[b]naphthalinderivate gemäß mindestens einem der Ansprüche 1 bis 3, **dadurch gekennzeichnet, dass** in den Formeln (II) bis (VI) L² und L³ unabhängig voneinander Wasserstoff, ein Alkoxyrest mit 1 bis 7 C-Atomen, Fluor oder Chlor sind.

5. Cyclopenta[b]naphthalinderivate gemäß mindestens einem der Ansprüche 1 bis 4, **dadurch gekennzeichnet, dass** in den Formeln (II) bis (VI) L⁴ und L⁶ unabhängig voneinander -CF₃, Fluor oder Chlor sind.

6. Cyclopenta[b]naphthalinderivate gemäß Anspruch 1, **dadurch gekennzeichnet, dass** in den Formeln (VII) bis (XI) 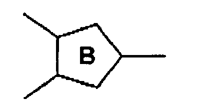 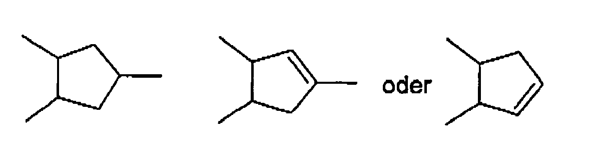 ist.

7. Cyclopenta[b]naphthalinderivate gemäß Anspruch 1 oder 6,
**dadurch gekennzeichnet, dass** in den Formeln (VII) bis (XI) A 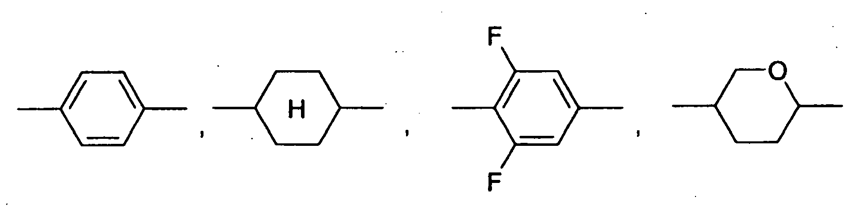 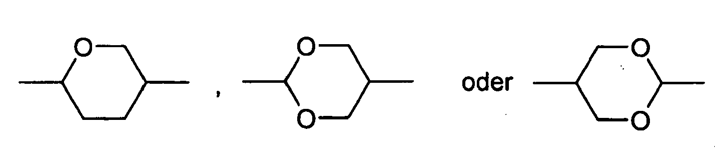 ist.

8. Cyclopenta[b]naphthalinderivate gemäß mindestens einem der Ansprüche 1, 6 und 7, **dadurch gekennzeichnet, dass** in den Formeln (VII) bis (XI) L² und L³ unabhängig voneinander, gleich oder verschieden, Halogen, -CN, -SCN, -NCS, -SF₅, -CF₃, -CHF₂, -OCF₃ oder -OCHF₂ sind.

9. Cyclopenta[b]naphthalinderivate gemäß mindestens einem der Ansprüche 1 und 6 bis 8, **dadurch gekennzeichnet, dass** in den Formeln (VII) bis (XI) L¹ und L⁴ unabhängig voneinander, gleich oder verschieden, Wasserstoff oder Fluor sind.

10. Cyclopenta[b]naphthalinderivate gemäß mindestens einem der Ansprüche 1 und 6 bis 9, **dadurch gekennzeichnet, dass** in den Formeln (VII) bis (XI) L⁵ und L⁶ Wasserstoff sind.

11. Cyclopenta[b]naphthalinderivate gemäß mindestens einem der Ansprüche 1 und 6 bis 10, **dadurch gekennzeichnet, dass** in den Formeln (VII) bis (XI) L¹, L², L³ und L⁴ Fluor und L⁵ und L⁶ Wasserstoff sind.

12. Cyclopenta[b]naphthalinderivate gemäß mindestens einem der vorhergehenden Ansprüche, **dadurch gekennzeichnet, dass** Z eine Einfachbindung, -CF₂O-, -OCF₂-, -CF₂CF₂-, -CH=CH-, -CF=CH-, -CH=CF- oder -CF=CF- ist.

13. Cyclopenta[b]naphthalinderivate gemäß mindestens einem der vorhergehenden Ansprüche, **dadurch gekennzeichnet, dass** R ein Alkylrest, Alkoxyrest oder Alkenylrest mit 1 bis 7 bzw. 2 bis 7 C-Atomen ist.

14. Verwendung von Cyclopenta[b]naphthalinderivaten gemäß mindestens einem der vorhergehenden Ansprüche in flüssigkristallinen Medien.

15. Flüssigkristallines Medium mit mindestens zwei flüssigkristallinen Verbindungen, **dadurch gekennzeichnet, dass** es mindestens ein Cyclopenta[b]naphthalinderivat gemäß mindestens einem der Ansprüche 1 bis 13 enthält.

16. Elektrooptisches Anzeigeelement, enthaltend ein flüssigkristallines Medium gemäß Anspruch 15.

17. Mesogenes Medium, **dadurch gekennzeichnet, dass** es mindestens ein Cyclopenta[b]naphthalinderivat der Formeln (VII) bis (XI) gemäß mindestens einem der Ansprüche 1 und 6 bis 13 enthält.

18. Elektrooptisches Lichtsteuerelement, das eine Elektrodenanordnung, mindestens ein Element zur Polarisation des Lichts und ein mesogenes Steuermedium enthält, wobei das Lichtsteuerelement bei einer Temperatur betrieben wird, bei der das mesogene Steuermedium im nicht angesteuerten Zustand in der isotropen Phase vorliegt, **dadurch gekennzeichnet, dass** das mesogene Steuermedium mindestens ein Cyclopenta[b]naphthalinderivat der Formeln (VII) bis (XI) gemäß mindestens einem der Ansprüche 1 und 6 bis 13 enthält.

## Claims

1. Cyclopenta[b]naphthalene derivatives of the general formulae (II) to (XI) 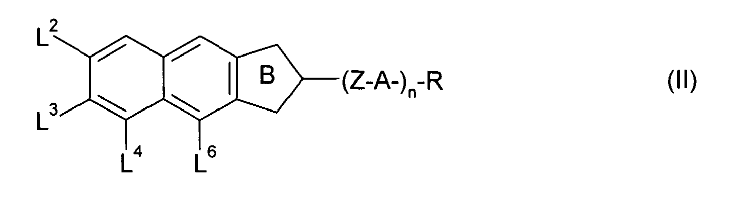 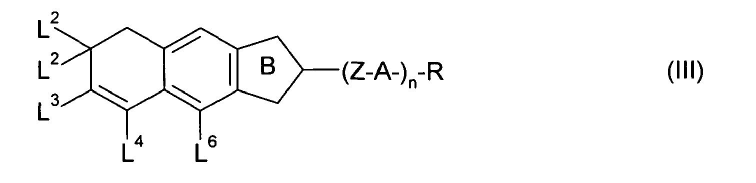 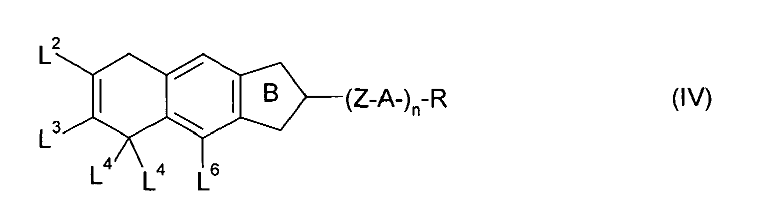 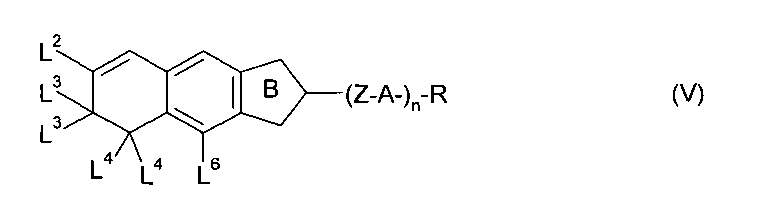 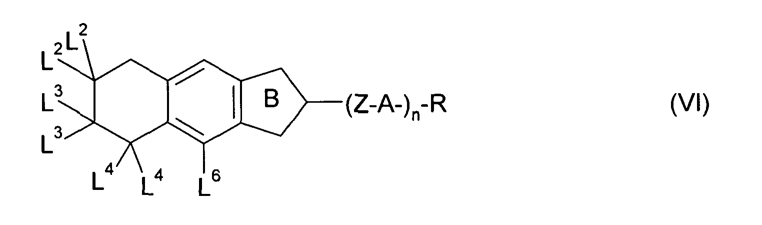 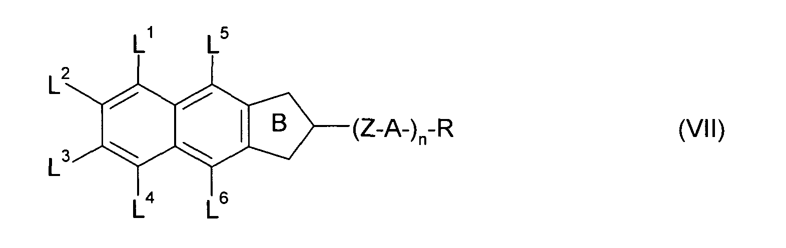 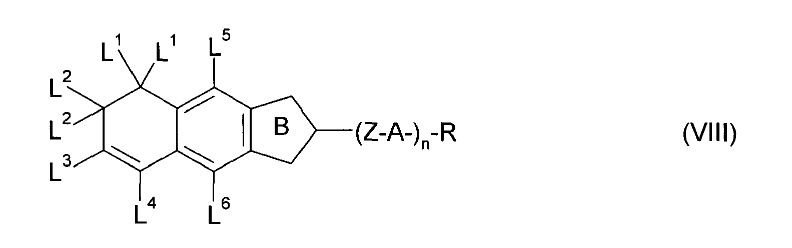 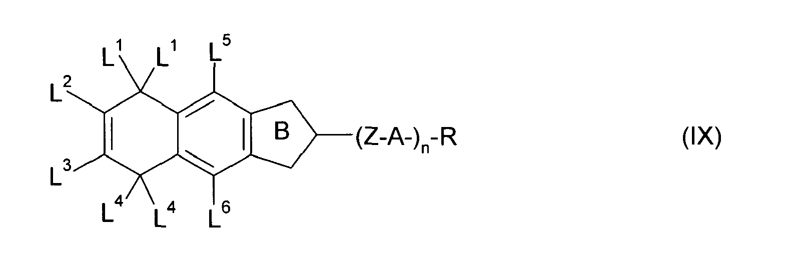 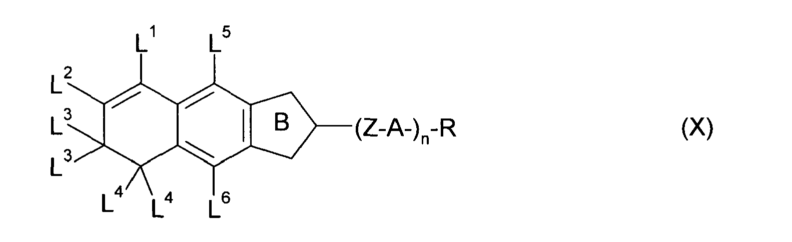 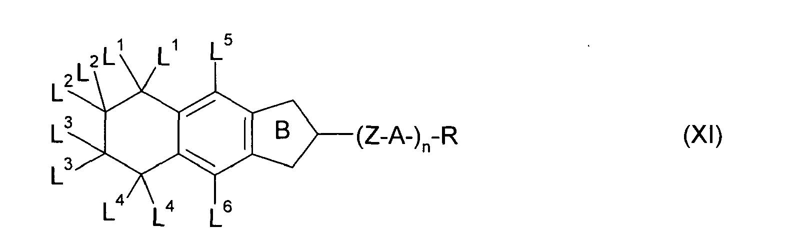 in which, in the formulae (II) to (XI),
Z in each case, independently of one another, denotes a single bond, a double bond, -CF₂O-, -OCF₂-, -CH₂CH₂-, -CF₂CF₂-, -C(O)O-, -OC(O)-, -CH₂O-, -OCH₂-, -CF=CH-, -CH=CF-, -CF=CF-, -CH=CH- or -C≡C-,
A in each case, independently of one another, denotes 1,4-phenylene, in which =CH- may be replaced once or twice by =N-, and which may be mono- to tetrasubstituted, independently of one another, by halogen (-F, -Cl, -Br, -I), -CN, -CH₃, -CH₂F, -CHF₂, -CF₃, -OCH₃, -OCH₂F, -OCHF₂ or -OCF₃, 1,4-cyclohexylene, 1,4-cyclohexenylene or 1,4-cyclohexadienylene, in which -CH₂- may be replaced once or twice, independently of one another, by -O- or -S- in such a way that heteroatoms are not directly adjacent, and which may be mono- or polysubstituted by halogen, or 1,3-cyclobutylene or bicyclo[2.2.2]octane,
R denotes hydrogen, an alkyl, alkoxy, alkenyl or alkynyl radical having 1 to 15 or 2 to 15 C atoms respectively which is unsubstituted, monosubstituted by -CF₃ or at least monosubstituted by halogen, where, in addition, one or more CH₂ groups in these radicals may each be replaced, independently of one another, by -O-, -S-, -CO-, -COO-, -OCO- or -OCO-O- in such a way that heteroatoms are not directly adjacent, halogen, -CN, -SCN, -NCS, -SF₅, -CF₃, -OCF₃, -OCHF₂ or -OCH₂F, and
n denotes 0, 1, 2 or 3,
in which, in the formulae (II) to (VI), 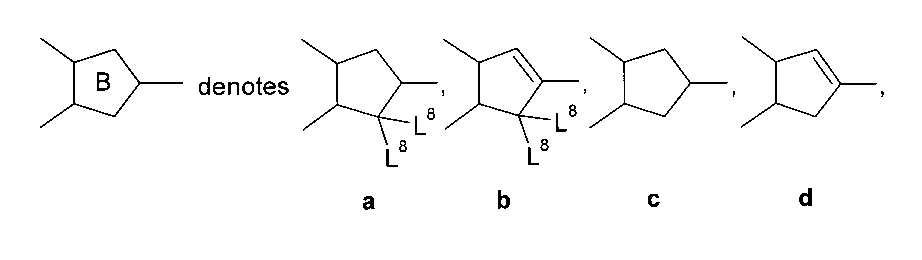 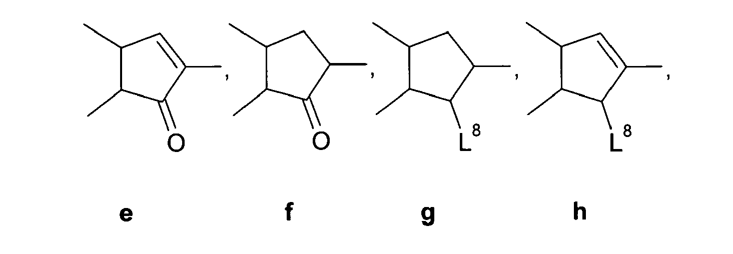 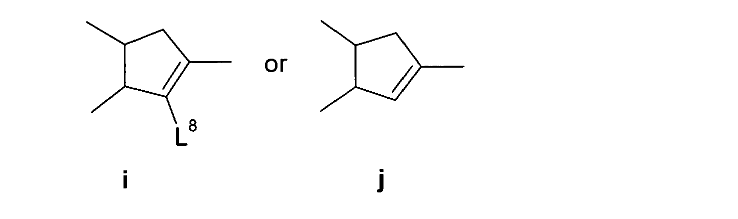
L², L³, L⁸ each, independently of one another, denote hydrogen, an alkyl, alkoxy, alkenyl or alkynyl radical having 1 to 15 or 2 to 15 C atoms respectively which is unsubstituted or at least monosubstituted by halogen, where, in addition, one or more CH₂ groups in these radicals may each be replaced, independently of one another, by -O-, -S-, -CO-, -COO-, -OCO- or -OCO-O- in such a way that heteroatoms are not directly adjacent, halogen, -CN, -SCN, -NCS, -SF₅, -CF₃, -OCF₃, -OCHF₂, -OCH₂F or -(Z-A-)ₙ-R, and
L⁴, L⁶ each, independently of one another, denote hydrogen, an alkyl, alkoxy, alkenyl or alkynyl radical having 1 to 15 or 2 to 15 C atoms respectively which is at least monosubstituted by halogen, where, in addition, one or more CH₂ groups in these radicals may each be replaced, independently of one another, by -O-, -S-, -CO-, -COO-, -OCO- or -OCO-O- in such a way that heteroatoms are not directly adjacent, halogen, -CN, -SF₅, -SCN, -NCS, -CF₃, -OCF₃, -OCHF₂ or -OCH₂F, with the proviso that L⁴ and L⁶ cannot simultaneously be hydrogen,
and
in which, in the formulae (VII) to (XI), 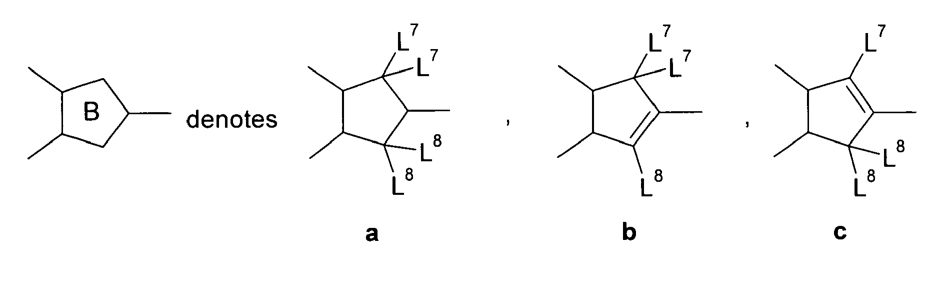 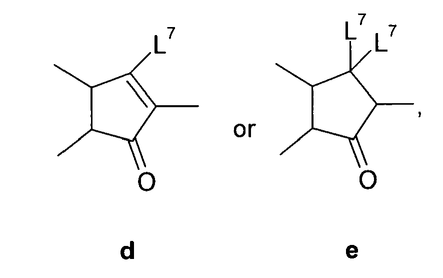
L¹ - L⁸ each, independently of one another, denote hydrogen, an alkyl, alkoxy, alkenyl or alkynyl radical having 1 to 15 or 2 to 15 C atoms respectively which is unsubstituted or at least monosubstituted by halogen, where, in addition, one or more CH₂ groups in these radicals may each be replaced, independently of one another, by -O-, -S-, -CO-, -COO-, -OCO- or -OCO-O- in such a way that heteroatoms are not directly adjacent, halogen, -CN, -SCN, -NCS, -SF₅, -CF₃, -OCF₃, -OCHF₂, -OCH₂F or -(Z-A-)ₙ-R, with the proviso that L² and/or L³ is not a hydrogen.

2. Cyclopenta[b]naphthalene derivatives according to Claim 1, **characterised in that**, in the formulae (II) to (VI), 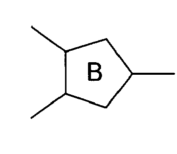 is

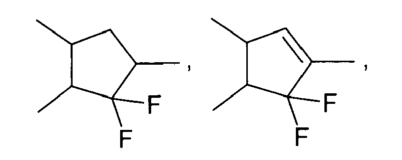

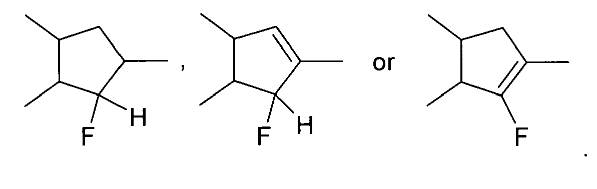

3. Cyclopenta[b]naphthalene derivatives according to Claim 1 or 2, **characterised in that**, in the formulae (II) to (VI), A is

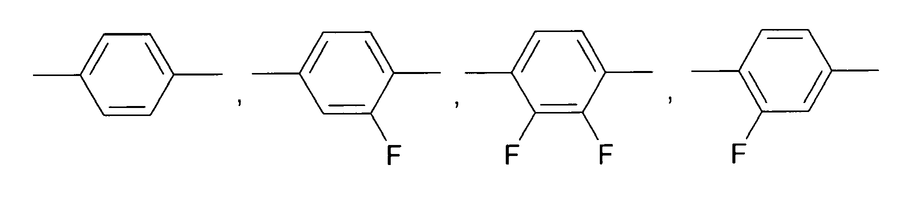

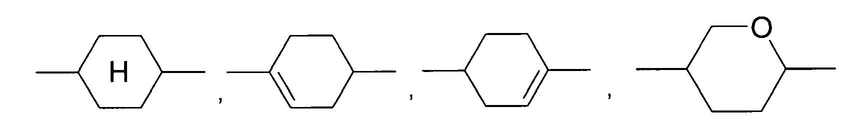

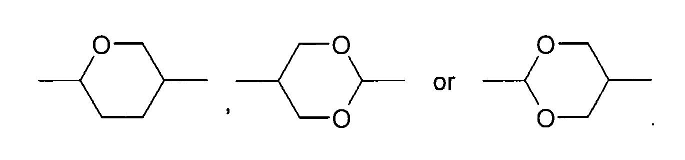

4. Cyclopenta[b]naphthalene derivatives according to at least one of Claims 1 to 3, **characterised in that**, in the formulae (II) to (VI), L² and L³ independently of one another, are hydrogen, an alkoxy radical having 1 to 7 C atoms, fluorine or chlorine.

5. Cyclopenta[b]naphthalene derivatives according to at least one of Claims 1 to 4, **characterised in that**, in the formulae (II) to (VI), L⁴ and L⁶, independently of one another, are -CF₃, fluorine or chlorine.

6. Cyclopenta[b]naphthalene derivatives according to Claim 1, **characterised in that**, in the formulae (VII) to (XI), 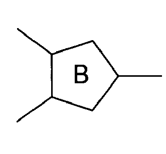 is

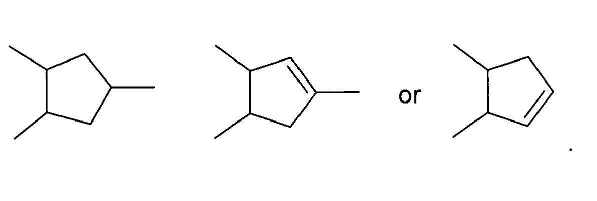

7. Cyclopenta[b]naphthalene derivatives according to Claim 1 or 6, **characterised in that**, in the formulae (VII) to (XI), A is

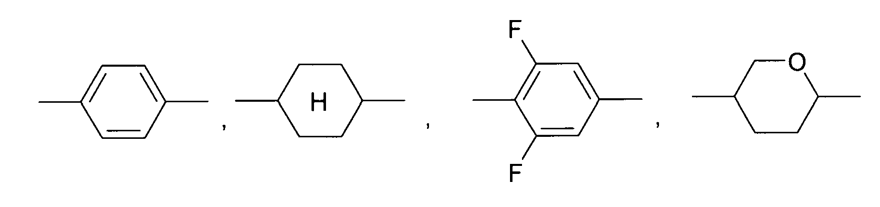

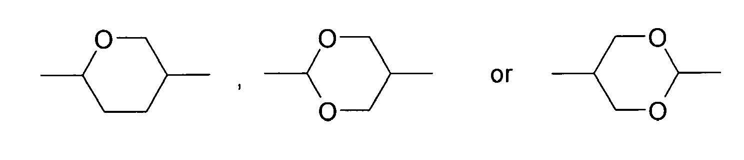

8. Cyclopenta[b]naphthalene derivatives according to at least one of Claims 1, 6 and 7, **characterised in that**, in the formulae (VII) to (XI), L² and L³, independently of one another, are, identically or differently, halogen, -CN, -SCN, -NCS, -SF₅, -CF₃, -CHF₂, -OCF₃ or -OCHF₂.

9. Cyclopenta[b]naphthalene derivatives according to at least one of Claims 1 and 6 to 8, **characterised in that**, in the formulae (VII) to (XI), L¹ and L⁴, independently of one another, are, identically or differently, hydrogen or fluorine.

10. Cyclopenta[b]naphthalene derivatives according to at least one of Claims 1 and 6 to 9, **characterised in that**, in the formulae (VII) to (XI), L⁵ and L⁶ are hydrogen.

11. Cyclopenta[b]naphthalene derivatives according to at least one of Claims 1 and 6 to 10, **characterised in that**, in the formulae (VII) to (XI), L¹, L², L³ and L⁴ are fluorine and L⁵ and L⁶ are hydrogen.

12. Cyclopenta[b]naphthalene derivatives according to at least one of the preceding claims, **characterised in that** Z is a single bond, -CF₂O-, -OCF₂-, -CF₂CF₂-, -CH=CH-, -CF=CH-, -CH=CF- or -CF=CF-.

13. Cyclopenta[b]naphthalene derivatives according to at least one of the preceding claims, **characterised in that** R is an alkyl radical, alkoxy radical or alkenyl radical having 1 to 7 or 2 to 7 C atoms respectively.

14. Use of cyclopenta[b]naphthalene derivatives according to at least one of the preceding claims in liquid-crystalline media.

15. Liquid-crystalline medium comprising at least two liquid-crystalline compounds, **characterised in that** it comprises at least one cyclopenta[b]naphthalene derivative according to at least one of Claims 1 to 13.

16. Electro-optical display element containing a liquid-crystalline medium according to Claim 15.

17. Mesogenic medium, **characterised in that** it comprises at least one cyclopenta[b]naphthalene derivative of the formulae (VII) to (XI) according to at least one of Claims 1 and 6 to 13.

18. Electro-optical light-modulation element which comprises an electrode arrangement, at least one light polarisation element and a mesogenic modulation medium, where the light-modulation element is operated at a temperature at which the mesogenic modulation medium in the unaddressed state is in the isotropic phase, **characterised in that** the mesogenic modulation medium comprises at least one cyclopenta[b]naphthaene derivative of the formulae (VII) to (XI) according to at least one of Claims 1 and 6 to 13.

## Revendications

1. Dérivés de cyclopenta[b]naphtalène des formules générales (II) à (XI) 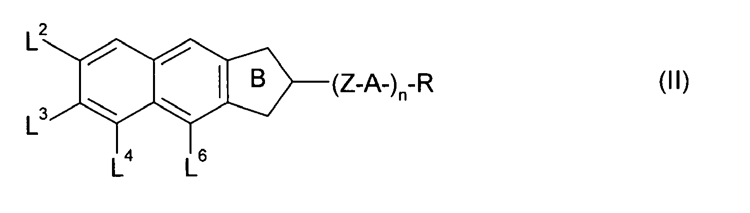 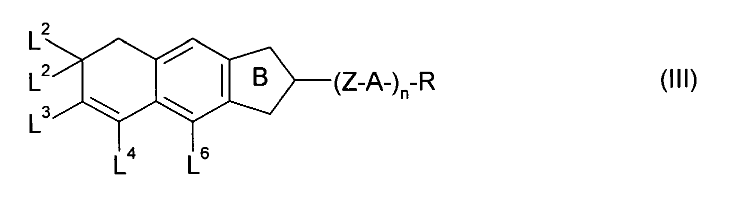 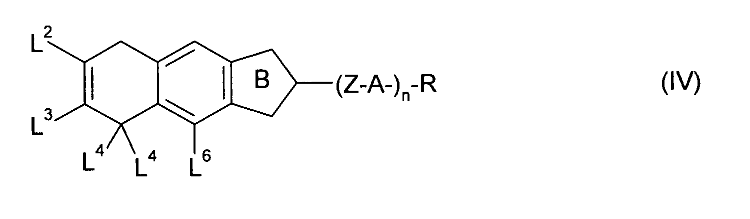 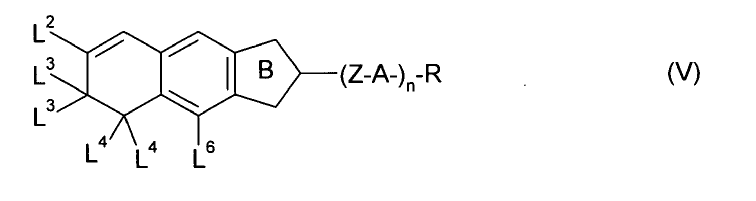 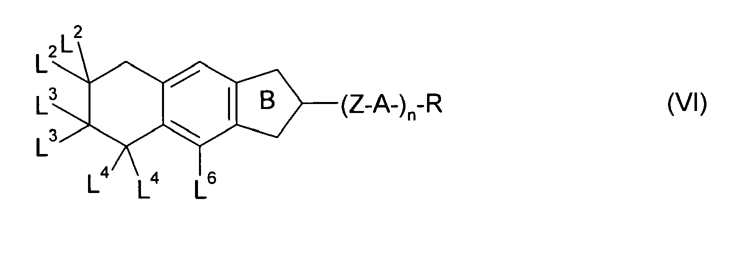 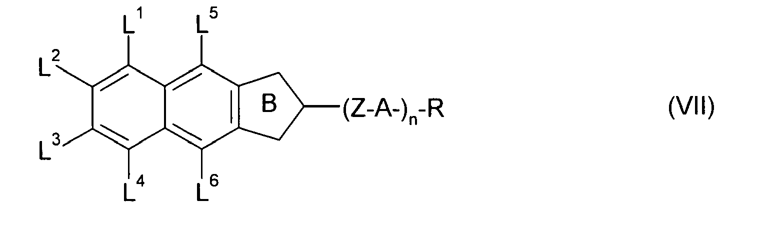 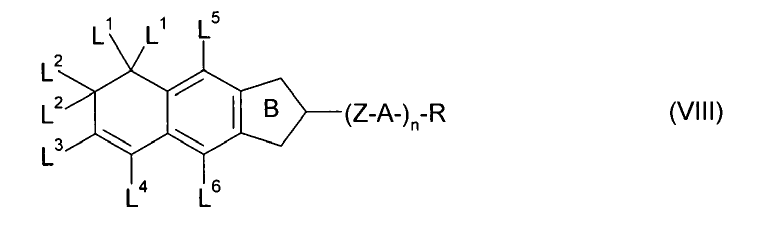 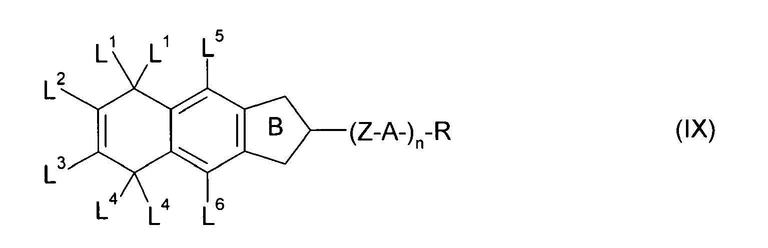 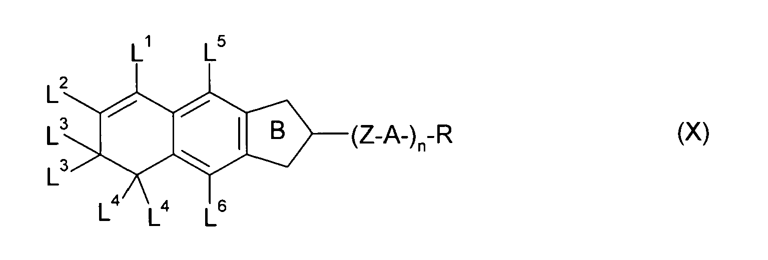 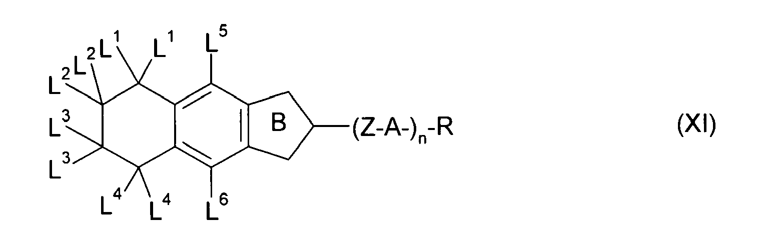 dans lesquels, dans les formules (II) à (XI),
Z dans chaque cas, indépendamment l'un de l'autre, représente une liaison simple, une double liaison, -CF₂O-, -OCF₂-, -CH₂CH₂-, -CF₂CF₂-, -C(O)O-, -OC(O)-, -CH₂O-. -OCH₂-, -CF=CH-, -CH=CF-, -CF=CF-, -CH=CH- ou -C≡C-,
A dans chaque cas, indépendamment l'un de l'autre, représente 1,4-phénylène, dans lequel =CH- peut être remplacé une fois ou deux fois par =N-, et qui peut être mono- à tétrasubstitué, indépendamment l'un de l'autre, par halogène (-F, -CI, -Br, -I), -CN, -CH₃, -CH₂F, -CHF₂, -CF₃, -OCH₃, -OCH₂F, -OCHF₂ ou -OCF₃, 1,4-cyclohexylène, 1,4-cyclohexénylène ou 1,4-cyclohexadiénylène, dans lequel -CH₂- peut être remplacé une fois ou deux fois, indépendamment l'un de l'autre, par -O- ou -S- de telle sorte que des hétéroatomes ne soient pas directement adjacents, et qui peut être mono- ou polysubstitué par halogène, ou 1,3-cyclobutylène ou bicyclo[2.2.2]octane,
R représente hydrogène, un radical alkyle, alcoxy, alkényle ou alkynyle ayant 1 à 15 ou 2 à 15 atomes de C respectivement qui est non substitué, monosubstitué par -CF₃ ou au moins monosubstitué par halogène, où, en addition, un ou plusieurs groupes CH₂ dans ces radicaux peuvent chacun être remplacés, indépendamment l'un de l'autre, par -O-, -S-, -CO-, -COO-, -OCO- ou -OCO-O- de telle sorte que des hétéroatomes ne soient pas directement adjacents, halogène, -CN, -SCN, -NCS, -SF₅, -CF₃, -OCF₃, -OCHF₂ ou -OCH₂F, et
n représente 0, 1, 2 ou 3,
dans lesquels, dans les formules (II) à (VI), 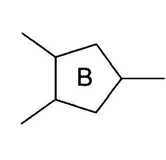 représente 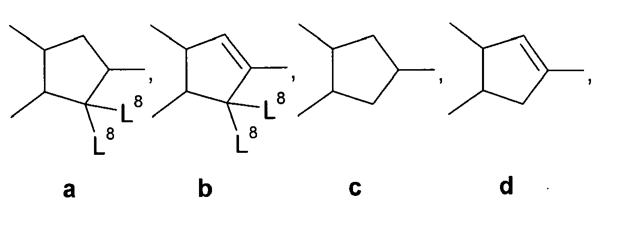 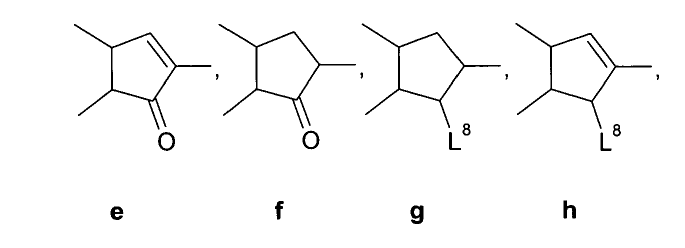 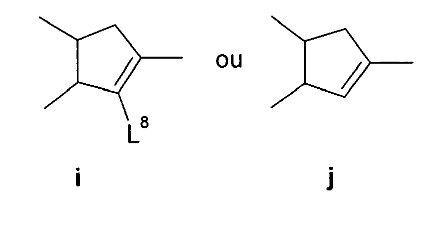
L² L³, L⁸ chacun, indépendamment l'un de l'autre, représentent hydrogène, un radical alkyle, alcoxy, alkényle ou alkynyle ayant 1 à 15 ou 2 à 15 atomes de C respectivement qui est non substitué ou au moins monosubstitué par halogène, où, en addition, un ou plusieurs groupes CH₂ dans ces radicaux peuvent chacun être remplacés, indépendamment l'un de l'autre, par -O-, -S-, -CO-, -COO-, -OCO- ou -OCO-O- de telle sorte que des hétéroatomes ne soient pas directement adjacents, halogène, -CN, -SCN, -NCS, -SF₅, -CF₃, -OCF₃, -OCHF₂, -OCH₂F ou -(Z-A-)ₙ-R, et
L⁴, L⁶ chacun, indépendamment l'un de l'autre, représentent hydrogène, un radical alkyle, alcoxy, alkényle ou alkynyle ayant 1 à 15 ou 2 à 15 atomes de C respectivement qui est non substitué ou au moins monosubstitué par halogène, où, en addition, un ou plusieurs groupes CH₂ dans ces radicaux peuvent chacun être remplacés, indépendamment l'un de l'autre, par -O-, -S-, -CO-, -COO-, -OCO- ou -OCO-O- de telle sorte que des hétéroatomes ne soient pas directement adjacents, halogène, -CN, -SF₅, -SCN, -NCS, -CF₃, -OCF₃, -OCHF₂ ou -OCH₂F, sous réserve que L⁴ et L⁶ ne puissent pas être simultanément hydrogène,
et
dans lesquels, dans les formules (VII) à (XI), 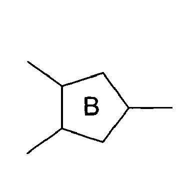 représente 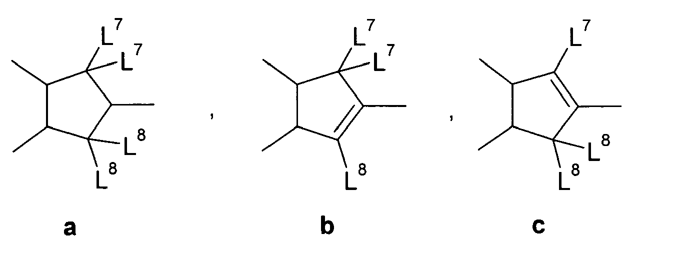 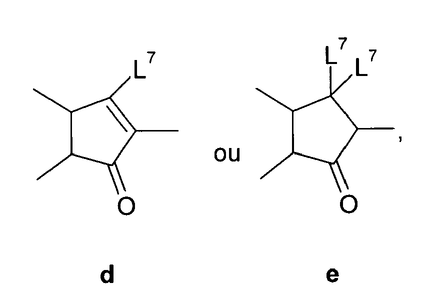
L¹ - L⁸ chacun, indépendamment l'un de l'autre, représentent hydrogène, un radical alkyle, alcoxy, alkényle ou alkynyle ayant 1 à 15 ou 2 à 15 atomes de C respectivement qui est non substitué ou au moins monosubstitué par halogène, où, en addition, un ou plusieurs groupes CH₂ dans ces radicaux peuvent chacun être remplacés, indépendamment l'un de l'autre, par -O-, -S-, -CO-, -COO-, -OCO- ou -OCO-O- de telle sorte que des hétéroatomes ne soient pas directement adjacents, halogène, -CN, -SCN, -NCS, -SF₅, -CF₃, -OCF₃, -OCHF₂, -OCH₂F ou -(Z-A-)ₙ-R, sous réserve que L² et/ou L³ ne soit pas un hydrogène.

2. Dérivés de cyclopenta[b]naphtalène selon la revendication 1, **caractérisés en ce que**, dans les formules (11) à (VI), 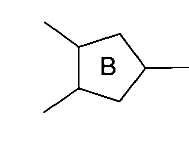 est

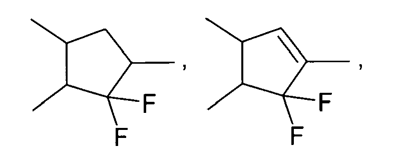

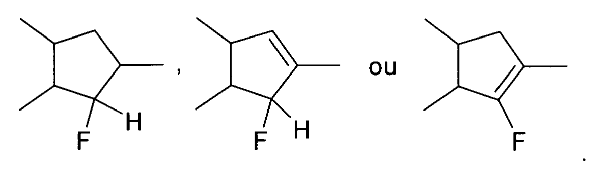

3. Dérivés de cyclopenta[b]naphtalène selon la revendication 1
ou 2, **caractérisés en ce que**, dans les formules (II) à (VI), A est

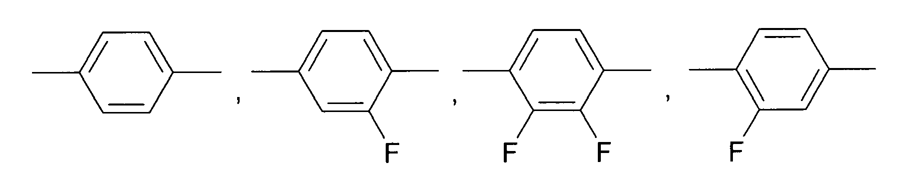

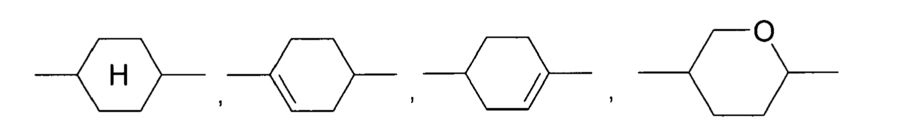

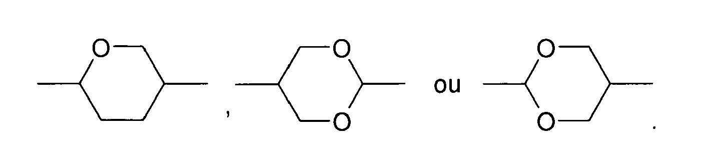

4. Dérivés de cyclopenta[b]naphtalène selon au moins l'une des revendications 1 à 3, **caractérisés en ce que**, dans les formules (II) à (VI), L² et L³, indépendamment l'un de l'autre, sont hydrogène, un radical alcoxy ayant 1 à 7 atomes de C, fluor ou chlore.

5. Dérivés de cyclopenta[b]naphtalène selon au moins l'une des revendications 1 à 4, **caractérisés en ce que**, dans les formules (II) à (VI), L⁴ et L⁶, indépendamment l'un de l'autre, sont -CF₃, fluor ou chlore.

6. Dérivés de cyclopenta[b]naphtalène selon la revendication 1, **caractérisés en ce que**, dans les formules (VII) à (XI), 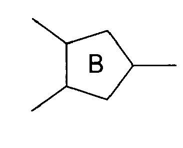 est

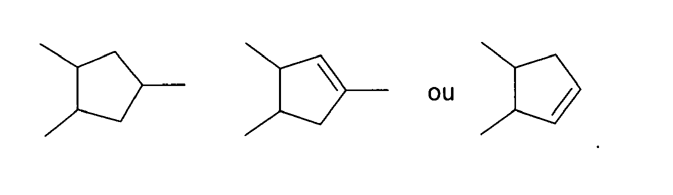

7. Dérivés de cyclopenta[b]naphtalène selon la revendication 1
ou 6, **caractérisés en ce que**, dans les formules (VII) à (XI), A est

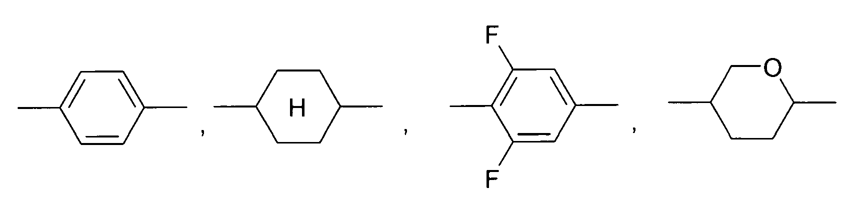

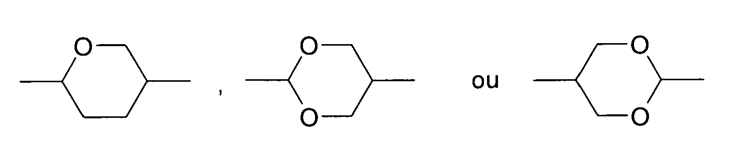

8. Dérivés de cyclopenta[b]naphtalène selon au moins l'une des revendications 1, 6 et 7, **caractérisés en ce que**, dans les formules (VII) à (XI), L² et L³, indépendamment l'un de l'autre, sont, de façon identique ou différente, halogène, -CN, -SCN, -NCS, -SF₅, -CF₃, -CHF₂, -OCF₃ ou -OCHF₂.

9. Dérivés de cyclopenta[b]naphtalène selon au moins l'une des revendications 1 et 6 à 8, **caractérisés en ce que**, dans les formules (VII) à (XI), L¹ et L⁴, indépendamment l'un de l'autre, sont, de façon identique ou différente, hydrogène ou fluor.

10. Dérivés de cyclopenta[b]naphtalène selon au moins l'une des revendications 1 et 6 à 9, **caractérisés en ce que**, dans les formules (VII) à (XI), L⁵ et L⁶ sont hydrogène.

11. Dérivés de cyclopenta[b]naphtalène selon au moins l'une des revendications 1 et 6 à 10, **caractérisés en ce que**, dans les formules (VII) à (XI), L¹, L², L³ et L⁴ sont fluor et L⁵ et L⁶ sont hydrogène.

12. Dérivés de cyclopenta[b]naphtalène selon au moins l'une des revendications précédentes, **caractérisés en ce que** Z est une liaison simple, -CF₂O-, -OCF₂-, -CF₂CF₂-, -CH=CH-, -CF=CH-, -CH=CF- ou -CF=CF-.

13. Dérivés de cyclopenta[b]naphtalène selon au moins l'une des revendications précédentes, **caractérisés en ce que** R est un radical alkyle, un radical alcoxy ou un radical alkényle ayant 1 à 7 ou 2 à 7 atomes de C respectivement.

14. Utilisation de dérivés de cyclopenta[b]naphtalène selon au moins l'une des revendications précédentes dans des milieux cristallins liquides.

15. Milieu cristallin liquide comprenant au moins deux composés cristallins liquides, **caractérisé en ce qu'**il comprend au moins un dérivé de cyclopenta[b]naphtalène selon au moins l'une des revendications 1 à 13.

16. Elément d'affichage électro-optique contenant un milieu cristallin liquide selon la revendication 15.

17. Milieu mésogène, **caractérisés en ce qu'**il comprend au moins un dérivé de cyclopenta[b]naphtalène des formules (VII) à (XI) selon au moins l'une des revendications 1 et 6 à 13.

18. Elément de modulation de lumière électro-optique qui comprend un agencement d'électrodes, au moins un élément de polarisation de lumière et un milieu de modulation mésogène, dans lequel l'élément de modulation de lumière est activé à une température à laquelle le milieu de modulation mésogène dans l'état non adressé est dans la phase isotrope, **caractérisé en ce que** le milieu de modulation mésogène comprend au moins un dérivé de cyclopenta[b]naphtalène des formules (VII) à (XI) selon au moins l'une des revendications 1 et 6 à 13.
